(19) Europäisches Patentamt
European Patent Office
Office européen des brevets

(11) EP 4 458 824 A1

(12) **EUROPEAN PATENT APPLICATION**
published in accordance with Art. 153(4) EPC

(43) Date of publication:
**06.11.2024 Bulletin 2024/45**

(21) Application number: **22914630.3**

(22) Date of filing: **26.12.2022**

(51) International Patent Classification (IPC):
***C07D 409/14*** (2006.01) ***C07D 209/04*** (2006.01)
***C07D 487/02*** (2006.01) ***C07D 495/02*** (2006.01)
***C07D 247/00*** (2006.01) ***C07D 241/04*** (2006.01)
***A61K 31/4045*** (2006.01) ***A61K 31/496*** (2006.01)
***A61P 25/28*** (2006.01) ***A61P 25/16*** (2006.01)

(52) Cooperative Patent Classification (CPC):
**A61K 31/4045; A61K 31/496; A61P 25/16; A61P 25/28; C07D 209/04; C07D 241/04; C07D 247/00; C07D 409/14; C07D 487/02; C07D 495/02**

(86) International application number:
**PCT/CN2022/141820**

(87) International publication number:
**WO 2023/125376 (06.07.2023 Gazette 2023/27)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **27.12.2021 CN 202111615487**

(71) Applicants:
- **Shanghai Jingxin Biomedical Co., Ltd.**
  **Shanghai 201210 (CN)**
- **Zhejiang Jingxin Pharmaceutical Co., Ltd.**
  **Shaoxing, Zhejiang 312500 (CN)**

(72) Inventors:
- **JIANG, Qingyun**
  **Shanghai 201210 (CN)**
- **FANG, Xin**
  **Shanghai 201210 (CN)**
- **LI, Ping**
  **Shanghai 201210 (CN)**
- **LI, Ningning**
  **Shanghai 201210 (CN)**
- **XIE, ZHihan**
  **Shanghai 201210 (CN)**
- **WU, Yinhui**
  **Shanghai 201210 (CN)**
- **MA, Ningtian**
  **Shanghai 201210 (CN)**
- **HOU, Jian**
  **Shanghai 201210 (CN)**

(74) Representative: **Greaves Brewster LLP**
**Copa House**
**Station Road**
**Cheddar, Somerset BS27 3AH (GB)**

# (54) FUSED BICYCLIC HETEROARYL AMIDE COMPOUND AS PROTEIN AGGREGATION INHIBITOR

(57) Provided are a fused bicyclic heteroaryl amide compound as a protein aggregation inhibitor, as well as the use of such compound in the treatment or prevention of neurodegenerative diseases characterized by protein aggregation, such as Alzheimer's disease, Parkinson's disease, frontotemporal dementia, dementia with Lewy bodies, Parkinson's disease dementia, multiple system atrophy, amyotrophic lateral sclerosis, Huntington's disease, and cancer.

EP 4 458 824 A1

## Description

### Technical Field

The present invention relates to fused bicyclic heteroaryl amide compound which inhibiting protein aggregation, pharmaceutical compositions, and use in the treatment or prevention of neurodegenerative diseases characterized by protein aggregation, such as Alzheimer's disease, Parkinson's disease, frontotemporal dementia, Lewy body disease, Parkinson's disease dementia, multiple system atrophy, amyotrophic lateral sclerosis, Huntington's disease, and cancer.

### Background of the Invention

Neurodegenerative diseases, such as Alzheimer's disease, Parkinson's disease, and frontotemporal dementia, are one of the important causes of death for the elderly. The common feature among these neurodegenerative diseases is the chronic accumulation of proteins into neurotoxic aggregates.

The initial stage of protein aggregation involves mutations or post-translational modifications (e.g., nitrosylation, oxidation) of the target protein, which generate abnormal conformations and promote interactions with proteins that resemble misfolding. Abnormal proteins then aggregate to form dimers, trimers, and higher-order multimers (also termed soluble oligomers), which may disrupt synaptic function. In addition, subsequent aggregation is fixed in the cell membrane and forms spherical oligomers (which in turn form pores in the membrane) and/or protofibrils or fibrils. These larger insoluble fibers may function as reservoirs for bioactive oligomers.

Research has shown that there is a causal relationship between the progressive accumulation of protein aggregates and the onset of neurodegenerative diseases. Various other proteins can accumulate in the brain of patients with neurodegeneration, such as α-synuclein (SYN), Aβ Protein, Tau, and TDP43. The cognitive impairment of these patients is closely associated to synaptic loss in the neocortex and limbic system, and the increased levels of protein aggregation may lead to this synaptic loss. Many studies focus on detailing the mechanism through which the accumulation of metabolites of α-synaptic nucleoproteins and other amyloid precursor proteins promotes synaptic damage and neurodegeneration. Meanwhile, many studies support the hypothesis that the formation of small aggregates (also known as oligomers) plays a major role in neurotoxicity. These peptide oligomers can be organized into higher-order multimers such as dimers, trimers, tetramers, and pentamers. High levels of these oligomers are predictive of dementia and synaptic loss in patients. Multiple pieces of evidence indicate oligomers rather than smaller precursor filaments are the toxic substances, so compounds targeting these early aggregation processes in a specific manner can be used as potential new drugs for the treatment or prevention of related diseases such as Alzheimer's and Parkinson's disease.

Various neurodegenerative diseases involve the accumulation of neurotoxic protein-based aggregates. In idiopathic Parkinson's disease, dementia with Lewy bodies, Parkinson's disease with dementia, and multiple system atrophy, neurotoxic aggregates are composed of α-synuclein, which is a synaptic protein that is intracellular under normal conditions. In frontotemporal dementia and amyotrophic lateral sclerosis, neurotoxic aggregates originate from other intracellular proteins such as tau, TDP-43, or SOD1. For certain diseases (such as Alzheimer's disease), α-Synuclein and other major proteins (such as Aβ Protein) aggregation. In Huntington's disease, aggregates are formed from the cleavage products of Htt proteins.

Two mechanisms are implicated in these protein aggregation processes. In the first mechanism, misfolded and/or aggregated proteins are fixed to multiple cell membrane structures. The binding of misfolded or aggregated molecules to the plasma membrane or the membrane of organelles (such as mitochondria or lysosomes) may interfere with protein transcription, autophagy, mitochondrial function, and pore formation. For example, neurotoxic α-synuclein aggregates and interacts with lipids in cell membranes by a specific part of the C-terminal region of the synuclein protein. Compounds which bind to this region can inhibit protein-protein or protein-lipid interactions and can therefore be used to block neurotoxic oligomerization of α-synuclein or other proteins and their interactions with membranes. In the second mechanism, aggregated proteins are released from fixed subunits and propagate to adjacent cells. This cell-to-cell propagation of toxic protein aggregates may be the cause of further deterioration of neurodegenerative diseases. Herefore, small molecule drugs that interact with target proteins may limit release and propagation, and thereby reducing the neurotoxic effects of aggregated proteins.

WO2015116663A1 describes a series of compounds that inhibit protein aggregation and use in the treatment of neurodegenerative diseases. Although there has been some progress in the field of drugs for treating neurodegenerative diseases, it is still necessary to further develop protein aggregation inhibitors that meet clinical needs.

### Summary of the Invention

The invention provides a series of fused bicyclic heteroaryl amide compounds with advantages such as high inhibition rate of α-synuclein aggregation, easier entry into the brain, longer half-life, higher bioavailability, lower cardiac

toxicity, and lower effective dose.

Specifically, the invention provides a compound of formula (I) or a pharmaceutically acceptable salt thereof:

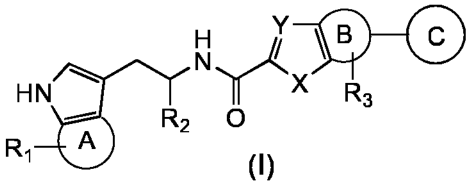

(I)

Wherein,

X is selected from O, S and $NR_a$;
Y is selected from $CR_b$ and N;
Ring A is selected from benzene ring and pyridine ring;
Ring B is selected from benzene ring, pyridine ring, pyridazine ring, pyrimidine ring and pyrazine ring;

Ring C is selected from

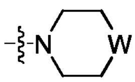 , 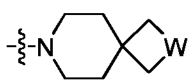 , 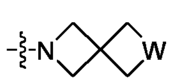 , 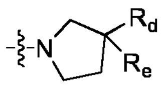 ,

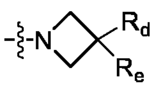 , 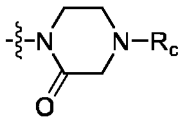 and 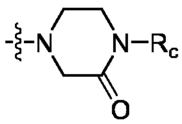 ;

W is selected from O, $NR_c$ and $CR_dR_e$;
$R_1$ is absent or is selected from fluoro, chloro, bromo, iodo and $C_{1-6}$ alkyl;
$R_2$ is selected from $C_{1-6}$ alkyl, $C_{2-6}$ alkenyl, -$C_{1-4}$ alkylene-O-$C_{1-4}$ alkyl, and -$C_{1-4}$ alkylene-$NR_aR_b$;
$R_3$ is absent or is selected from fluoro, chloro, bromo, iodo, cyano and $C_{1-6}$ alkyl;
$R_a$ and $R_b$ are independently selected from H and $C_{1-6}$ alkyl;
$R_c$ is selected from H, cyano, $C_{1-6}$ alkyl, cyclopropyl, cyclobutyl, cyclopentyl, cyclohexyl, phenyl, -$SO_2R_f$, and 1-$C_{1-6}$ alkyl-4-piperidinyl;
$R_d$ and $R_e$ are independently selected from H, fluoro, chloro, bromo, iodo, hydroxyl, cyano, -$NR_aR_b$, $C_{1-6}$ alkyl, cyclopropyl, cyclobutyl, cyclopentyl, cyclohexyl, phenyl, and 1-$C_{1-6}$ alkyl-4-piperidinyl;

Rε is selected from methyl, ethyl, n-propyl, isopropyl, cyclopropyl, cyclobutyl, cyclopentyl, and cyclohexyl.

In another aspect, the invention provides a pharmaceutical composition comprising a compound of formula (I) or a pharmaceutically acceptable salt thereof, and one or more pharmaceutically acceptable excipients.

In another aspect, the invention provides a method of treating neurodegenerative diseases associated with protein aggregation, wherein the method comprises administering to a subject in need of such treatment an effective amount of at least one compound of Formula (I) or a pharmaceutically acceptable salt thereof.

In another aspect, the invention provides a method of interfering with the accumulation of protein or peptide aggregates in cells or regulating, preventing, delaying, reversing, or inhibiting protein or peptide aggregates in cells. The method comprises contacting cells with an effective amount of at least one compound of formula (I) or a salt thereof and/or with at least one pharmaceutical composition of the invention, wherein the contact is in vitro, ex vivo, or in vivo.

In another aspect, the invention provides the use of the compound of formula (I) or pharmaceutically acceptable salt thereof in the preparation of drugs for the treatment of neurodegenerative diseases associated with protein aggregation.

## Detailed Description of the Embodiments

Before the invention is further described, it should be understood that the invention is not limited to the specific

embodiments described, as such may, of course, vary. It should also be understood that the terminology used herein is for the purpose of describing particular embodiments only, and is not intended to be limiting, since the scope of the present invention will be limited only by the appended claims.

Unless defined otherwise, all technical terms used herein have the same meaning as is commonly understood by one of ordinary skill in the art to which the invention belongs. All patents, applications, published applications, and other publications referred to herein are incorporated by reference in their entireties. If the definitions in this section are contrary to or otherwise inconsistent with the definitions in patents, applications, and other publications that is herein incorporated by reference, the definitions in this section will prevails over the definitions incorporated by reference.

As used herein and in the appended claims, the singular forms "a", "an", and "the" include plural referents unless the context clearly dictates otherwise. It should also be noted that the claims may be drafted to exclude any optional elements. Similarly, this explanation is intended to serve as antecedent basis for the use of exclusionary terms such as " solely", "only" and the like in relation to the elements of the cited claims, or the use of "negative" limitation.

As used herein, the terms "including", "containing", and " comprising" are used in their open, non-limiting sense.

To provide a more concise description, the term "about" is not used before some quantitative expressions herein. It should be understood that regardless of whether the term " about " is explicitly used or not, every content given herein represents an actual given value, and it also represents an approximate value of such given value that would reasonably be inferred based on the ordinary skill in the art, including equivalent and approximations due to experimental and/or measurement conditions for such given value. Whenever the yield is given as a percentage, such yield represents the ratio of the mass of the entity used to calculate the yield to the maximum amount of the same entity that could be obtained under the particular stoichiometric conditions. The concentration that is given as percentages represents the mass ratio, Unless defined otherwise.

Unless defined otherwise, all technical terms used herein have the same meanings as commonly understood by one of ordinary skill in the art to which the invention belongs. Although any methods and materials similar or equivalent to those described herein may also be used in the practice or testing of the invention, the preferred methods and materials are described below. All publications mentioned herein are incorporated by reference to disclose and describe the methods and/or materials in conjunction with the cited publications.

**Definitions**

As used herein, the term "alkyl" refers to a saturated monovalent hydrocarbon group with a straight or branched chain. For example, "$C_{1-6}$ alkyl" refers to an alkyl group with 1 to 6 carbon atoms in the chain. Examples of alkyl groups include but are not limited to methyl, ethyl, n-propyl, isopropyl, n-butyl, isobutyl, sec-butyl, tert-butyl, n-pentyl, isopentyl, neopentyl, n-hexyl, and isohexyl.

As used herein, the term "alkylene" refers to a divalent group obtained by removing hydrogen atoms from an alkyl group as defined above. Alkylene groups can be either straight or branched divalent alkyl groups. For example, "$C_{1-4}$ alkylene" refers to an alkylene group with 1 to 4 carbon atoms. Examples of alkylene include, but are not limited to, methylene (i.e. $-CH_2-$), ethylene (i.e. $-CH_2CH_2-$ or $-CH(CH_3)-$), and propylene (i.e. $-CH_2-CH_2-CH_2-$, $-CH(CH_2CH_3)-$ or $-CH_2CH(CH_3)-$).

As used herein, the term "alkenyl" refers to a straight or branched hydrocarbon group containing one or more double bonds, typically 2 to 20 carbon atoms in length. For example, "$C_{2-6}$ alkenyl" contains 2 to 6 carbon atoms. Examples of alkenyl include but are not limited to vinyl, propylene, butenol, 2-methyl-2-butene-1-yl, etc.

As used herein, the term "substituted" refers to a specific group or moiety bears one or more substituents. The term "unsubstituted" refers to a specific group does not have a substituent. The term "optionally substituted" refers to a specific group is either unsubstituted or substituted by one or more substituents. When the term "substituted" is used to describe a structural system, substitution can occur at any valence-allowed position on the system.

As used herein, the term "independently" refers to when more than one substituent is selected from many possible substituents, these substituents may be the same or different. That is, each substituent is individually selected from the entire group of the listed possible substituents.

When using a class of substituents herein, the nomenclature "$C_{i-j}$" (where j>i) represents an embodiment of the invention that independently realizes each number of carbon atoms from i to j (including i and j). For example, the term $C_{1-3}$ independently represents an embodiment that haves one carbon atom (Ci), an embodiment that haves two carbon atoms ($C_2$), and an embodiment that haves three carbon atoms ($C_3$).

As used herein, the wavy line



represents the point of attachment of the group to the rest of the molecule.

As used herein, the term "pharmaceutically acceptable salt" refers to a salt of free acid or base of the compound described herein, that is non-toxic, biologically tolerable, or biologically suitable for administration to subjects.

As used herein, the terms "treat" or "treatment" encompass both "preventive" and "therapeutic" treatments.

As used herein, the term "subject" refers to a mammalian patient in need of such treatment, such as a human.

One of Ordinary skill in the art will recognize that the species listed or shown in the definitions provided herein are not exhaustive, and other species within the scope of these definitions may also be selected.

## Compound

The invention provides a compound of formula (I) or a pharmaceutically acceptable salt thereof,

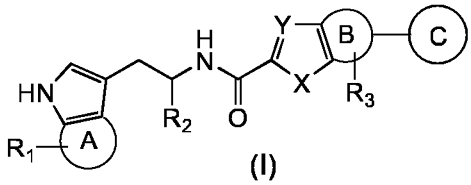

(I)

Wherein,

X is selected from O, S and $NR_a$;
Y is selected from $CR_b$ and N;
Ring A is selected from benzene ring and pyridine ring;
Ring B is selected from benzene ring, pyridine ring, pyridazine ring, pyrimidine ring and pyrazine ring;
Ring C is selected from

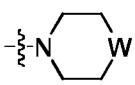, 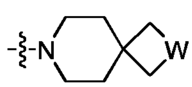, 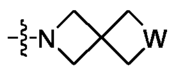, 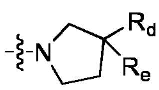,

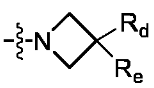, 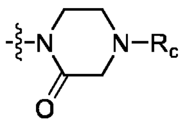 and 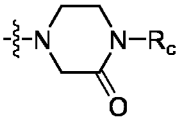;

W is selected from O, $NR_c$ and $CR_dR_e$;
$R_1$ is absent or is selected from fluoro, chloro, bromo, iodo and $C_{1-6}$ alkyl;
$R_2$ is selected from $C_{1-6}$ alkyl, $C_{2-6}$ alkenyl, -$C_{1-4}$ alkylene-O-$C_{1-4}$ alkyl, and -$C_{1-4}$ alkylene-$NR_aR_b$;
$R_3$ is absent or is selected from fluoro, chloro, bromo, iodo, cyano and $C_{1-6}$ alkyl;
$R_a$ and $R_b$ are independently selected from H and $C_{1-6}$ alkyl;
$R_c$ is selected from H, cyano, $C_{1-6}$ alkyl, cyclopropyl, cyclobutyl, cyclopentyl, cyclohexyl, phenyl, -$SO_2R_f$, and 1-$C_{1-6}$ alkyl-4-piperidinyl;
$R_d$ and $R_e$ are independently selected from H, fluoro, chloro, bromo, iodo, hydroxyl, cyano, -$NR_aR_b$, $C_{1-6}$ alkyl, cyclopropyl, cyclobutyl, cyclopentyl, cyclohexyl, phenyl, and 1-$C_{1-6}$ alkyl-4-piperidinyl;
Rε is selected from methyl, ethyl, n-propyl, isopropyl, cyclopropyl, cyclobutyl, cyclopentyl, and cyclohexyl.

It will be appreciated by those skilled in the art that in the compound of formula (I), the ring C is connected to the ring carbon atom of the ring B through its ring nitrogen atom.

In some embodiments, X is O, Y is $CR_b$; in some embodiments, X is S, Y is $CR_b$; in some embodiments, X is $NR_a$, Y is $CR_b$; in some embodiments, X is S, Y is N.

In some embodiments, X is O, Y is CH; in some embodiments, X is S, Y is CH; in some embodiments, X is S, Y is $CCH_3$; in some embodiments, X is S, Y is N; in some embodiments, X is NH, Y is CH; in some embodiments, X is $NCH_3$, Y is CH.

In some embodiments, Ring A is a benzene ring.

In some embodiments, Ring B is selected from a benzene ring and a pyridine ring; in some embodiments, Ring B is a benzene ring; in some embodiments, Ring B is a pyridine ring.

In some embodiments, the ring C is

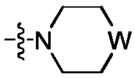,

in some embodiments, the ring C is selected from

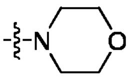, 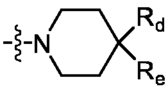 and 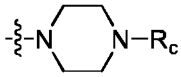,

in some embodiments, the ring C is

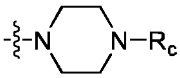.

In some embodiments, the ring C is selected from

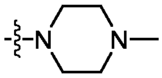, 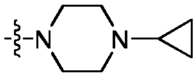,

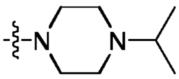, 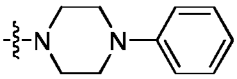, 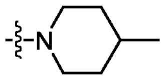, 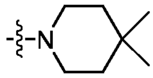, 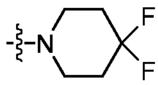, 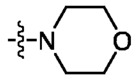,

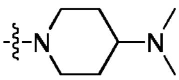, 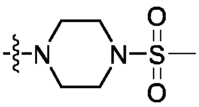, 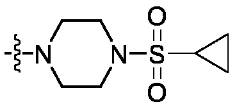, 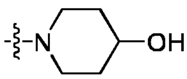, 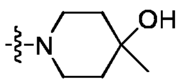, 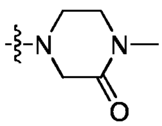

and 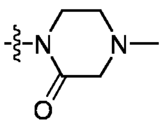.

In some embodiments, the ring C is

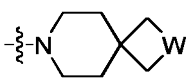,

in some embodiments, the ring C is selected from

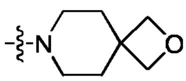, 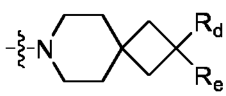 and 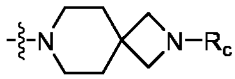,

in some embodiments, the ring C is

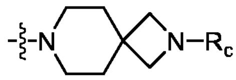

.

In some embodiments, the ring C is selected from

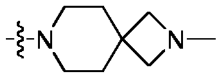

and

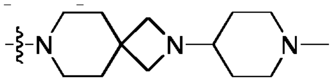

.

In some embodiments, the ring C is

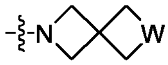

,

in some embodiments, the ring C is selected from

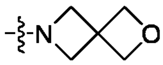

,

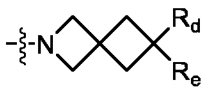

and

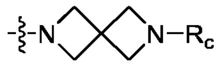

,

in some embodiments, the ring C is

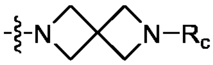

.

In some embodiments, the ring C is selected from

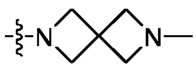

and

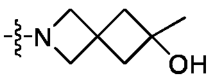

.

In some embodiments, the ring C is

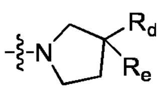

.

In some embodiments, the ring C is

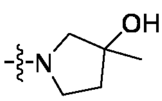

.

In some embodiments, the ring C is

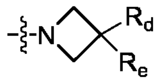

.

In some embodiments, the ring C is

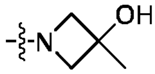

.

In some embodiments, $R_1$ is absent or is selected from fluoro, chloro, bromo, iodo, methyl, ethyl, n-propyl and isopropyl; in some embodiments, $R_1$ is absent or is selected from fluoro, chloro, methyl, ethyl, n-propyl, and isopropyl; in some embodiments, $R_1$ is absent or is selected from fluoro and methyl.

In some embodiments, $R_2$ is selected from $C_{4-6}$ alkyl, $C_{4-6}$ alkenyl, -$C_{1-3}$ alkylene-O-$C_{1-3}$ alkyl, and -$C_{1-3}$ alkylene-$NR_aR_b$; in some embodiments, $R_2$ is selected from $C_{4-6}$ alkyl and $C_{4-6}$ alkenyl; in some embodiments, $R_2$ is $C_{4-6}$ alkyl.

In some embodiments, $R_2$ is selected from n-butyl, isopentyl, $-CH_2CH_2OCH_3$, - $CH_2OCH_2CH_3$, $-CH_2CH_2OCH_2CH_3$, $-CH_2CH_2N(CH_3)_2$, $-CH_2CHCHCH_3$, $-CHCHCH_2CH_3$ and $-CH_2CHC(CH_3)_2$.

In some embodiments, $R_3$ is absent or is selected from fluoro, chloro, bromo, iodo, cyano, methyl, ethyl, n-propyl and isopropyl; in some embodiments, $R_3$ is absent or is selected from fluoro, chloro, cyano, methyl, ethyl, n-propyl and isopropyl; in some embodiments, $R_3$ is absent or is selected from fluoro and methyl.

In some embodiments, $R_c$ is selected from H, cyano, methyl, ethyl, n-propyl, isopropyl, cyclopropyl, phenyl, $-SO_2R_f$, and 1-$C_{1-3}$ alkyl-4-piperidinyl; in some embodiments, $R_c$ is selected from H, methyl, ethyl, n-propyl, isopropyl, cyclopropyl, phenyl, methylsulfonyl, cyclopropylsulfonyl and 1-methyl -4-piperidinyl; in some embodiments, $R_c$ is methyl.

In some embodiments, $R_d$ and $R_e$ are independently selected from H, fluoro, chloro, hydroxyl, cyano, $-NR_aR_b$, methyl, ethyl, n-propyl, isopropyl, cyclopropyl, phenyl, and 1-$C_{1-3}$ alkyl-4-piperidinyl; in some embodiments, $R_d$ and $R_e$ are independently selected from H, fluoro, $-N(CH_3)_2$, methyl, ethyl, n-propyl, isopropyl, cyclopropyl, phenyl and 1-methyl-4-piperidinyl; in some embodiments, $R_d$ and $R_e$ are independently selected from H, fluoro, $-N(CH_3)_2$ and methyl.

In some embodiments, Rε is selected from methyl, ethyl, n-propyl, isopropyl and cyclopropyl; in some embodiments, $R_f$ is selected from methyl and cyclopropyl.

In some embodiments, $R_a$ and $R_b$ are independently selected from H, methyl, ethyl, n-propyl and isopropyl; In some embodiments, $R_a$ and $R_b$ are independently selected from Hand methyl.

In some embodiments,

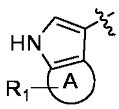 is 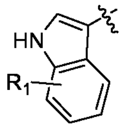, 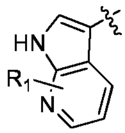, 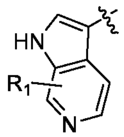, 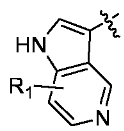 or

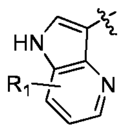

;

in some embodiments,

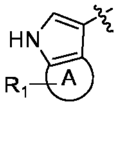

is preferably

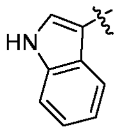, 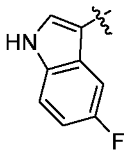 or 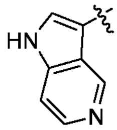;

In some embodiments,

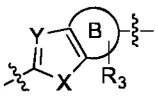 is 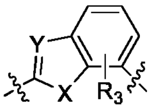, 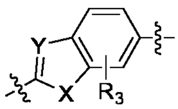, 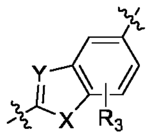,

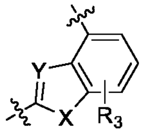, 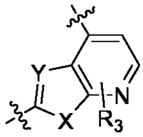, 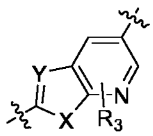, 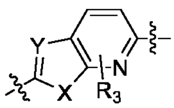, 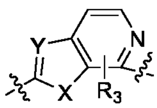, 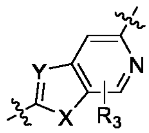,

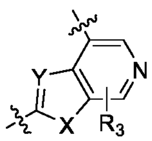, 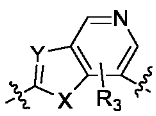, 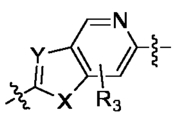, 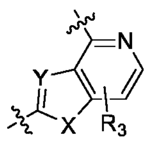, 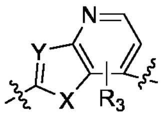, 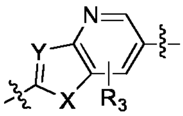,

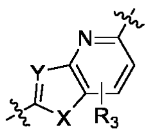, 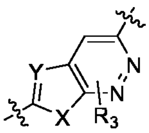, 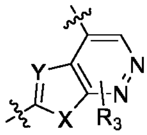, 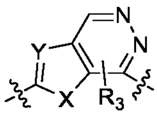, 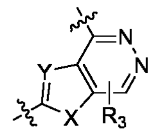, 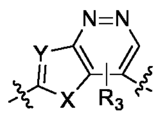,

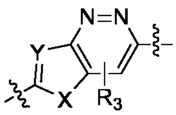, 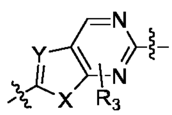, 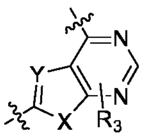, 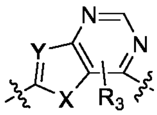, 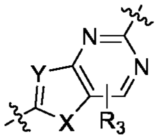, 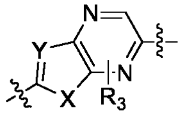 or

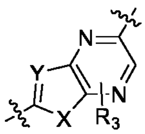;

in some embodiments,

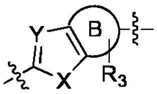

is preferably

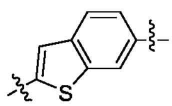, 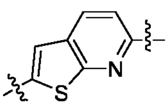,

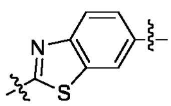, 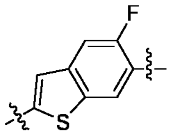, 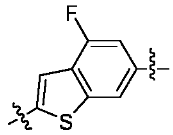, 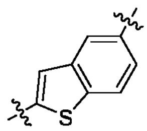, 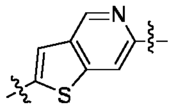, 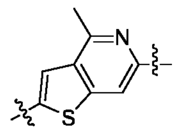,

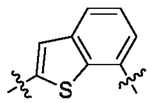, 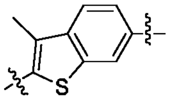, 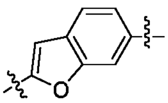, 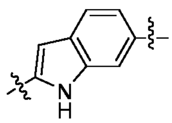, 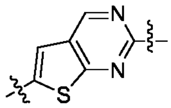 or 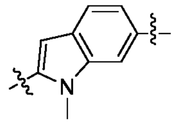.

In one embodiment of formula (I), the compound of formula (I) is the compound of formula (II) with the following structure,

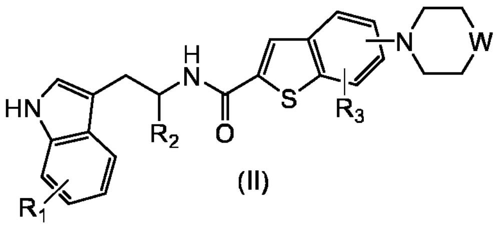

(II)

Wherein, $R_1$, $R_2$, $R_3$ and W are defined as in the formula (I).

In some embodiments,

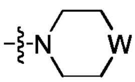

is selected from

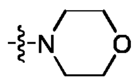, 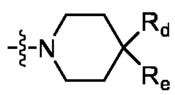

and

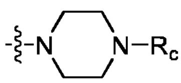;

in some embodiments, the ring C is

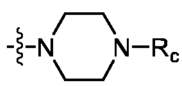.

In some embodiments,

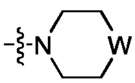

is selected from

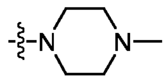, 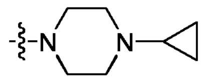,

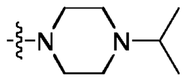, 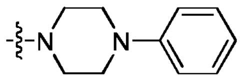, 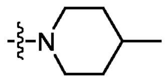, 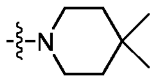, 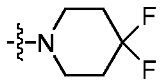, 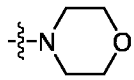,

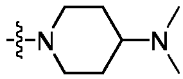, 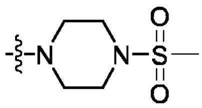, 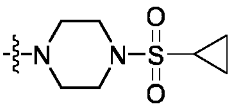, 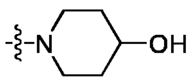 and 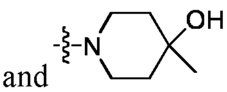.

In some embodiments, $R_1$ is absent or is selected from fluoro, chloro, bromo, iodo, methyl, ethyl, n-propyl and isopropyl; in some embodiments, $R_1$ is absent or is selected from fluoro, chloro, methyl, ethyl, n-propyl, and isopropyl; in some embodiments, $R_1$ is absent or is selected from fluoro and methyl.

In some embodiments, $R_2$ is selected from $C_{4-6}$ alkyl, $C_{4-6}$ alkenyl, -$C_{1-3}$ alkylene-O-$C_{1-3}$ alkyl, and -$C_{1-3}$ alkylene-$NR_aR_b$; in some embodiments, $R_2$ is selected from $C_{4-6}$ alkyl and $C_{4-6}$ alkenyl; in some embodiments, $R_2$ is $C_{4-6}$ alkyl.

In some embodiments, $R_2$ is selected from n-butyl, isopentyl, $-CH_2CH_2OCH_3$, - $CH_2OCH_2CH_3$, $-CH_2CH_2OCH_2CH_3$, $-CH_2CH_2N(CH_3)_2$, $-CH_2CHCHCH_3$, $-CHCHCH_2CH_3$ and $-CH_2CHC(CH_3)_2$. In some embodiments, $R_2$ is selected from n-butyl, $-CH_2CH_2OCH_3$ and - $CH_2CHC(CH_3)_2$.

In some embodiments, $R_3$ is absent or is selected from fluoro, chloro, bromo, iodo, cyano, methyl, ethyl, n-propyl and isopropyl; in some embodiments, $R_3$ is absent or is selected from fluoro, chloro, cyano, methyl, ethyl, n-propyl and isopropyl; in some embodiments, $R_3$ is absent or is selected from fluoro and methyl.

In some embodiments, $R_c$ is selected from H, cyano, methyl, ethyl, n-propyl, isopropyl, cyclopropyl, phenyl, $-SO_2R_f$, and 1-$C_{1-3}$ alkyl-4-piperidinyl; in some embodiments, $R_c$ is selected from H, methyl, ethyl, n-propyl, isopropyl, cyclopropyl, phenyl, methylsulfonyl, cyclopropylsulfonyl and 1-methyl -4-piperidinyl; in some embodiments, $R_c$ is methyl.

In some embodiments, $R_d$ and $R_e$ are independently selected from H, fluoro, chloro, hydroxyl, cyano, $-NR_aR_b$, methyl, ethyl, n-propyl, isopropyl, cyclopropyl, phenyl, and 1-$C_{1-3}$ alkyl-4-piperidinyl; in some embodiments, $R_d$ and $R_e$ are independently selected from H, fluoro, $-N(CH_3)_2$, methyl, ethyl, n-propyl, isopropyl, cyclopropyl, phenyl and 1-methyl-4-piperidinyl; in some embodiments, $R_d$ and $R_e$ are independently selected from H, fluoro, $-N(CH_3)_2$ and methyl.

In some embodiments, Rε is selected from methyl, ethyl, n-propyl, isopropyl and cyclopropyl; in some embodiments, $R_f$ is selected from methyl and cyclopropyl.

In some embodiments, $R_a$ and $R_b$ are independently selected from H, methyl, ethyl, n-propyl and isopropyl; In some embodiments, $R_a$ and $R_b$ are independently selected from Hand methyl.

In some embodiments of formula (I) or formula (II), the compound of the invention is selected from:

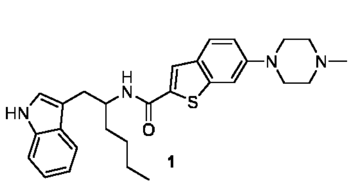

1

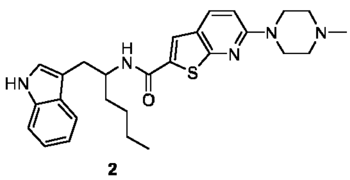

2

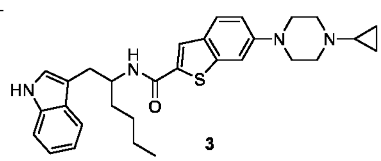

3

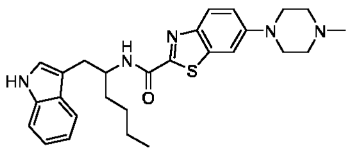

4

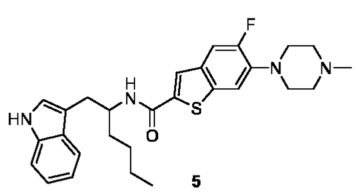

5

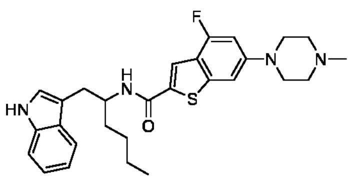

6

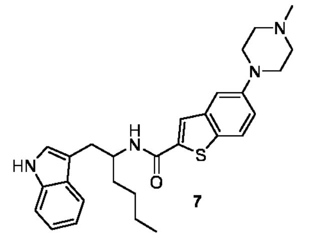

7

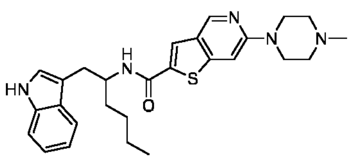

8

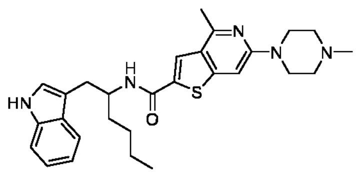

9

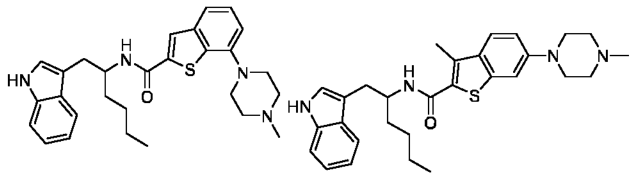

10

11

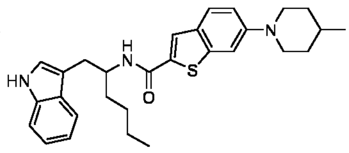

12

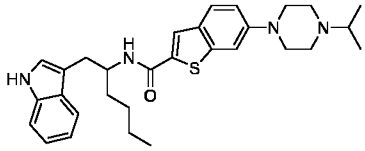

13

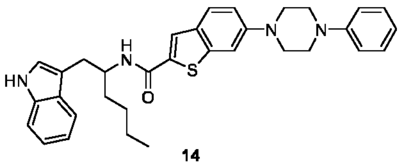

14

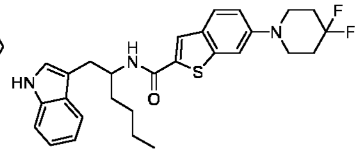

15

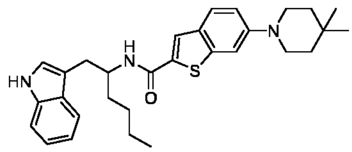

16

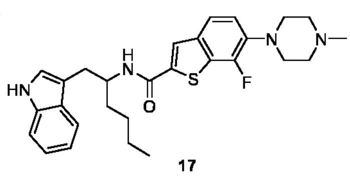

17

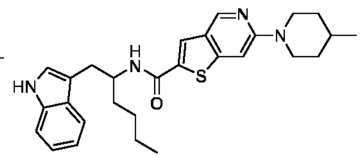

18

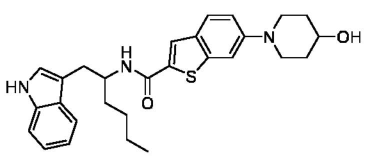

19

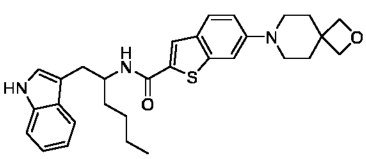

20

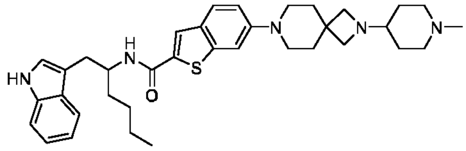

21

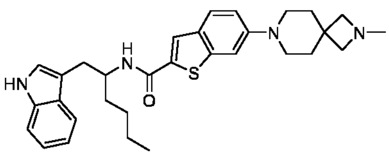

22

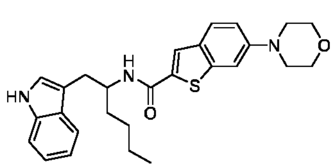

23

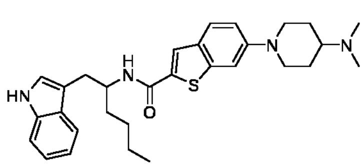

24

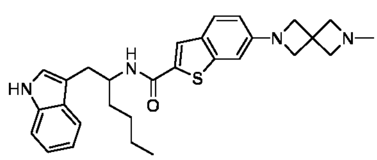

25

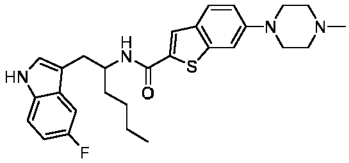

26

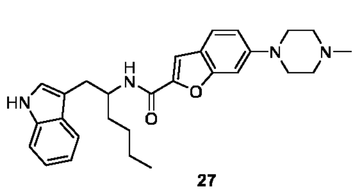

27

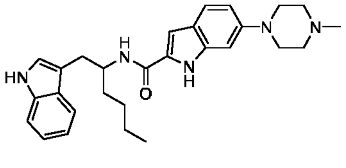

28

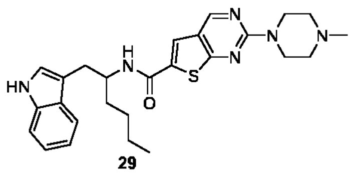

29

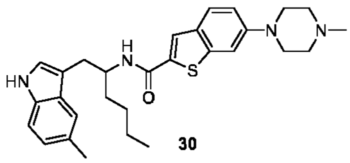

30

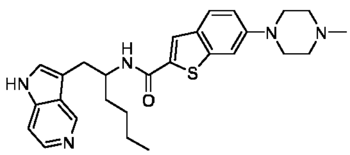

31

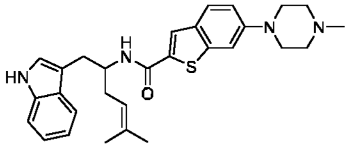

32

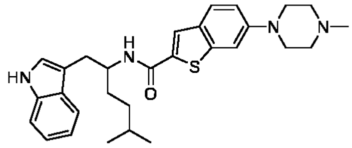

33

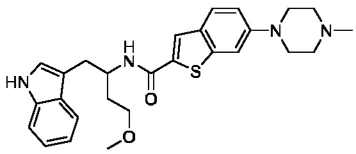

34

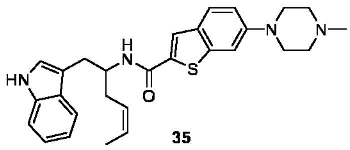

35

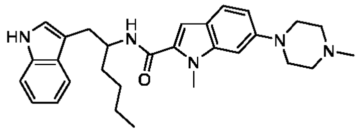

36

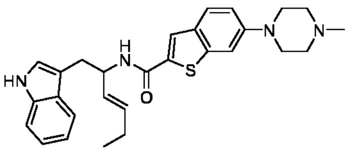

37

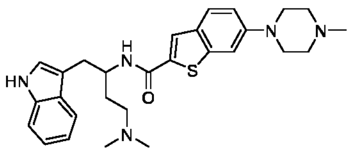

38

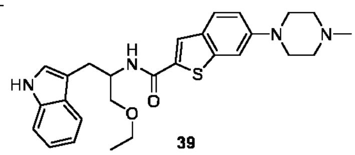

39

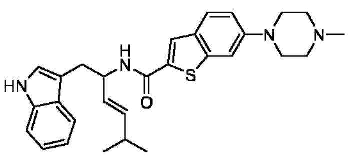

40

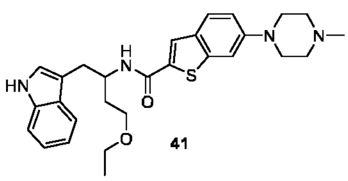

41

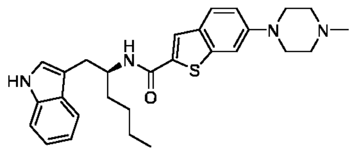

42

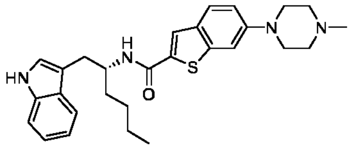

43

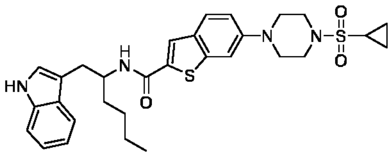

44

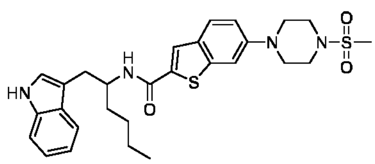

45

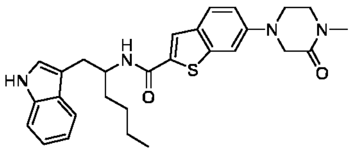

46

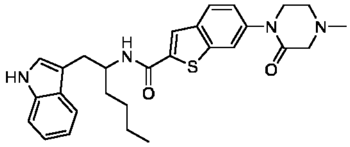

47

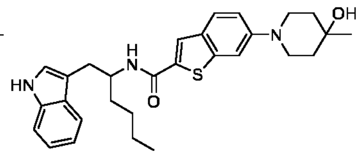

48

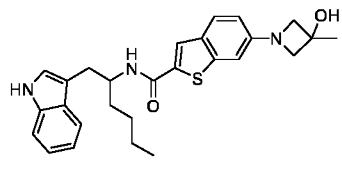

49

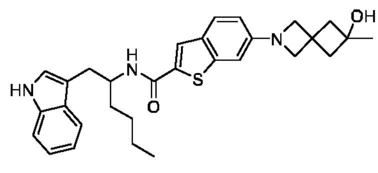

50

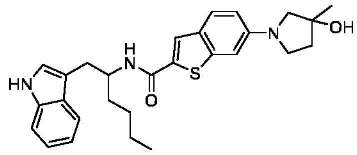

51

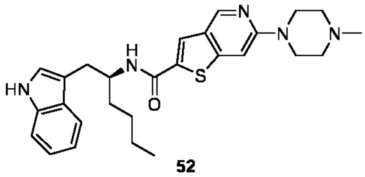

52

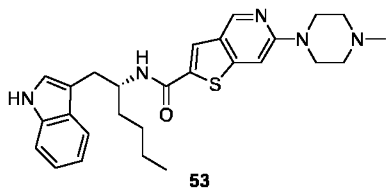

53

.

In the compounds of the invention (including the general formula and the above-mentioned specific compounds), the carbon atom connected to the substituent $R_2$ may be a chiral carbon atom, specifically an R configuration, an S configuration, or a mixture thereof.

In addition to the free base form of the compound described herein, the salt of the compound is also within the scope of the invention. The salt or pharmaceutically acceptable salt of the compound described herein can be prepared in situ during the final separation and purification process of the compound, or by separately reacting the purified compound in its free base form with appropriate bases or acids.

For the compound of formula (I) containing basic groups, pharmaceutically acceptable acid addition salt can be formed by treatment with a suitable acid. Suitable acid include pharmaceutically acceptable inorganic acid and pharmaceutically acceptable organic acid.

In the invention, the compound of formula (I) or salt thereof may exist in stereoisomeric forms (e.g., they may contain one or more asymmetric carbon atoms). Individual stereoisomers (enantiomers and diastereomers) and their mixtures are included within the scope of the invention. Different isomers can be separated or split from each other through conventional methods, or any given isomer can be obtained through conventional synthesis methods or through stereospecific or asymmetric synthesis.

In the invention, the compound of formula (I) can exist in a tautomeric form. For example, some compounds exhibit ketone-enol tautomerism. In some cases, only one of a pair of tautomeric forms is in formula (I). These alternative tautomers also constitute a part of the invention.

The invention also includes isotope-labeled compounds and salts, which are the same as the compound of formula (I) or salt thereof, except that one or more atoms are replaced by atoms having an atomic mass or mass number different from the most common atom in nature. Examples of isotopes of hydrogen, carbon, nitrogen, and fluorine that can be incorporated into the compound of formula (I) or salt thereof, such as $^{2}H$, $^{3}H$, $^{11}C$, $^{14}C$ and $^{18}F$. Such isotopically labeled compound of formula (I) or salt thereof can be used for drug and/or substrate tissue distribution determination. For example, $^{11}C$ and $^{18}F$ isotopes are used in PET (positron emission tomography). PET is used for brain imaging. Isotope-labeled compound of formula (I) and salt thereof can be prepared by disclosed operations, replacing non-isotopically labeled reagents with readily available isotope-labeled reagents. In some embodiments, the compound of formula (I) or salt thereof may not be isotopic labeled.

**Pharmaceutical Compositions**

The compound of the invention or its pharmaceutically acceptable salt can be formulated as a pharmaceutical composition before administration to the subject. Therefore, in another aspect, the invention provides a pharmaceutical composition comprising a compound of formula (I) or a pharmaceutically acceptable salt thereof, and optionally comprising one or more pharmaceutically acceptable excipients.

**Treatment Methods and** Uses

In another aspect, the invention provides a method for treating neurodegenerative diseases associated with protein aggregation, comprising administering a therapeutically effective amount of at least the compound of formula (I) or a pharmaceutically acceptable salt thereof to subjects in need.

In another aspect, the present provides a method for treating neurodegenerative diseases related to protein aggregation, wherein the method comprises administering a pharmaceutical composition to subjects in need, comprising

a therapeutically effective amount of the compound of formula (I) or a pharmaceutically acceptable salt thereof, and one or more pharmaceutically acceptable excipients.

Exemplary neurodegenerative diseases characterized by protein aggregation include Alzheimer's disease, Parkinson's disease, frontotemporal dementia, Lewy body disease (Lewy body dementia), Parkinson's disease dementia, multiple system atrophy, amyotrophic lateral sclerosis, Huntington's disease, and cancer (e.g., melanoma).

The compounds, compositions, and methods of the invention can also be used to mitigate deleterious effects secondary to protein aggregation, such as neuronal cell death.

In some aspects, the compounds, compositions, and methods of the invention are used to target $\alpha$-synuclein aggregation.

In the inhibitory method of the invention, "effective amount" refers to an amount sufficient to reduce, delay the progression of protein or peptide aggregation, or reverse protein or peptide aggregation.

In the treatment method according to the invention, "effective amount" refers to an amount or dosage sufficient to obtain the desired therapeutic benefit to subjects in need of such treatment.

In another aspect, the invention provides the use of the compound of formula (I) or pharmaceutically acceptable salt thereof in the preparation of a medicament for the treatment of neurodegenerative diseases associated with protein aggregation.

In some embodiments, the invention provides the compound of formula (I) or a pharmaceutically acceptable salt thereof in the preparation of a medicament for treating a neurodegenerative disease associated with the aggregation of $\alpha$-synuclein.

In some embodiments, the invention provides the use of the compound of formula (I) or pharmaceutically acceptable salt thereof in the preparation of a medicament for Alzheimer's disease, Parkinson's disease, frontotemporal dementia, Lewy body disease (Lewy body dementia), Parkinson's disease dementia, multiple system atrophy, amyotrophic lateral sclerosis, or Huntington's disease.

In one embodiment, the invention provides the use of the compound of formula (I) or a pharmaceutically acceptable salt thereof in the preparation of a medicament for Parkinson's disease.

**Combination therapy**

The compound of the invention can be used in combination with one or more other active ingredients used in the treatment of neurodegenerative diseases, for pharmaceutical compositions or methods. Other active ingredients can be administered in the form of pharmaceutical combinations independent of the compounds of the invention, or can be included in a single pharmaceutical combination with the compounds of the invention. Other active ingredients can be administered simultaneously with, before or after the compound of the invention.

**Examples**

The invention is further illustrated below by way of examples, but the invention is not limited to the scope of the examples described. The experimental methods in the following examples that do not specify specific conditions shall be selected according to conventional methods and conditions, or according to the product instructions. In addition, those skilled in the art will recognize that the following synthesis reactions and schemes can be modified by selecting appropriate starting materials and reagents to obtain the compounds of the present invention.

The following abbreviations are used in the Examples and Biological Examples:

Xanthos: 4,5-bis(diphenylphosphino)-9,9-dimethyloxanthracene
HATU: 2- (7-azobenzotriazole) - N, N, N', N' - Tetramethyluronium hexafluorophosphate
DIEA: N, N-diisopropylethylamine
DMF: N, N-dimethylformamide
MeOH: Methanol
PE: Petroleum ether
EA: Ethyl acetate
DCM: Dichloromethane
LCMS: Liquid chromatography-mass spectrometry
DMSO: Dimethyl sulfoxide
DMSO-d6: Deuterated dimethyl sulfoxide
Chloroform-d: deuterated chloroform
Methanol-d4: deuterated methanol
SFC: Supercritical Fluid Chromatography
TLC: Thin layer chromatography

HPLC: High performance liquid chromatography.

In the preparative HPLC purification method of the examples, the chromatographic column is Xbridge Prep C18 column OBD (10 μ m. 19x250mm); The mobile phase is 0.1% ammonia aqueous solution/acetonitrile.

Example 1 N-(1-(1H-indol-3-yl) hexane-2-yl)-6-(4-methylpiperazin-1-yl)benzo[b]thiohene-2-carboxamide

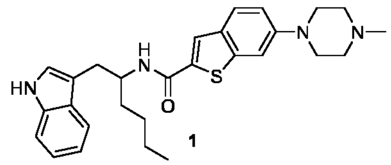

1

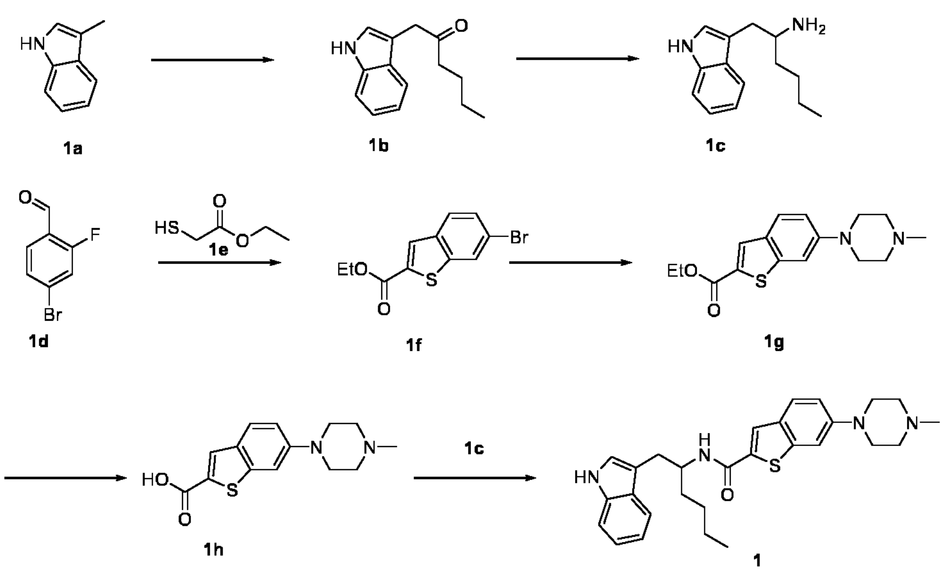


1a 1b 1c

1d 1f 1g

1h 1

Step 1: 1- (1H indole-3-yl) hexane-2-one 1b

Compound 1a (40 g, 304.9 mmol) was dissolved in 1,2-dichloroethane (2000 mL), aluminum chloride (81.3 g, 609.8 mmol) was added at 0°C, then valeryl chloride (44.1 g, 365.9 mmol) was added after stirring at room temperature for 0.5 hours, and the reaction mixture was stirred at 15°C for 14 hours. Water (300 mL) was added to the reaction mixture to quench the reaction, and it was extracted with ethyl acetate (300 mL×3), and the combined organic phase was washed with saturated salt water (300 mL×2), dried with anhydrous sodium sulfate, filtered, and concentrated to obtain a crude product 1b (71 g), which was directly used in the next step.

Step 2: 1-(1H-indol-3-yl) hexane-2-amine 1c

Compound 1b (71 g, 329.7 mmol) was dissolved in methanol (1000 mL), and then ammonium acetate (77.08 g, 1000 mmol) and sodium cyanoborohydride (31.08 g, 494.6 mmol) were added at room temperature, and the reaction mixture was stirred at 70°C for 5 hours. After the reaction mixture was cooled, water (300 mL) was added to quench the reaction, and it was extracted with ethyl acetate (300 mL×3), and the combined organic phase was washed with saturated salt water (300 mL×2), dried with anhydrous sodium sulfate, filtered, and concentrated to obtain a crude product, which was purified by silica gel column chromatography (PE:EA= 1:1) to obtain compound 1c (31 g, yield 43.5%).

Step 3: ethyl 6-bromobenzo[b]thiophene-2-carboxylate 1f

Compound 1d (2.0 g, 9.85 mmol) was dissolved in acetonitrile (10 mL), potassium carbonate (2.04 g 14.76 mmol) and compound 1e (1.42 g, 11.82 mmol) were added at room temperature, and the reaction mixture was stirred at 80°C for 1 hour. The reaction mixture was filtered to remove potassium carbonate, and the filtrate was concentrated

to obtain a crude product, which was purified by silica gel column chromatography (PE: EA=5:1) to obtain compound 1f (2.2 g, yield 78.3%).

Step 4: ethyl 6-(4-methylpiperazin-1-yl)-1-benzothiophene-2-carboxylate 1g

Compound 1f (0.5g, 1.753mmol) was dissolved in 1,4-dioxane (10mL), 1-methylpiperazine (0.264g, 2.63mmol), palladium acetate (0.02g, 0.089mmol), Xantphos (0.047g, 0.081mmol) and cesium carbonate (1.713g, 5.26mmol) were added at room temperature, and the reaction mixture was stirred at 110°C for 2 hours. After the reaction mixture was cooled, water (100mL) was added to quench the reaction, and it was extracted with ethyl acetate (30mL×3), and the combined organic phase was washed with saturated salt water (30mL×2), dried with anhydrous sodium sulfate, filtered, concentrated, and purified by silica gel column chromatography (PE:EA= 1:1) to obtain compound 1g (0.41g, yield 76.8%).

Step 5: 6-(4-Methylpiperazin-1-yl)-1-benzothiophene-2-carboxylic acid 1h

Compound 1g (0.41g, 1.347mmol) was dissolved in tetrahydrofuran (10mL) and water (10mL), sodium hydroxide (0.16g, 4.00mmol) was added at room temperature, and the reaction mixture was stirred at 70°C for 1 hours. the reaction mixture was adjusted the pH to 3-4 with dilute hydrochloric acid, There are many solids precipitate. Filterd and collected the solids to obtain compound 1h (0.32g, yield 86%).

Step 6: N-(1-(1H-indol-3-yl) hexane-2-yl)-6-(4-methylpiperazin-1-yl) benzo[b]thiophene-2-carboxamide 1

Compound 1c (1.0 g, 4.622 mmol) was dissolved in DMF (10 mL), and compound 1h (1.40 g, 5.07 mmol), triethylamine (2.34 g, 23.1 mmol), and 1-propylphosphonic anhydride (2.2 g, 6.91 mmol) were added at room temperature. After the reaction mixture was stirred at room temperature for 1 hour, water (50 mL) was added to quench the reaction, and the mixture was extracted with ethyl acetate (30 mL × 3). The combined organic phase was washed with saturated salt water (30 mL × 2), dried with anhydrous sodium sulfate, filtered, and concentrated to obtain a crude product, which was purified by silica gel column chromatography (PE: EA = 1: 1) to obtain compound 1 (1200 mg, yield 54.7%).

$^1H$ NMR (400 MHz, Chloroform-d) δ 8.10 (s, 1H), 7.64 (dd, *J* = 27.7, 8.4 Hz, 2H), 7.47-7.32 (m, 2H), 7.25 (d, *J* = 11.6 Hz, 3H), 7.12 (ddd, *J* = 8.1, 7.0, 1.0 Hz, 1H), 7.09-7.03 (m, 2H), 5.85 (d, *J* = 8.8 Hz, 1H), 4.56-4.38 (m, 1H), 3.42-3.22 (m, 4H), 3.16-3.01 (m, 2H), 2.61 (t, *J* = 5.0 Hz, 4H), 2.37 (s, 3H), 1.66-1.28 (m, 3H), 0.87 (t, *J* = 7.1 Hz, 3H).

MS m/z (ESI):475.5 $[M+H]^+$.

Example 2 N-[1-(1H-indol-3-yl) hexyl-2-yl]-6-(4-methylpiperazin-1-yl) thiophene[2,3-b] pyridine-2-carboxamide

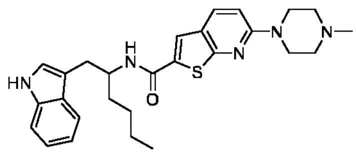

2

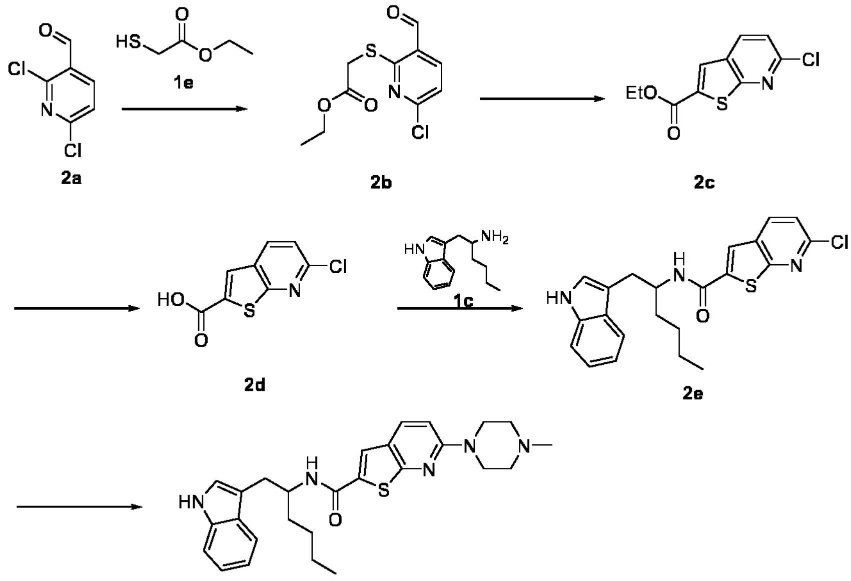

1e 1c

2a 2b 2c 2d 2e 2

Step 1: 2-[(6-chloro-3-formylpyridin-2-yl) mercapto] ethyl acetate 2b

2,6-dichloropyridine-3-carboxaldehyde 2a (800 mg, 4.60 mmol) was dissolved in dichloromethane (20 mL), triethylamine (0.7 mL, 4.60 mmol) was added, and then compound 1e (600 mg, 4.60 mmol) was added. The reaction mixture was stirred at room temperature for 2 hours, and the reaction was completed. The reaction mixture was purified by silica gel column chromatography (PE: EA=2:1) to obtain compound 2b (120 mg, yield 9.24%).
MS m/z (ESI):260.0 $[M+H]^+$.

Step 2: ethyl 6-Chlorothiophene[2,3-b] pyridine-2-carboxylate 2c

2-[(6-chloro-3-formylpyridin-2-yl) mercapto] ethyl acetate 2b (120 mg, 0.5 mmol) was dissolved in DMF (2.5 mL), potassium carbonate (60 mg, 0.5 mmol) was added, and the reaction was carried out at 70°C for 3 hours. The reaction was completed. After the reaction mixture was diluted with ethyl acetate (50 mL), the organic phase was washed with saturated salt water (30 mL × 2), dried with anhydrous sodium sulfate, and the crude product obtained after concentration was purified by silica gel column chromatography (PE: EA = 2: 1) to obtain compound 2c (88 mg, yield 78.8%).
MS m/z (ESI): 242.2 $[M+H]^+$.

Step 3: 6-Chlorothiophene[2,3-b] pyridine-2-carboxylic acid 2d

Ethyl 6-Chlorothiophene[2,3-b] pyridine-2-carboxylate 2c (88 mg, 0.365 mmol) was dissolved in tetrahydrofuran (10 mL), sodium hydroxide (80 mg, 2.19 mmol) was dissolved in water (5 mL) and added to the reaction mixture, heated to 60°C for 3 hours. After the reaction was completed, the reaction mixture was concentrated to remove tetrahydrofuran, and the pH was adjusted to 6 with 1M hydrochloric acid. The solid was precipitated, filtered and dried to obtain compound 2d (80 mg), with a yield of 98.2%.
MS m/z (ESI): 214.2 $[M+H]^+$.

Step 4: 6-Chloro-N-[1-(1H-indol-3-yl) hexyl-2-yl] thiophene[2,3-b] pyridine-2-carboxamide 2e

1-(1H-indol-3-yl) hexyl-2-amine (96 mg, 0.376 mmol) and 6-chlorothiophene[2,3-b] pyridine-2-carboxylic acid (80 mg, 0.376 mmol) were mixed in DMF (2 mL), DIEA (0.13 mL) was added, and then HATU (143 mg, 0.376 mmol) was added. The mixture was stirred at room temperature for 3 hours. The reaction mixture was diluted with ethyl acetate (50 mL), and the organic phase was washed with saturated salt water (30 mL×2), dried, concentrated, and purified by silica gel column chromatography (PE:EA= 1:1) to obtain compound 2e (140 mg, yield 91%).
MS m/z (ESI): 413.1 $[M+H]^+$.

Step 5: N-[1-(1H-indol-3-yl) hexyl-2-yl]-6-(4-methylpiperazin-1-yl) thiophene[2,3-b] pyridine-2-carboxamide 2

6-Chloro-N-[1-(1H-indol-3-yl) hexyl-2-yl] thiophene[2,3-b] pyridine-2-carboxamide 2e (70 mg, 0.170 mmol) was dissolved in 1,4-dioxane (2.5 mL), 1-methylpiperazine (0.3 mL) was added, and then placed in a microwave tube. After microwave heating at 150°C for 2 hours, the reaction was completed. After the reaction mixture was concentrated, it was diluted with ethyl acetate (50 mL), washed with water (30 mL×2) and dried. After concentration, it was purified by silica gel column chromatography (PE:EA= 1:1) to obtain compound 2 (45 mg, yield 55.7%).

$^1$H NMR (400MHz, Chloroform-d) δ: 8.09(s, 1H), 7.78-7.72(d, $J$ = 8 Hz, 1H), 7.70-7.64(d, $J$ = 8 Hz, 1H), 7.42-7.35(m, 2H), 7.24-7.17(m, 1H), 7.16-7.10(m, 1H), 7.09-7.05(m, 1H), 6.75-6.69(m, 1H), 5.83-5.73(m, 1H), 4.52-4.42(m, 1H),3.75-3.65(m, 4H), 3.13-3.04(m, 2H), 2.59-2.50(m, 4H), 2.36(s, 3H), 1.54-1.29(m, 6H), 0.88(t, $J$ = 7.2 Hz, 3H).

MS m/z (ESI): 476.3 [M+H]$^+$.

Example 3 N-(1-(1H-indol-3-yl) hexane-2-yl)-6-(4-cyclopropylpiperazin-1-yl) benzo[b]-thiophene-2-carboxamide

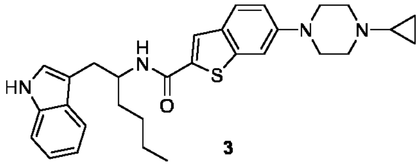

3

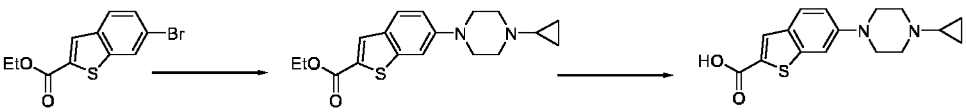

1f 3a 3b

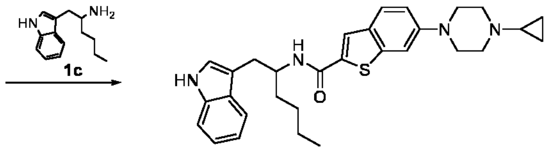


3

Step 1: ethyl 6-(4-cyclopropylpiperazine-1-yl) benzo[b]thiophene-2-carboxylate 3a

Compound 1f (1000 mg, 3.5 mmol), palladium acetate (40 mg, 0.2 mmol), Xantphos (202 mg, 0.35 mmol), cesium carbonate (3.42 g, 10.5 mmol) were added to the reaction bottle, 1-cyclopropylpiperazine (674 mg, 5.3 mmol) was dissolved in 1,4-dioxane (10 mL), nitrogen protection, stirring was started, and the reaction was carried out at 100°C for 3 hours. After the reaction was completed, water (50 mL) was added to quench the reaction, it was extracted with ethyl acetate (50 mL×3), the organic layers were combined, dried with anhydrous sodium sulfate, concentrated, and purified by silica gel column chromatography (PE:EA = 5:1) to obtain compound 3a (450 mg, yield 41%).

MS m/z (ESI):331.2[M+H]$^+$.

Step 2: 6-(4-cyclopropylpiperazine-1-yl) benzo[b]thiophene-2-carboxylic acid 3b

Compound 3a (450 mg, 2.0 mmol) was dissolved in 5 mL of tetrahydrofuran, and then 5 mL of methanol was added. Sodium hydroxide was dissolved in 3 mL of water and added to the reaction bottle, and stirring was started. The reaction was carried out at 70°C for 1 hour. After the reaction, the solvent was dried by rotary evaporation, the solid was dissolved in water, and the pH was adjusted to 5-6 with 1M hydrochloric acid. The filter cake obtained by filtration was dissolved in methanol (50 mL), and the solvent was evaporated to obtain compound 3b (380 mg, yield 92%).

MS m/z (ESI):303.2[M+H]$^+$.

Step 3: N-(1-(1H-indol-3-yl) hexane-2-yl)-6-(4-cyclopropylpiperazine-1-yl) benzo[b]thiophene -2-carboxamide 3

Compound 3b (308 mg, 1.1 mmol) and compound 1c (200 mg, 0.9 mmol) were added to the reaction bottle, 5mL of DMF was added to dissolve, and triethylamine (467 mg, 4.6 mmol) and 1-propylphosphoric acid cyclic anhydride (441 mg, 1.4 mmol) were added, stirring was started, and the reaction was carried out at room temperature for 1 hour. After the reaction was completed, water (50 mL) was added to quench the reaction, and ethyl acetate (50 mL × 3) was used for extraction. The organic layers were combined, dried with anhydrous sodium sulfate, and concentrated. Compound 3 (125 mg, yield 25%) was purified and separated by preparative HPLC.

MS m/z (ESI):503.3[M+H]$^+$.

Example 4 N-(1-(1H-indol-3-yl) hexane-2-yl)-6-(4-methylpiperazin-1-yl) benzo[d]thiazole-2-carboxamide

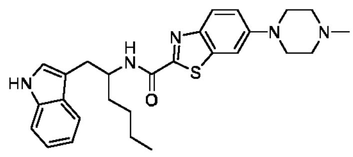

4

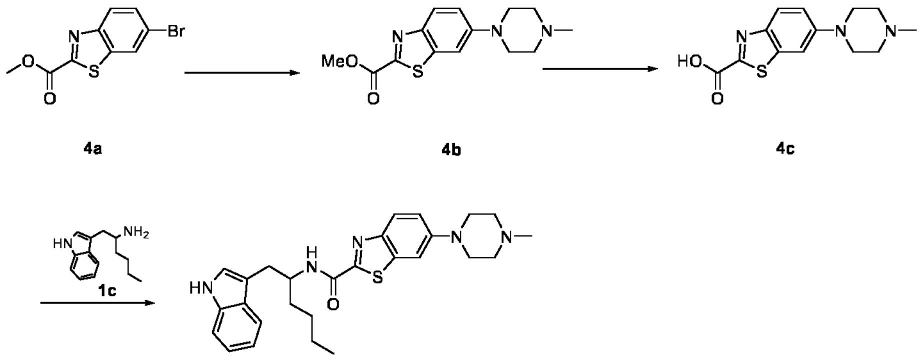

4a 4b 4c

1c

4

Step 1: ethyl 6-(4-methylpiperazin-1-yl) benzo[d]thiazole-2-carboxylate 4b

Compound 4a (200 mg, 3.5 mmol), palladium acetate (40 mg, 0.2 mmol), Xantphos (202 mg, 0.35 mmol), cesium carbonate (3.42 g, 10.5 mmol) were added to the reaction bottle, 1-methylpiperazine (674 mg, 5.3 mmol) was dissolved in 1,4-dioxane (10 mL), nitrogen protection, stirring was started, and the reaction was carried out at 100°C for 3 hours. After the reaction was completed, water (50 mL) was added to quench the reaction, it was extracted with ethyl acetate (50 mL × 3), the organic layers were combined, dried with anhydrous sodium sulfate, concentrated, and purified by silica gel column chromatography (PE: EA = 1: 1) to obtain compound 4b (180 mg, yield 75%).

MS m/z (ESI):292.2[M+H]$^+$.

Step 2: 6-(4-methylpiperazine-1-yl) benzo[d]thiazole-2-carboxylic acid 4c

Compound 4b (180 mg, 1.5 mmol) was dissolved in 3 mL of tetrahydrofuran, and then 3 mL of methanol was added. Sodium hydroxide was dissolved in water (3 mL) and added to the reaction bottle, and stirring was started. The reaction was carried out at 70°C for 1 hour. After the reaction, the solvent was dried by rotary evaporation, the solid was dissolved in water, and the pH was adjusted to 5-6 with 1M hydrochloric acid, and the filter cake was dissolved in methanol (20 mL). The solvent was evaporated to obtain compound 4c (120 mg, yield 75%).

MS m/z (ESI):278.2[M+H]$^+$.

Step 3: N-(1-(1H-indol-3-yl) hexane-2-yl)-6-(4-methylpiperazine-1-yl) benzo[d]thiazole-2-carboxamide 4

Compound 4c (120 mg, 1.1 mmol) and compound 1c (95 mg, 0.89 mmol) were added to the reaction bottle, 5 mL DMF was added to dissolve, and triethylamine (467 mg, 4.6 mmol) and 1-propylphosphoric acid cyclic anhydride

(441 mg, 1.4 mmol) were added, and stirring was started. The reaction was reacted at room temperature for 1 hour. After the reaction was completed, water (50 mL) was added to quench the reaction, and it was extracted with ethyl acetate (50 mL×3). The organic layers were combined, dried with anhydrous sodium sulfate, and concentrated to obtain a crude product. The crude product was purified by preparative HPLC to obtain compound 4 (42 mg, yield 35%).

$^1$H NMR (400 MHz, Chloroform-d) δ 8.06 (s, 1H), 7.85 (d, J = 9.3 Hz, 1H), 7.69 (d, J = 7.9 Hz, 1H), 7.41-7.31 (m, 3H), 7.25-7.07 (m, 4H), 4.47 (s, 1H), 3.33 (d, J = 5.6 Hz, 4H), 3.18- 3.00 (m, 2H), 2.62 (d, J = 5.2 Hz, 4H), 2.38 (s, 3H), 1.48-1.22 (m, 6H), 0.92-0.82 (m, 3H)。

MS m/z (ESI):476.5 [M+H]$^+$.

Example 5 N-(1-(1H-indol-3-yl) hexane-2-yl)-5-fluoro-6-(4-methylpiperazin-1-yl) benzo[b]thiophene-2-carboxamide

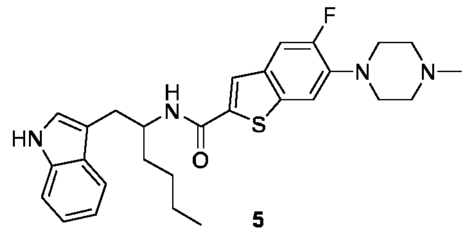

5

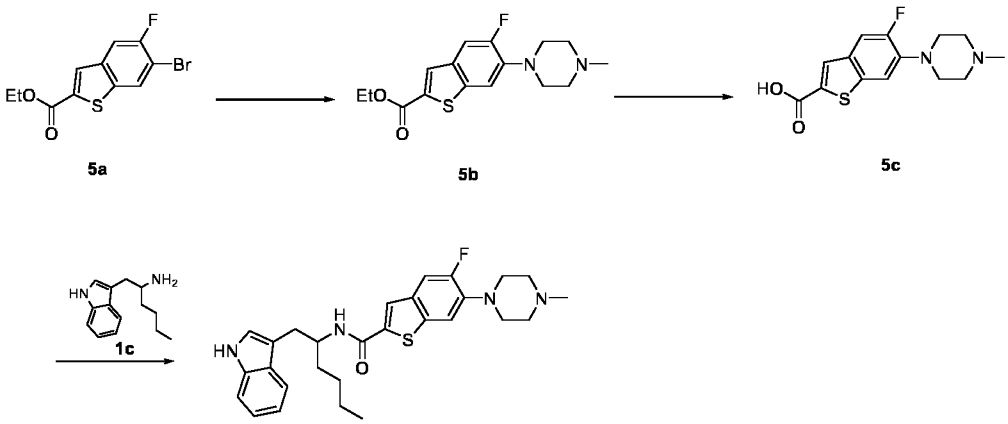

5a 5b 5c 1c 5

Step 1: ethyl 5-fluoro-6-(4-methylpiperazin-1-yl) benzo[b]thiophene-2-carboxylate 5b

Compound 5a (500 mg, 1.65 mmol), palladium acetate (40 mg, 0.2 mmol), Xantphos (202 mg, 0.35 mmol), cesium carbonate (3.42 g, 10.5 mmol) were added to the reaction bottle, 1-methylpiperazine (674 mg, 5.3 mmol) was dissolved in 1,4-dioxane (10 mL), nitrogen protection, stirring was started, and the reaction was carried out at 100°C for 3 hours. After the reaction was completed, water (50 mL) was added to quench the reaction, it was extracted with ethyl acetate (50 mL×3), the organic layers were combined, dried with anhydrous sodium sulfate, concentrated, and purified by silica gel column chromatography (PE:EA = 1: 1) to obtain compound 5b (460 mg, yield 89%).

MS m/z (ESI):323.4[M+H]$^+$.

Step 2: 5-Fluoro-6-(4-methylpiperazine-1-yl) benzo[b]thiophene-2-carboxylic acid 5c

Compound 5b (200 mg, 0.62 mmol) was dissolved in tetrahydrofuran (2 mL), and then 2 mL of methanol was added. Sodium hydroxide was dissolved in water (2 mL) and added to the reaction bottle, and stirring was started. The reaction was reacted at 70°C for 1 hour. The reaction mixture was dried by rotary evaporation, the solid was dissolved in water, and the pH was adjusted to 5-6 with 1M hydrochloric acid, filtered, and the filter cake was dissolved in methanol (20 mL). The solvent was evaporated to obtain compound 5c (130 mg, yield 78%).

MS m/z (ESI):295.3[M+H]$^+$.

Step 3: N-(1-(1H-indol-3-yl) hexane-2-yl)-5-fluoro-6-(4-methylpiperazine-1-yl) benzo[b]thiophene-2-carboxamide 5

Compound 5c (130 mg, 0.44 mmol) and compound 1c (100 mg, 0.5 mmol) were added to a reaction bottle, and DMF (5 mL) was added to dissolve, and triethylamine (467 mg, 4.6 mmol) and 1-propylphosphoric acid cyclic anhydride (351 mg, 1.0 mmol) were added, and stirring was started. The reaction was allowed to react at room temperature for 1 hour. After the reaction was completed, water (50mL) was added to quench the reaction, ethyl acetate (50 mL × 3) was used for extraction, The organic layers were combined, dried with anhydrous sodium sulfate, and concentrated to obtain a crude product, which was purified by silica gel column chromatography (PE:EA= 1: 1) to obtain compound 5 (25mg, yield 35%).

MS m/z (ESI):493.5 $[M+H]^+$.

Example 6 N-(1-(1H-indol-3-yl) hexane-2-yl)-4-fluoro-6-(4-methylpiperazin-1-yl) benzo[b]thiophene-2-carboxamide

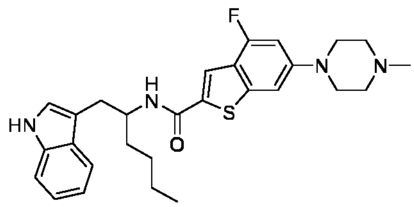

6

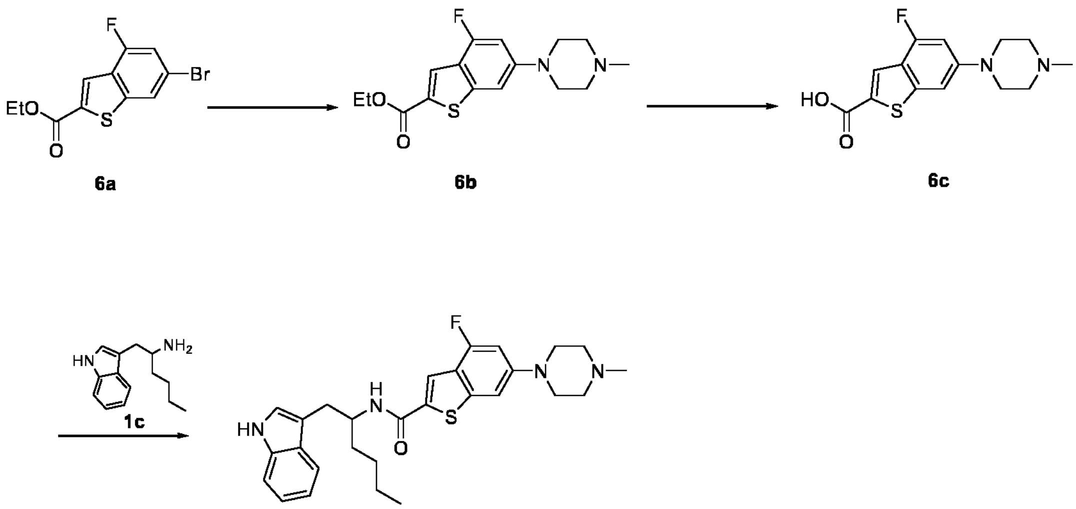

6a 6b 6c

1c

6

Step 1: ethyl 4-fluoro-6-(4-methylpiperazine-1-yl) benzo[b]thiophene-2-carboxylate 6b

Compound 6a (500 mg, 1.65 mmol), palladium acetate (40 mg, 0.2 mmol), Xantphos (202 mg, 0.35 mmol), cesium carbonate (3.42 g, 10.5 mmol) were added to the reaction bottle, 1-methylpiperazine (674 mg, 5.3 mmol) was dissolved with 1,4-dioxane (10 mL), nitrogen protection, stirring was started, and the reaction was carried out at 100°C for 3 hours. After the reaction was completed, water (50 mL) was added to quench the reaction, it was extracted with ethyl acetate (50 mL×3), the organic layers were combined, dried with anhydrous sodium sulfate, concentrated, and compound 6b was purified by silica gel column chromatography (PE: EA=5:1) (420 mg, yield 84%).

MS m/z (ESI):323.4$[M+H]^+$.

Step 2: 4-Fluoro-6-(4-methylpiperazine-1-yl) benzo[b]thiophene-2-carboxylic acid 6c

Compound 6b (200 mg, 0.62 mmol) was dissolved in 2 mL of tetrahydrofuran, and then 2 mL of methanol was added. Sodium hydroxide was dissolved in water (2 mL) and added to the reaction bottle, stirred, and reacted at 70°C for 1 hour. The reaction mixture was dried by rotary evaporation, the solid was dissolved in water, and the pH was adjusted to 5-6 with 1 M hydrochloric acid, filtered, and the filter cake was dissolved in methanol (20 mL). The solvent

was evaporated to obtain compound 6c (150 mg, yield 89%).

MS m/z (ESI):295.3[M+H]$^+$.

Step 3: N-(1-(1H-indol-3-yl) hexane-2-yl)-4-fluoro-6-(4-methylpiperazine-1-yl) benzo[b]thiophene-2-carboxamide 6

Compound 6c (130 mg, 0.44 mmol) and compound 1c (100 mg, 0.5 mmol) were added to the reaction bottle, 5 mL of DMF was added to dissolve, and triethylamine (467 mg, 4.6 mmol) and 1-propylphosphoric acid cyclic anhydride (351 mg, 1.0 mmol) were added, stirring was started, and the reaction was carried out at room temperature for 1 hour. After the reaction was completed, water (50 mL) was added to quench the reaction, and ethyl acetate (50 mL × 3) was used for extraction. The organic layers were combined, dried with anhydrous sodium sulfate, and concentrated to obtain a crude product, which was purified by silica gel column chromatography (PE:EA = 1:1) to obtain compound 6 (35 mg, yield 38%).

MS m/z (ESI):493.5 [M+H]$^+$.

Example 7 N-(1-(1H-indol-3-yl) hexane-2-yl)-5-(4-methylpiperazine-1-yl) benzo[b]thiophene-2-carboxamide

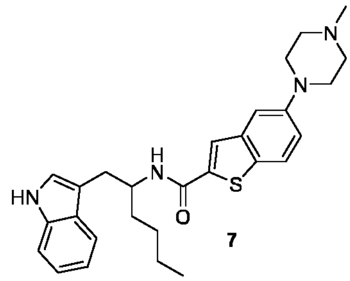

7

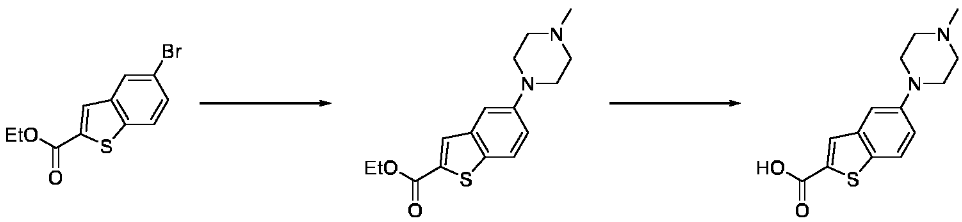

7a 7b 7c

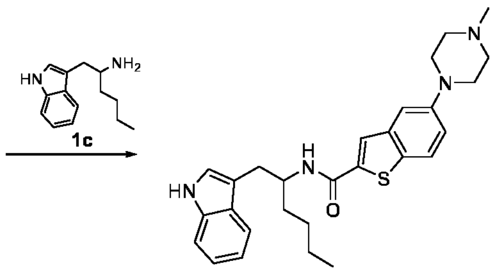

1c

7

Step 1: ethyl 5-(4-methylpiperazine-1-yl) benzo[b]thiophene-2-carboxylate 7b

Compound 7a (500 mg, 1.65 mmol), palladium acetate (40 mg, 0.2 mmol), Xantphos (202 mg, 0.35 mmol), cesium carbonate (3.42 g, 10.5 mmol) were added to the reaction bottle, 1-methylpiperazine (674 mg, 5.3 mmol) was dissolved in 1,4-dioxane (10 mL), nitrogen protection, stirring was started, and the reaction was carried out at 100°C for 3 hours. After the reaction was completed, water (50 mL) was added to quench the reaction, and ethyl acetate (50 mL × 3) was used for extraction. The organic layers were combined, dried with anhydrous sodium sulfate, concentrated, and purified by silica gel column chromatography (PE: EA = 5: 1) to obtain compound 7b (480 mg, yield 94%).

MS m/z (ESI):305.4[M+H]+.

Step 2: 5-(4-methylpiperazine-1-yl) benzo[b]thiophene-2-carboxylic acid 7c

Compound 7b (200 mg, 0.62 mmol) was dissolved in 2 mL of tetrahydrofuran, and then 2 mL of methanol was added. Sodium hydroxide was dissolved in water (2 mL) and added to the reaction bottle, stirred, and reacted at 70°C for 1 hour. After the reaction, the solvent was dried by rotary evaporation, the solid was dissolved in water, and then 1 M hydrochloric acid was used to adjust pH to 5-6, filtered, and the obtained filter cake was dissolved in methanol (20 mL), and the solvent was evaporated to obtain compound 7c (110 mg, yield 89%).

MS m/z (ESI):277.3[M+H]+.

Step 3: N-(1-(1H-indol-3-yl) hexane-2-yl)-5-(4-methylpiperazine-1-yl) benzo[b]thiophene-2-carboxamide 7

Compound 7c (121.6 mg, 0.44 mmol) and compound 1c (100 mg, 0.5 mmol) were added to the reaction bottle, 5 mL DMF was added to dissolve, and triethylamine (467 mg, 4.6 mmol) and 1-propylphosphoric acid cyclic anhydride (351 mg, 1.0 mmol) were added, and stirring was started. Reacted at room temperature for 1 hour. After the reaction was completed, water (50 mL) was added to quench the reaction, and ethyl acetate (50 mL×3) was used for extraction. The organic layers were combined, dried with anhydrous sodium sulfate, and concentrated to obtain a crude product, which was purified by silica gel column chromatography (PE:EA= 1: 1) to obtain compound 7 (35 mg, yield 38%).

MS m/z (ESI):475.5 [M+H]+.

Example 8 N-(1-(1H-indol-3-yl) hexane-2-yl)-6-(4-methylpiperazin-1-yl) thieno[3,2-c] pyridine-2-carboxamide

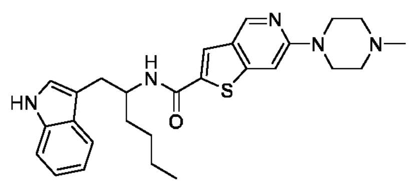

8

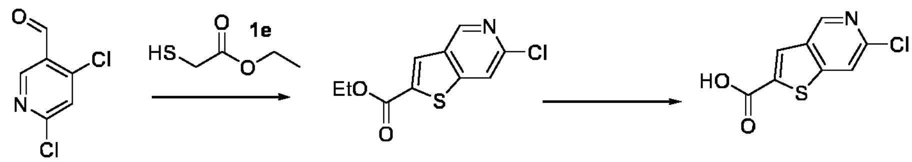


8a 8b 8c

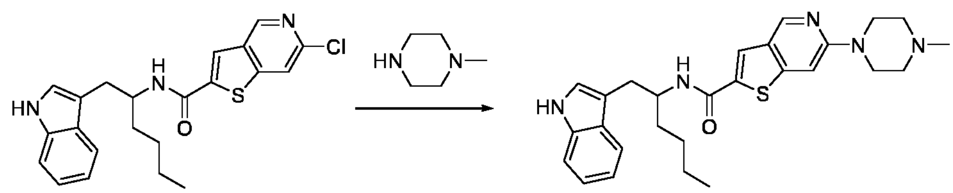

8d 8

Step 1: ethyl 6-chlorothieno[3,2-c] pyridine-2-carboxylate 8b

Compound 8a (1.0 g, 5.682 mmol) was dissolved in DMF (5 mL), and potassium carbonate (1.18 g, 0.853 mmol) and compound 1e (0.75 g, 6.25 mmol) were added at room temperature, and the reaction mixture was stirred at room temperature for 2 hours. The reaction mixture was quenched with water (50 mL), extracted with ethyl acetate (50 mL×3), and the organic phase was washed with saturated salt water (50 mL×3), dried with anhydrous sodium sulfate, filtered, concentrated, and purified by silica gel column chromatography (PE:EA= 5:1) to obtain compound 8b (0.95 g, yield 70%).

Step 2: 6-Chlorothieno[3,2-c] pyridine-2-carboxylic acid 8c

Compound 8b (0.9 g, 3.72 mmol) was dissolved in tetrahydrofuran (5 mL) and water (5 mL), and then sodium hydroxide (0.74 g, 18.62 mmol) was added at room temperature, and the reaction mixture was stirred at 70°C for 1 hour. The reaction mixture was adjusted the pH to 3-4 with dilute hydrochloric acid, and a lot of solids precipitated. The mixture was filtered and the solids were collected to obtain compound 8c (0.65 g, yield 82%).

Step 3: N-(1-(1H-indol-3-yl) hexane-2-yl)-6-chlorothieno[3,2-c] pyridine-2-carboxamide 8d

Compound 1c (0.3 g, 1.387 mmol) was dissolved in DMF (5 mL), and then compound 8c (0.33 g, 0.525 mmol), triethylamine (0.70 g, 6.934 mmol) and 1-propylphosphoric acid cyclic anhydride (0.66 g, 2.08 mmol) were added at room temperature, and the reaction mixture was stirred at room temperature for 1 hour. The reaction mixture was quenched with water (30 mL), extracted with ethyl acetate (50 mL×3), and the organic phase was washed with saturated salt water (50 mL×3), dried with anhydrous sodium sulfate, filtered, concentrated and purified by silica gel column chromatography (PE:EA=5:1) to obtain compound 8d.

Step 4: N-(1-(1H-indol-3-yl) hexane-2-yl)-6-(4-methylpiperazin-1-yl) thieno[3,2-c] pyridine-2-carboxamide 8

Compound 8d (0.24 g, 2.427 mmol) and 1-methylpiperazine (0.310 g, 3.09 mmol) were placed in a microwave reactor and reacted at 170°C for 3 hours. The reaction mixture was quenched with water (30 mL), extracted with ethyl acetate (50 mL×3), and the organic phase was washed with saturated salt water (50 mL×3), dried with anhydrous sodium sulfate, filtered, concentrated, and purified by silica gel column chromatography (PE:EA=5:1) to obtain compound 8 (35 mg, yield 15%).

MS m/z (ESI):477.5 $[M+H]^+$.

Example 9 N-(1-(1H-indol-3-yl) hexane-2-yl)-4-methyl-6-(4-methylpiperazin-1-yl) thieno[3,2-c]pyridine-2-carboxamide

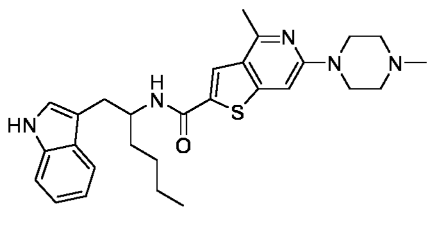

9

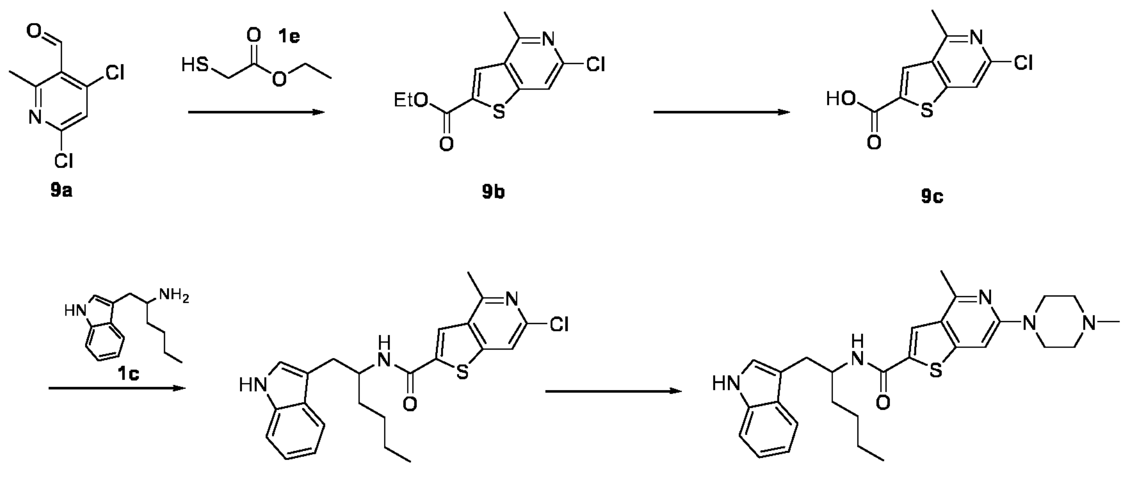


9a 9b 9c

9d 9

Step 1: ethyl 6-chloro-4-methylthieno[3,2-c] pyridine-2-carboxylate 9b

Compound 9a (0.25 g, 5.682 mmol) was dissolved in DMF (5 mL), and then potassium carbonate (0.27 g, 1.973 mmol) and compound 1e (0.17 g, 1.447 mmol) were added at room temperature, and the reaction mixture was stirred at room temperature for 1 hour. The reaction mixture was quenched with water (50 mL), extracted with ethyl acetate (50 mL × 3), the organic phase was washed with saturated salt water (50 mL × 2), dried with anhydrous sodium sulfate, filtered, concentrated and purified by silica gel column chromatography (PE: EA = 5: 1) to obtain compound 9b (0.21 g, yield 62%).

Step 2: 6-Chloro-4-methylthieno[3,2-c] pyridine-2-carboxylic acid 9c

Compound 9b (0.21 g, 0.821 mmol) was dissolved in tetrahydrofuran (5 mL) and water (5 mL), then sodium hydroxide (0.16 g, 4.106 mmol) was added at room temperature, and the reaction mixture was stirred at 70°C for 1 hour. The reaction mixture was adjusted the pH to 3-4 with dilute hydrochloric acid, and a lot of solids precipitated. The mixture was filtered and the solids were collected to obtain compound 9c (0.15 g, yield 80%).

Step 3: N-(1-(1H-indol-3-yl) hexane-2-yl)-6-chloro-4-methylthieno[3,2-c] pyridine-2-carboxamide 9d

Compound 1c (0.15 g, 1.387 mmol) was dissolved in DMF (5 mL), and then compound 9c (0.33 g, 0.525 mmol), triethylamine (0.35 g, 3.467 mmol) and 1-propylphosphoric acid cyclic anhydride (0.33 g, 1.04 mmol) were added at room temperature, and the reaction mixture was stirred at room temperature for 1 hour. The reaction mixture was quenched with water (30 mL), extracted with ethyl acetate (30 mL×3), and the organic phase was washed with saturated salt water (50 mL×2), dried with anhydrous sodium sulfate, filtered, concentrated, and purified by silica gel column chromatography (PE:EA = 5:1) to obtain compound 9d (0.16 g, yield 54%).

Step 4: N-(1-(1H-indol-3-yl) hexane-2-yl)-4-methyl-6-(4-methylpiperazin-1-yl) thieno[3,2-c]pyridine-2-carboxamide 9

Compound 9d (0.16 g, 0.376 mmol) and 1-methylpiperazine (0.19 g, 1.878mmol) were reacted in a microwave reactor at 180°C for 3 hours. The reaction mixture was quenched with water (30 mL), extracted with ethyl acetate (30 mL×3), and the organic phase was washed with saturated salt water (30 mL×2), dried with anhydrous sodium sulfate, filtered, concentrated, purified and separated by preparative HPLC to obtain compound 9 (0.035 g, yield 15%).

MS m/z (ESI):490.5 $[M+H]^+$.

Example 10 N-(1-(1H-indol-3-yl) hexane-2-yl)-7-(4-methylpiperazin-1-yl) benzo[b]thiophene-2-carboxamide

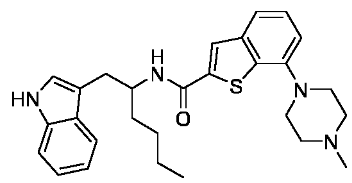

**10**

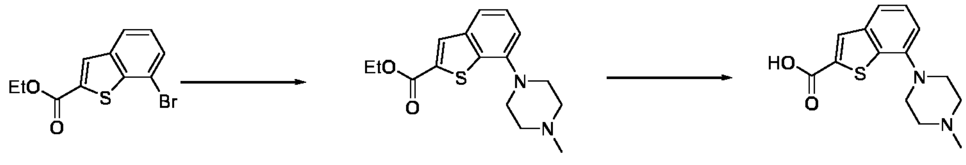

10a 10b 10c

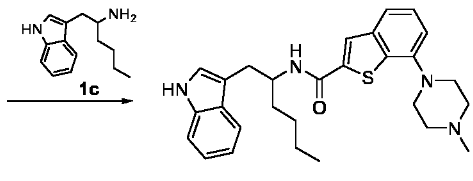


10

Step 1: ethyl 7-(4-methylpiperazine-1-yl) benzo[b]thiophene-2-carboxylate 10b

Compound 10a (500 mg, 1.65mmol), palladium acetate (40 mg, 0.2mmol), Xantphos (202 mg, 0.35mmol), cesium carbonate (3.42 g, 10.5mmol) were added to the reaction bottle, 1-methylpiperazine (674 mg, 5.3mmol) was dissolved in 1,4-dioxane (10 mL), nitrogen protection, stirring was started, and the reaction was carried out at 100°C for 3 hours. The reaction mixture was quenched by adding water (50 mL), extracted with ethyl acetate (30 mL×3), the organic layers were combined, dried with anhydrous sodium sulfate, concentrated, and purified by silica gel column chromatography (PE:EA=5:1) to obtain compound 10b (380 mg, yield 68%).

MS m/z (ESI):305.4$[M+H]^+$.

Step 2: 7-(4-methylpiperazine-1-yl) benzo[b]thiophene-2-carboxylic acid 10c

Compound 10b (200 mg, 0.62 mmol) was dissolved in 2 mL of tetrahydrofuran, and then 2 mL of methanol was added. Sodium hydroxide was dissolved in water (2 mL) and added to the reaction bottle, and stirring was started. The reaction was carried out at 70°C for 1 hour. The solvent of the reaction mixture was dried by rotary evaporation, the solid was dissolved in water (10 mL), and the pH was adjusted to 5-6 with 1M hydrochloric acid, filtered, and the filter cake was dissolved in methanol (20 mL), and the solvent was evaporated. Compound 10c (110 mg, yield 89%) was obtained.

MS m/z (ESI):277.3$[M+H]^+$.

Step 3: N-(1-(1H-indol-3-yl)hexane-2-yl)-7-(4-methylpiperazine-1-yl)benzo[b]thiophene-2-carboxamide 10

Compound 10c (130 mg, 0.44 mmol) and compound 1c (100 mg, 0.5mmol) were added to the reaction bottle, 5 mL of DMF was added to dissolve, and triethylamine (467 mg, 4.6mmol) and 1-propylphosphoric acid cyclic anhydride (351 mg, 1.0mmol) were added, stirring was started, and the reaction was carried out at room temperature for 1 hour. After the reaction was completed, the reaction was quenched with water (50 mL), extracted with ethyl acetate (50 mL×3), the organic layers were combined, dried with anhydrous sodium sulfate, and concentrated to obtain a crude product, which was separated and purified by preparative HPLC to obtain compound 10 (35 mg, yield 38%).

MS m/z (ESI):475.5 $[M+H]^+$.

Example 11 N-(1-(1H-indol-3-yl) hexane-2-yl)-3-methyl-6-(4-methylpiperazin-1-yl) benzo[b]thiophene-2-carboxamide

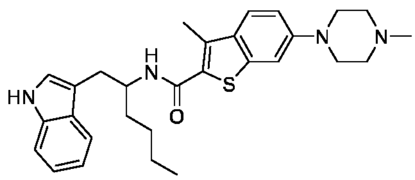

11

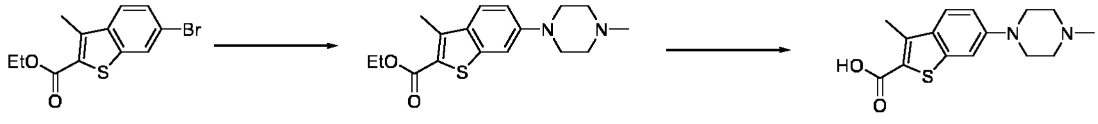

11a 11b 11c

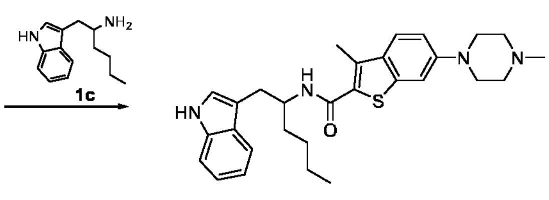


11

Step 1: ethyl 3-methyl-6-(4-methylpiperazin-1-yl) benzo[b]thiophene-2-carboxylate 11b

Compound 11a (0.25 g, 0.836mmol) was dissolved in 1,4-dioxane (10 mL), and then 1-methylpiperazine (0.167 g, 1.672mmol), palladium acetate (9.38 mg, 0.042mmol), Xantphos (48.35 mg, 0.084mmol) and cesium carbonate (52.6 mmol, 17.1mmol) were added at room temperature, nitrogen protection, the reaction mixture was stirred at 110°C for 3 hours. After cooling, the reaction mixture was quenched with water (50mL), extracted with ethyl acetate (50mL×3), the organic phase was washed with saturated salt water (50mL×3), dried with anhydrous sodium sulfate, filtered, concentrated and purified by silica gel column chromatography (PE:EA=5:1) to obtain compound 11b (0.21g, yield 78%).

Step 2: 3-Methyl-6-(4-methylpiperazine-1-yl) benzo[b]thiophene-2-carboxylic acid 11c

Compound 11b (0.21g, 0.659mmol) was dissolved in tetrahydrofuran (5mL) and water (5mL), and then sodium hydroxide (0.08g, 1.978mmol) was added at room temperature, and the reaction mixture was stirred at 70°C for 1 hour. The reaction mixture was adjusted the pH to 3-4 with 1M dilute hydrochloric acid, and a lot of solids precipitated. The mixture was filtered and the solids were collected to obtain compound 11c (0.17g, yield 89%).

Step 3: N-(1-(1H-indol-3-yl) hexane-2-yl)-3-methyl-6-(4-methylpiperazin-1-yl) benzo[b]thiophene-2-carboxamide 11

Compound 1c (200 mg, 0.9 mmol) and compound 11c (295 mg, 1.0 mmol) were dissolved in 5 mL DMF, and triethylamine (467 mg, 4.6mmol) was added, and finally 1-propylphosphoric acid cyclic anhydride (441 mg, 1.4mmol) was added and stirred at room temperature for 1 hour. After the reaction was completed, 30 mL of water was added to quench the reaction, and ethyl acetate (50 mL×3) was used for extraction. The organic layers were combined, dried with anhydrous sodium sulfate, filtered, concentrated, purified and separated by preparative HPLC to obtain compound 11 (90 mg, yield 20%).

MS m/z (ESI):489.3[M+H]$^+$.

Example 12 N-(1-(1H-indol-3-yl) hexane-2-yl)-3-methyl-6-(4-methylpiperazin-1-yl) benzo[b]thiophene-2-carboxamide

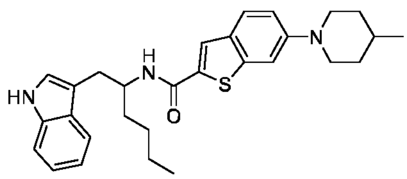

12

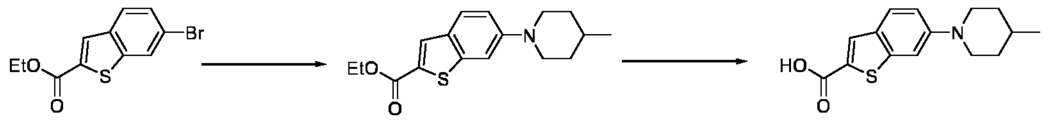

1f 12a 12b

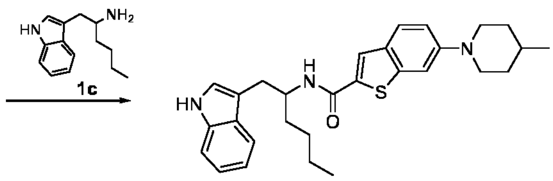

1c

12

Step 1: ethyl 6-(4-methylpiperidin-1-yl) benzo[b]thiophene-2-carboxylate 12a

Compound 1f (500 mg, 1.7 mmol) was added to the reaction bottle, and then palladium acetate (20 mg, 0.09 mmol), Xantphos (101 mg, 0.2 mmol) and cesium carbonate (1713 mg, 5.3mmol) were added, and finally 4-methylpiperidine (260 mg, 2.6mmol) and 1,4-dioxane (5 mL) were added. The temperature was raised to 100°C, and stirring was started under nitrogen protection, and the reaction was carried out for 3 hours. After the reaction was completed, water (50 mL) was added to quench the reaction, and ethyl acetate (50 mL×3) was used for extraction, the organic layers were combined, dried with anhydrous sodium sulfate, and concentrated to obtain a crude product, which was purified by silica gel column chromatography (PE: EA = 1: 1) to obtain compound 12a (200 mg, yield 38%).

MS m/z (ESI):304.1$[M+H]^+$.

Step 2: 6-(4-Methylpiperidin-1-yl) benzo[b]thiophene-2-carboxylic acid 12b

Compound 12a (164 mg, 0.5mmol) was dissolved in tetrahydrofuran (3 mL), and then methanol (3 mL) was added. Sodium hydroxide (86 mg, 2.1mmol) was dissolved in water (3 mL) and added to the reaction bottle, stirred, and reacted at 70°C for 1 hour. After the reaction, the solvent was dried by rotary evaporation, the solid was dissolved in water, and the pH was adjusted to pH = 5-6 with 1M hydrochloric acid, filtered, and the filter cake was dissolved in methanol (20 mL), and then the solvent was removed to obtain compound 12b (140 mg, yield 94%).

MS m/z (ESI):276.3$[M+H]^+$.

Step 3: N-(1-(1H-indol-3-yl) hexane-2-yl)-6-(4-methylpiperidin-1-yl) benzo[b]thiophene-2-carboxamide 12

Compound 1c (200 mg, 0.7 mmol) was dissolved in DMF (3 mL), and compound 12c (192 mg, 0.7 mmol) was added, and finally triethylamine (334 mg, 3.4mmol) and 1-propylphosphoric acid cyclic anhydride (315 mg, 1.0mmol) were added, and stirring was started to react for 1 hour. After the reaction was completed, 30 mL of water was added to quench the reaction, and ethyl acetate (50 mL×3) was used for extraction, and the organic layers were combined, dried with anhydrous sodium sulfate, filtered and concentrated to obtain a crude product, which was purified and separated by preparative HPLC to obtain compound 12 (70 mg, yield 22%).

MS m/z (ESI):474.3$[M+H]^+$.

Example 13 N-(1-(1H-indol-3-yl) hexane-2-yl)-6-(4-isopropylpiperazin-1-yl) benzo[b]thiophene-2-carboxamide

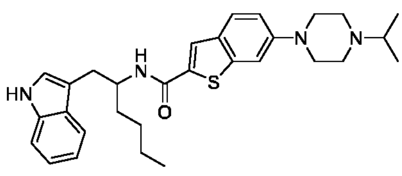

13

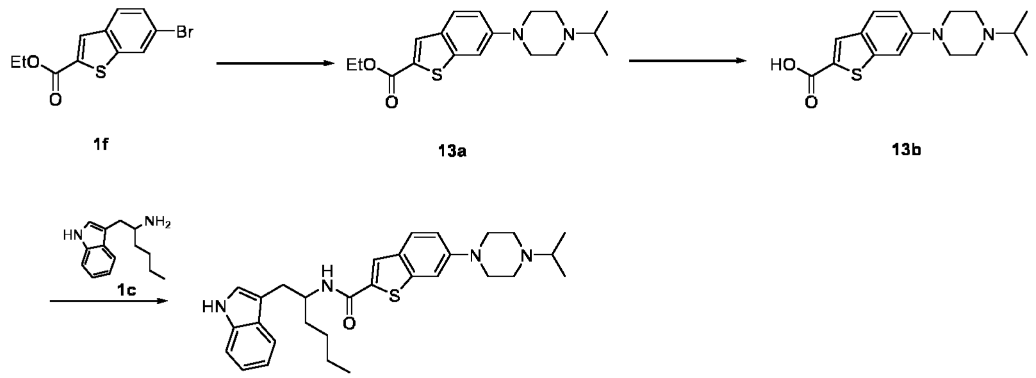

1f 13a 13b

1c

13

Step 1: ethyl 6-(4-isopropylpiperazine-1-yl) benzo[b]thiophene-2-carboxylate 13a

Compound 1f (1000 mg, 3.5 mmol), palladium acetate (40 mg, 0.2mmol), Xantphos (202 mg, 0.35mmol), cesium carbonate (3427 mg, 10.5mmol) were added to the reaction bottle, and 1-isopropylpiperazine (674 mg, 5.3 mmol) dissolved with 1,4-dioxane (10 mL) was added, nitrogen protection, stirring was started, and the reaction was carried out at 100°C for 3 hours. After the reaction was completed, water (50 mL) was added to quench the reaction, and ethyl acetate (50 mL×3) was used for extraction. The organic layers were combined, dried with anhydrous sodium sulfate, concentrated, and purified by silica gel column chromatography (PE: EA=5:1) to obtain compound 13a (500 mg, yield 43%).

MS m/z (ESI):333.2[M+H]$^+$.

Step 2: 6-(4-isopropylpiperazine-1-yl) benzo[b]thiophene-2-carboxylic acid 13b

Compound 13a (650 mg, 2.0mmol) was dissolved in tetrahydrofuran (3 mL), and then methanol (3 mL) was added. Sodium hydroxide was dissolved in 3 mL of water and added to the reaction bottle, stirred, and reacted at 70°C for 1 hour. The reaction mixture was dried by rotary evaporation, the solid was dissolved in water (20 mL), and the pH was adjusted to 5-6 with 1M hydrochloric acid, filtered, and the filter cake was dissolved in methanol (30 mL). The solvent was evaporated to obtain compound 13b (574 mg, yield 96%).

MS m/z (ESI):305.2[M+H]$^+$.

Step 3: N-(1-(1H-indol-3-yl) hexane-2-yl)-6-(4-isopropylpiperazin-1-yl) benzo[b]thiophene-2-carboxamide 13

Compound 13b (308 mg, 1.0 mmol) and compound 1c (200 mg, 0.9 mmol) were added to a reaction bottle, and DMF (5 mL) was added to dissolve. Then triethylamine (467 mg, 4.6 mmol) and 1-propylphosphoric acid cyclic anhydride (441 mg, 1.4 mmol) were added, and stirring was started at room temperature. The reaction was continued for 1 hour. After the reaction was completed, water (50 mL) was added to quench the reaction, and ethyl acetate (50 mL × 3) was used for extraction. The organic layers were combined, dried with anhydrous sodium sulfate, concentrated, and chromatographed to obtain compound 13 (110 mg, yield 23%).

MS m/z (ESI):503.3[M+H]$^+$.

Example 14 N-(1-(1H-indol-3-yl) hexane-2-yl)-6-(4-phenylpiperazine-1-yl) benzo[b]thiophene-2-carboxamide

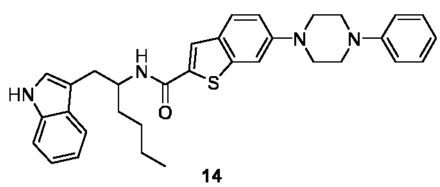

14

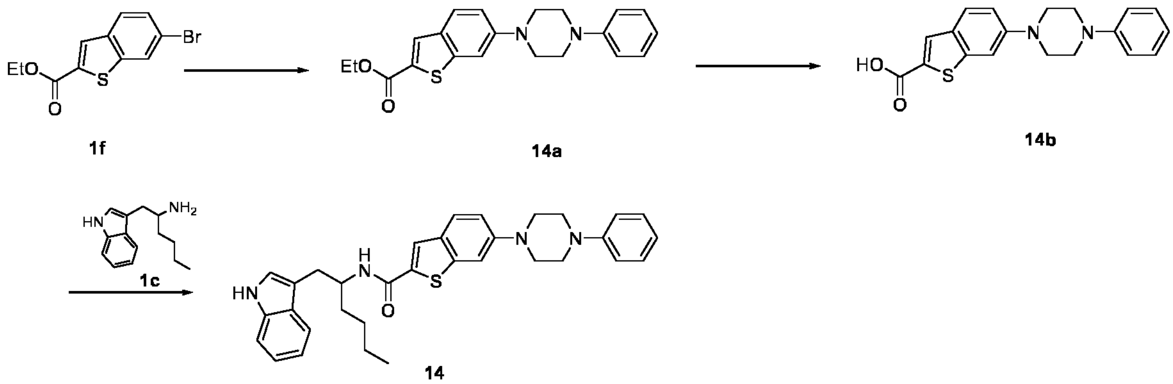

1f 14a 14b 1c 14

Step 1: Ethyl 6-(4-phenylpiperazin-1-yl) benzo[b]thiophene-2-carboxylate 14a

Compound 1f (1000 mg, 3.5 mmol), palladium acetate (40 mg, 0.2 mmol), Xantphos (202 mg, 0.3 mmol), cesium carbonate (3427 mg, 10.5 mmol) were added to the reaction bottle, and 1-phenylpiperazine (853 mg, 5.3 mmol) was added, dissolved with 1,4-dioxane (10 mL), nitrogen protection, stirred, and reacted at 100°C for 3 hours. The reaction mixture was quenched with water (50 mL), extracted with ethyl acetate (50 mL × 3), the organic layers were combined, dried with anhydrous sodium sulfate, and concentrated to obtain a crude product, which was purified by silica gel column chromatography (PE: EA = 5: 1) to obtain compound 14a (950 mg, yield 73.9%).
MS m/z (ESI):367.5$[M+H]^+$.

Step 2: 6-(4-phenylpiperazine-1-yl) benzo[b]thiophene-2-carboxylic acid 14b

Compound 14a (950 mg, 2.6 mmol) was dissolved in tetrahydrofuran (3 mL), and then 3 mL of methanol was added. Sodium hydroxide (518, 13.0 mmol) was dissolved in water (3 mL) and added to the reaction bottle. Stirring was started and the reaction was carried out at 70°C for 1 hour. The reaction mixture was dried by rotary evaporation and the solid was dissolved in water (10 mL). The pH was adjusted to 5-6 with 1 M hydrochloric acid, and then filtered. The filter cake was dissolved in methanol (20 mL), and the solvent was removed by rotary evaporation to obtain compound 14b (816 mg, yield 93%).
MS m/z (ESI):339.3$[M+H]^+$.

Step 3: N-(1-(1H-indol-3-yl) hexane-2-yl)-6-(4-phenylpiperazine-1-yl) benzo[b]thiophene-2-carboxamide 14

Compound 14b (344 mg, 1.0 mmol) and compound 1c (200 mg, 0.9 mmol) were added to a reaction bottle, dissolved with 5 mL DMF, and then triethylamine (467 mg, 4.6 mmol) and 1-propylphosphoric acid cyclic anhydride (441 mg, 1.4 mmol) were added, stirred, and reacted at room temperature for 1 hour. 30 mL of water was added to the reaction mixture to quench the reaction, and ethyl acetate (50 mL × 3) was used for extraction and separation. The organic layers were combined, dried with anhydrous sodium sulfate, and concentrated to obtain a crude product, which was purified and separated by preparative HPLC to obtain compound 14 (128 mg, yield 26%).
MS m/z (ESI):536.3$[M+H]^+$.

Example 15 N-(1-(1H-indol-3-yl) hexane-2-yl)-6-(4,4-difluoropiperidin-1-yl) benzo[b]thiophene-2-carboxamide

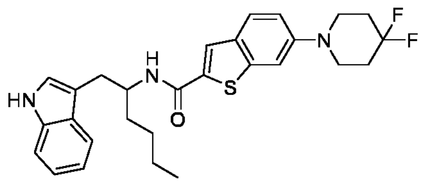

15

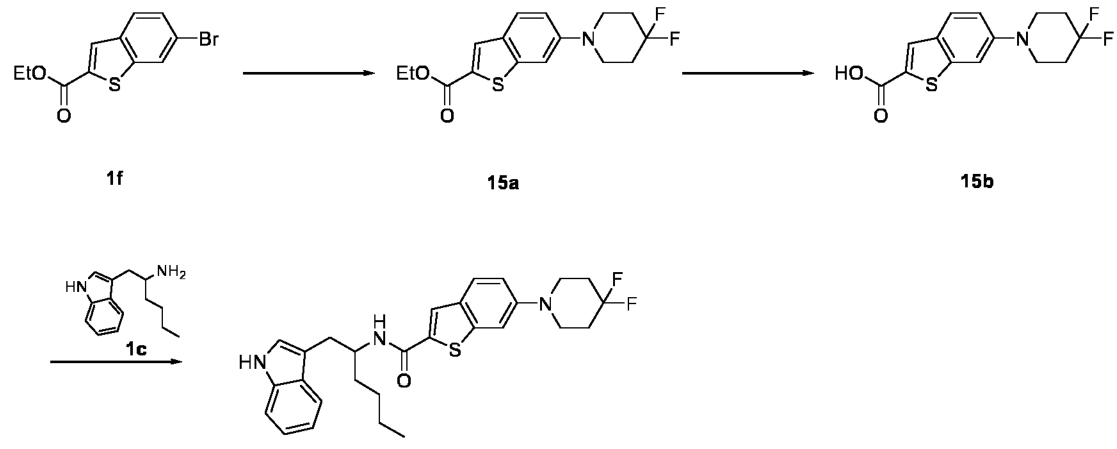


1f 15a 15b

15

Step 1: ethyl 6-(4,4-difluoropiperidin-1-yl) benzo[b]thiophene-2-carboxylate 15a

Compound 1f (392 mg, 1.37 mmol) and 4,4-difluoropiperidine (250 mg, 2.06 mmol) were added to the reaction bottle, and then palladium acetate (15 mg, 0.07 mmol), Xantphos (80 mg, 0.14 mmol), cesium carbonate (1345 mg, 4.13 mmol), and finally 1,4-dioxane (10 mL) were added, and stirring was started under nitrogen protection, and the reaction was carried out at 100°C for 3 hours. The reaction mixture was quenched with 30 mL of water, and then extracted with ethyl acetate (50 mL×3), separated, dried with anhydrous sodium sulfate, filtered, and concentrated to obtain a crude product, which was purified by silica gel column chromatography (PE:EA=5:1) to obtain compound 15a (396 mg, yield 88%).

MS m/z (ESI):326[M+H]$^+$.

Step 2: 6-(4,4-difluoropiperidin-1-yl) benzo[b]thiophene-2-carboxylic acid 15b

Compound 15a (396 mg, 1.2 mmol) was dissolved in tetrahydrofuran (3 mL) and methanol (3 mL), sodium hydroxide (243 mg, 6.1 mmol) was dissolved in water, then added to the reaction bottle, stirring was started. The reaction mixture was heated to 70°C and reacted for 1 hour. The reaction mixture was evaporated to remove the solvent, the solid was dissolved with 10 mL of water, and then the pH was adjusted to 5-6 with 1 M hydrochloric acid, filtered and dried to obtain compound 15b (349 mg, yield 96%).

MS m/z (ESI):298.1[M+H]$^+$.

Step 3: N-(1-(1H-indol-3-yl) hexane-2-yl)-6-(4,4-difluoropiperidin-1-yl) benzo[b]thiophene-2-carboxamide 15

Compound 15b (227 mg, 0.76 mmol), compound 1c (150 mg, 0.69 mmol), triethylamine (350 mg, 3.46 mmol) were added to a reaction bottle and dissolved with DMF (5 mL), and then 1-propylphosphoric acid cyclic anhydride (350 mg, 3.46 mmol) was weighed and added to the reaction bottle, stirring was started, and the reaction was carried out at room temperature for 1 hour. After the reaction was completed, 50 mL of organic phase was added and quenched with saturated sodium bicarbonate solution, extracted with ethyl acetate (50 mL×3), the organic layers were combined, dried with anhydrous sodium sulfate, and concentrated to obtain a crude product, which was purified and separated by preparative HPLC to obtain compound 15 (124 mg, yield 36%).

MS m/z (ESI):496.3[M+H]$^+$.

$^1$H NMR (400 MHz, Chloroform-*d*) δ 8.16 (s, 1H), 7.67 (d, *J* = 7.9 Hz, 1H), 7.61 (d, *J* = 8.8 Hz, 1H), 7.42 (s, 1H), 7.37 (d, *J* = 8.0 Hz, 1H), 7.27 - 7.16 (m, 1H), 7.16 - 7.07 (m, 1H), 7.10 - 7.01 (m, 2H), 5.90 (d, J = 8.8 Hz, 1H), 4.47 (tdd, *J* = 11.0, 6.9, 4.3 Hz, 1H), 3.46 - 3.38 (m, 4H), 3.15 - 3.00 (m, 2H), 2.19 - 2.05 (m, 4H), 1.74 - 1.63 (m, 1H), 1.58 - 1.45 (m, 1H), 1.45 - 1.38 (m, 1H), 1.43 - 1.23 (m, 3H), 0.87 (t, *J* = 7.1 Hz, 3H).

Example 16 N-(1-(1H-indol-3-yl) hexane-2-yl)-6-(4,4-dimethylpiperidin-1-yl) benzo[b]thiophene-2-carboxamide

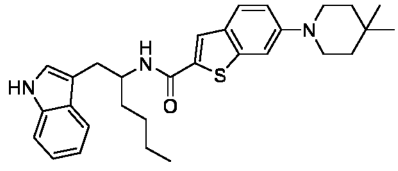

16

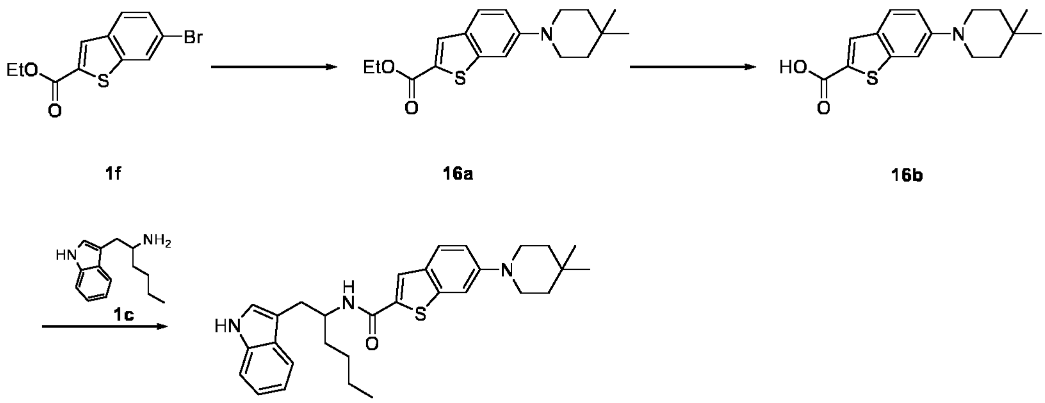

1f 16a 16b

1c

16

Step 1: ethyl 6-(4,4-dimethylpiperidin-1-yl) benzo[b]thiophene-2-carboxylate 16a

Compound 1f (500 mg, 1.84 mmol) and 4,4-dimethylpiperidine (313 mg, 2.76 mmol) were added to the reaction bottle, and then palladium acetate (20 mg, 0.09 mmol), Xantphos (107 mg, 0.18 mmol), cesium carbonate (1802 mg, 5.53 mmol), and finally 1,4-dioxane (10 mL) were added. Stirring was started under nitrogen protection, and the reaction mixture was heated to 100°C and reacted for 3 hours. After the reaction was completed, water (20 mL) was added to quench the reaction, and ethyl acetate (50 mL×3) was used for extraction. The organic layers were combined, dried with anhydrous sodium sulfate, concentrated, and purified by silica gel column chromatography (PE: EA=5:1) to obtain compound 16a (479 mg, yield 82%).

MS m/z (ESI):318.1$[M+H]^+$.

Step 2: 6-(4,4-dimethylpiperidin-1-yl) benzo[b]thiophene-2-carboxylic acid 16b

Compound 16a (479 mg, 1.5 mmol) was added to a reaction bottle and dissolved with tetrahydrofuran (3 mL) and methanol (3 mL). Sodium hydroxide (301 mg, 7.05 mmol) was weighed and dissolved with water (3 mL), then added to the reaction flask, stirred, and reacted at 70°C for 1 hour. The reaction mixture was evaporated to remove the solvent, then water (20 mL) was added to dissolve, the pH was adjusted to 5-6 with 1M hydrochloric acid, filtered, the filter cake was washed with water, and then dissolved with 20 mL of tetrahydrofuran, and the solvent was evaporated to obtain compound 16b (424 mg, yield 97%).

MS m/z (ESI):290.2$[M+H]^+$.

Step 3: N-(1-(1H-indol-3-yl) hexane-2-yl)-6-(4,4-dimethylpiperidin-1-yl) benzo[b]thiophene-2-carboxamide 16

Compound 16b (221 mg, 0.76 mmol) and compound 1c (150 mg, 0.69 mmol) were dissolved in DMF (10 mL), and triethylamine (350 mg, 3.47 mmol) was weighed and added to the reaction bottle, 1-propylphosphoric acid cyclic anhydride (330 mg, 1.04 mmol) was added to the reaction bottle, stirring was started, and the reaction was carried out at room temperature for 1 hour. After the reaction was completed, 30 mL of organic phase was added and quenched with saturated sodium bicarbonate solution, extracted with ethyl acetate (30 mL×3), the organic layers were combined, dried with anhydrous sodium sulfate, filtered and concentrated to obtain a crude product, and the crude product was purified and separated by preparative HPLC to obtain compound 16 (110 mg, yield 33%).

MS m/z (ESI):488.3$[M+H]^+$.

Example 17 7-Fluoro-N-[1-(1H-indol-3-yl) hexyl-2-yl]-6-(4-methylpiperazin-1-yl)-1-**[0265]** benzothiophene-2-carboxamide

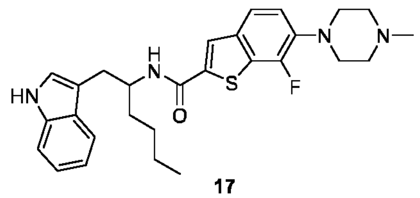

**17**

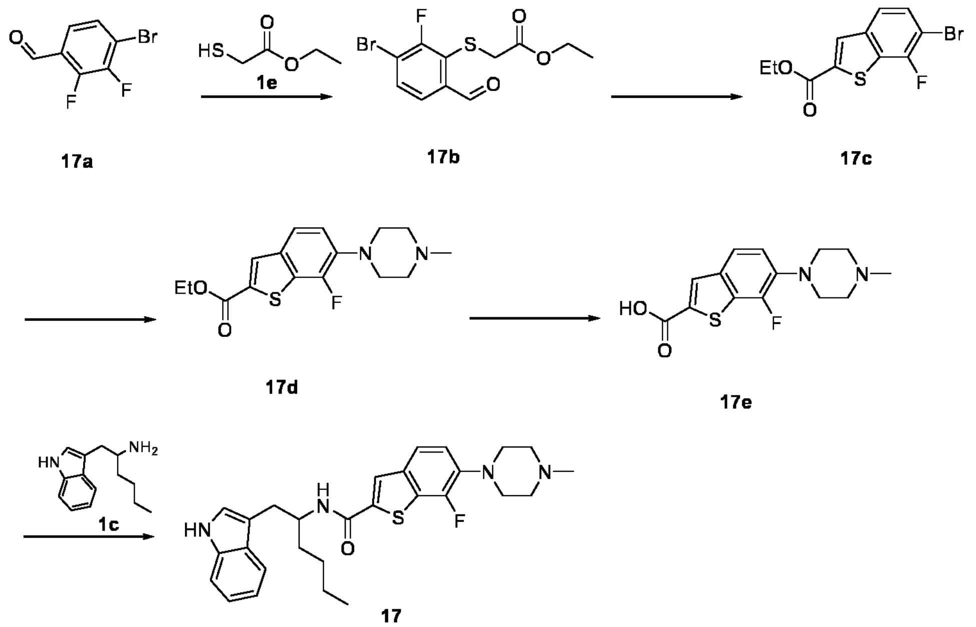

1e

17a

17b

17c

17d

17e

1c

**17**

Step 1: ethyl 2-[(3-bromo-2-fluoro-6-formylphenyl) mercapto] acetate 17b

Compound 17a (806 mg, 3.65 mmol) was dissolved in dichloromethane (20 mL), triethylamine (0.51 mL, 3.65 mmol) was added, and compound 1e (440 mg, 3.66 mmol) was added. After reacting at room temperature for 3 hours, the reaction mixture was diluted with dichloromethane (50 mL), washed with water (30 mL × 3), dried with anhydrous sodium sulfate, and the crude product obtained after concentration was purified by silica gel column chromatography (PE: EA = 5: 1) to obtain compound 17b (320 mg, yield 27.3%).

MS m/z (ESI): 321.1 $[M+H]^+$.

Step 2: Ethyl 6-bromo-7-fluoro-1-benzothiophene-2-carboxylate 17c

Ethyl 2-[(3-bromo-2-fluoro-6-formylphenyl) mercapto] acetate 17b (320 mg, 1 mmol) was dissolved in DMF (4 mL), potassium carbonate (160 mg, 1.16 mmol) was added, and the mixture was reacted at 70°C for 3 hours to complete the reaction. The reaction mixture was diluted with ethyl acetate (50 mL), the organic phase was washed with saturated salt water (30 mL × 2), and the organic phase was dried with anhydrous sodium sulfate and concentrated to obtain a crude product. The crude product was purified by silica gel column chromatography (PE: EA = 5: 1) to obtain compound 17c (223 mg, yield 73.8%).

Step 3: ethyl 7-Fluoro-6-(4-methylpiperazinyl-1-yl)-1-benzothiophene-2- carboxylate 17d

Ethyl 6-bromo-7-fluoro-1-benzothiophene-2-carboxylate 17c (223 mg, 0.736 mmol), 1-methylpiperazine (74 mg, 0.74 mmol), palladium acetate (20 mg, 0.09 mmol), Xantphos (90 mg, 0.155 mmol) and cesium carbonate (720 mg, 2.21 mmol) were mixed in 1,4-dioxane (10 mL), and nitrogen protection, the mixture was heated to 110°C for 4 hours to complete the reaction. The reaction mixture was concentrated and extracted with ethyl acetate (30 mL × 3), the

organic phase was dried with anhydrous sodium sulfate, filtered and concentrated, and the crude product was purified by silica gel column chromatography (PE: EA = 5: 1) to obtain compound 17d (230 mg, yield 97%).

MS m/z (ESI): 323.2 $[M+H]^+$.

Step 4: 7-Fluoro-6-(4-methylpiperazin-1-yl)-1-benzothiophene-2-carboxylic acid 17e

Ethyl 7-fluoro-6-(4-methylpiperazin-1-yl)-1-benzothiophene-2-carboxylate 17d (217 mg, 0.673 mmol) was dissolved in tetrahydrofuran (8 mL), and a solution of sodium hydroxide (160 mg, 4 mmol) dissolved in water (4 mL) was added to the reaction mixture. The reaction mixture was heated to 70°C for 2.5 hours, and the reaction was completed. After the reaction mixture was concentrated to remove tetrahydrofuran, water (6 mL) was added, and the pH was adjusted to 6 with 1M hydrochloric acid. A solid precipitate was precipitated, filtered, and the solid was dried to obtain compound 17e (160 mg, yield 80.8%).

MS m/z (ESI): 295.2 $[M+H]^+$.

Step 5: 7-Fluoro-N-[1-(1H-indol-3-yl) hexyl-2-yl]-6-(4-methylpiperazinyl-1-yl)-1-benzothiophene-2-carboxamide 17

Compound 1c (74 mg, 0.34 mmol) and compound 17e (100 mg, 0.34 mmol) were mixed in DMF (3 mL), DIEA (0.12 mL, 0.7 mmol) was added, and then HATU (129 mg, 0.34 mmol) was added. The reaction mixture was stirred at room temperature for 2 hours, and the reaction was completed. The reaction mixture was extracted with ethyl acetate (30 mL×3), the organic phase was washed with saturated salt water (30 mL×3), dried with anhydrous sodium sulfate, and the crude product obtained after concentration was purified by silica gel column chromatography (PE:EA=5:1) to obtain compound 17 (60 mg, yield 36%).

MS m/z (ESI): 493.3 $[M+H]^+$.

Example 18 N-(1-(1H-indol-3-yl) hexane-2-yl)-6-(4-methylpiperidin-1-yl) thieno[3,2-c] pyridine-2-carboxamide

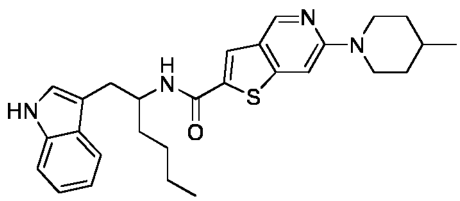

18

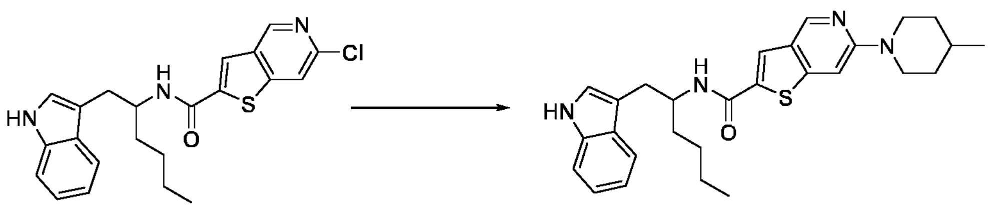

8d 18

Compound 8d (125 mg, 0.3 mmol), 4-methylpiperidine (250 mg, 2.5 mmol) and DIEA (300 mg, 2.3 mmol) were dissolved in 1,4-dioxane (30 mL), added into a microwave reactor, and reacted at 100°C for 3 hours. After the reaction, 30 mL of water was added to quench the reaction, and ethyl acetate (30 mL × 3) was used for extraction. The organic layers were combined, dried with anhydrous sodium sulfate, filtered and concentrated to obtain a crude product, which was purified and separated by preparative HPLC to obtain compound 18 (70 mg, yield 48%).

MS m/z (ESI):475.3$[M+H]^+$.

Example 19 N-(1-(1H-indol-3-yl) hexane-2-yl)-6-(4-hydroxypiperidin-1-yl) benzo[b]thiophene-2-carboxamide 19

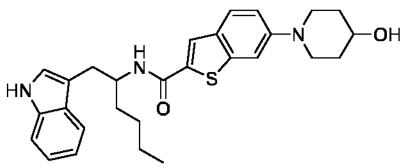

19

Compound 19 was prepared using a synthesis method similar to Example 16, replacing 4,4-dimethylpiperidine with piperidine-4-ol, yield 17.40%.
MS m/z (ESI): 476.2 [M+H]$^+$.

Example 20 N-[1-(1H-indol-3-yl) hexane-2-yl]-6-(2-oxa-7-azaspiro [3.5] nonan-7-yl)-1-benzothiophene-2-carboxamide

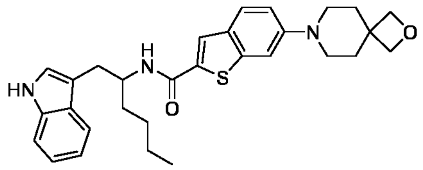

20

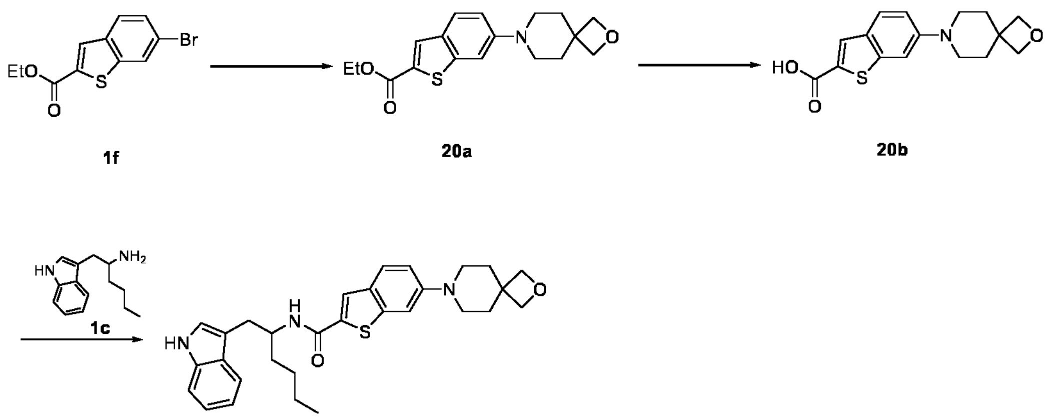

1f 20a 20b

1c

20

Step 1: ethyl 6-(2-oxa-7-azaspiro [3.5] nonane-7-yl)-1-benzothiophene-2-carboxamide 20a

Compound 1f (0.224 g, 0.786 mmol), 2-oxa-7-azaspiro [3.5] nonane (0.100 g, 0.786 mmol), palladium acetate (0.018 g, 0.079 mmol), Xantphos (0.068 g, 0.118 mmol) and cesium carbonate (0.769 g, 2.359 mmol) were weighed into a sealed tube, and then 1,4-dioxane (6 mL) was added. The reaction was completed by stirring at 100°C for 2 hours under nitrogen protection. After filtering with diatomite, the filtrate was concentrated to obtain a crude product, which was purified by silica gel column chromatography (PE: EA = 5: 1) to obtain compound 20a (0.247 g, yield 90.18%).
MS m/z(ESI): 332 [M+H]$^+$.

Step 2: 6-(2-oxa-7-azaspiro [3.5] nonane-7-yl)-1-benzothiophene-2-carboxylic acid 20b

Compound 20a (0.235 g, 0.709 mmol) was dissolved in a mixed solution of tetrahydrofuran (2 mL) and methanol (2 mL), and then 1.0 mL of an aqueous solution of sodium hydroxide (0.085 g, 2.127 mmol) was added. The reaction was completed after 1 hour at 70°C. After the solvent was removed by distillation under reduced pressure, 3 mL of water was added, and then the pH was adjusted to 5-6 with 1 M dilute hydrochloric acid. After filtering, the filter cake was dried to obtain compound 20b (0.183 g, yield 85.05%).
MS m/z (ESI):304 [M+H]$^+$.

Step 3: N-[1-(1H-indol-3-yl) hexane-2-yl]-6-(2-oxa-7-azaspiro [3.5] nonane-7-yl)-1-benzothiophene-2-carboxamide 20

Compound 20b (0.092 g, 0.303 mmol) and compound 1c (0.066 g, 0.303 mmol) were dissolved in 3 mL DMF. After dissolution, triethylamine (0.092 g, 0.910 mmol) mol) and 1-propylphosphoric acid cyclic anhydride (0.288 g, 0.455 mmol) dissolved in tetrahydrofuran was added, stirred at room temperature for 1 hour, the reaction was completed, ethyl acetate (50 mL) was directly added to the reaction solution, washed with water (20 mL × 3), and then extracted with ethyl acetate (30 mL × 3), the combined organic phases were dried with anhydrous sodium sulfate, filtered, concentrated, and separated by preparative HPLC to obtain compound 20 (0.052 g, yield 34.18%).

$^1$H NMR (400 MHz, Chloroform-d) δ 8.24 (s, 1H), 7.66 (d, J = 7.9 Hz, 1H), 7.58 (d, J = 8.9 Hz, 1H), 7.42 (s, 1H), 7.37 (d, J = 8.1 Hz, 1H), 7.24 - 7.16 (m, 2H), 7.11 (t, J = 7.5 Hz, 1H), 7.08 - 7.02 (m, 2H), 5.92 (d, J = 8.8 Hz, 1H), 4.48 (s, 4H), 3.20 - 3.14 (m, 4H), 3.07 (t, J = 5.7 Hz, 1H), 2.06 - 1.98 (m, 4H), 1.70 - 1.61 (m, 2H), 1.55 - 1.23 (m, 6H), 0.87 (t, J = 7.1 Hz, 3H).

MS m/z (ESI):502 $[M+H]^+$.

Example 21 N-[1-(1H-indol-2-yl) hexane-2-yl]-6-[2-(1-methylpiperidin-4-yl)-2,7-diazaspiro [3.5]nonane-7-yl]-1-benzothiophene-2-carboxamide

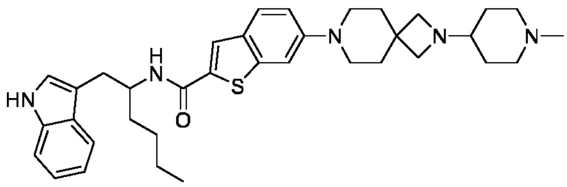

21

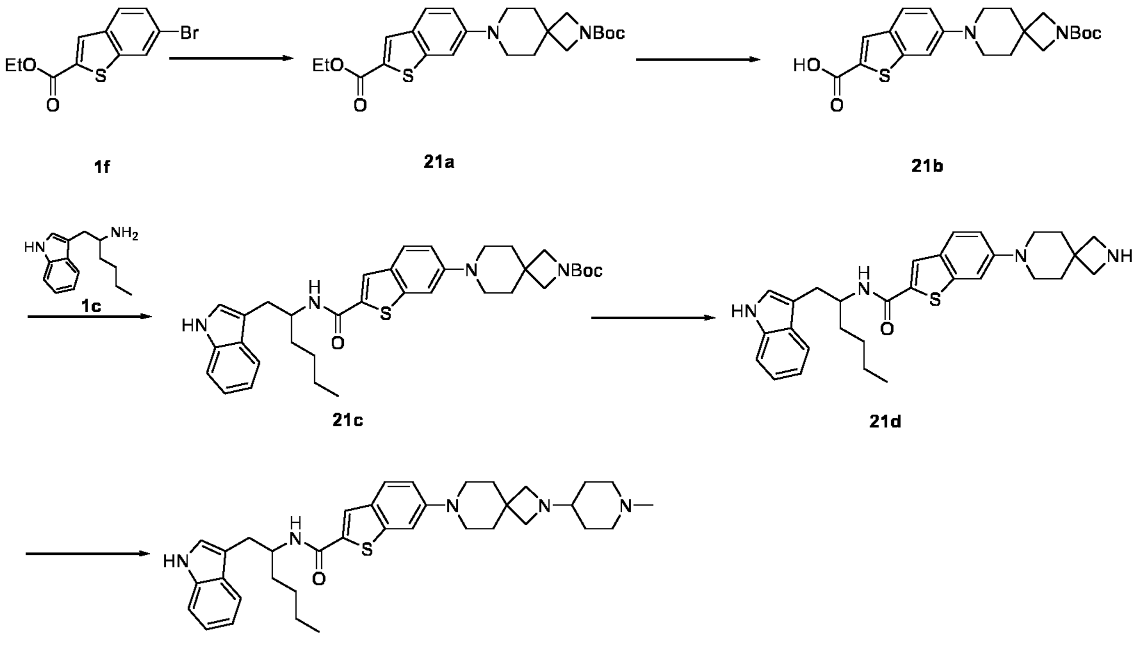

1f

21a

21b

1c

21c

21d

21

Step 1: tert-butyl 7-[2-(ethoxycarbonyl)-1-benzothiophen-6-yl]-2,7-diazaspiro [3.5] nonane-2-carboxylate 21a

Compound 21a was prepared using a synthesis method similar to compound 20a, replacing 2-oxa-7-azaspiro [3.5] nonane with tert-butyl 2,7-diazaspiro [3.5] nonane-2-carboxylate, yield 52.08%.

MS m/z (ESI):431 $[M+H]^+$.

Step 2: 6-(2-[(tert-Butoxy) carbonyl]-2,7-diazaspiro [3.5] nonane-7-yl)-1-benzothiophene-2-Carboxylic acid 21b

Compound 21b was prepared using a synthesis method similar to compound 20b, replacing 20a with 21a, yield 97.37%.

MS m/z (ESI):403 $[M+H]^+$.

Step 3: tert-Butyl 7-(2-([1-(1H-indol-2-yl) hexane-2-yl] carbamoyl)-1-benzothiophen-6-yl)-2,7-Diazaspiro [3.5] nonane-2-carboxylate 21c

Compound 21b (0.974 g, 2.420 mmol) and compound 1c (0.520 g, 2.420 mmol) were dissolved in 25 mL of DMF, and then N-methylimidazole (0.990 g, 12.100 mmol) was added. After stirring evenly, N, N, N', N'-tetramethylchloroformamidine hexafluorophosphate (1.020 g, 3.630 mmol) was added. After stirring at room temperature for 2 hours, the reaction was completed. Ethyl acetate (50 mL) was directly added to the reaction solution, washed with water (30 mL×3), and then extracted with ethyl acetate (30 mL×3). The organic phase was dried with anhydrous sodium sulfate and filtered. Compound 21c was purified by silica gel column chromatography (PE: EA=1:1) with a yield of 60.14%.

MS m/z (ESI): 601 $[M+H]^+$.

Step 4: 6-(2,7-diazaspiro [3.5] nonane-7-yl)-N-[1-(1H-indol-2-yl) hexane-2-yl]-1-benzothiophene-2-carboxamide 21d

Compound 21d was prepared using a synthesis method similar to compound 25d, replacing 25c with 21c, yield 104.50%.

MS m/z (ESI):501 $[M+H]^+$.

Step 5: N-[1-(1H-indol-2-yl) hexane-2-yl]-6-[2-(1-methylpiperidin-4-yl)-2,7-diazaspiro [3.5] nonane-7-yl]-1-benzothiophene-2-carboxamide 21

Compound 21 was prepared using a synthesis method similar to Example 25, replacing 25d with 21d, and replacing polyformaldehyde with 4-methylpiperidone, yield 25.25%.

MS m/z (ESI): 598$[M+H]^+$.

$^1H$ NMR (400 MHz, DMSO-d6) δ 10.77 (d, J = 2.4 Hz, 1H), δ 8.33 (d, J = 8.5 Hz, 1H), 7.94 (s, 1H), 7.71 (d, J = 8.9 Hz, 1H), 7.62 (d, J = 7.8 Hz, 1H), 7.44 (d, J = 2.2 Hz, 1H), 7.32 (d, J = 8.0 Hz, 1H), 7.19 - 7.11 (m, 2H), 7.08 - 7.01 (m, 1H), 6.96 (t, J = 7.4 Hz, 1H), 4.18 (td, J = 8.2, 5.4 Hz, 1H), 3.98 (s, 4H), 3.64 - 3.50 (m, 4H), 3.25 (s, 3H), 3.01 - 2.82 (m, 4H), 2.77 (s, 3H), 2.13 (d, J = 12.9 Hz, 2H), 1.89 (s, 4H), 1.66 - 1.52 (m, 4H), 1.39 - 1.18 (m, 4H), 0.81 (t, J = 6.8 Hz, 3H).

Example 22 N-[1-(1H-indol-2-yl) hexane-2-yl]-6-(2-methyl-2,7-diazaspiro [3.5] nonane-7-yl)-1-benzothiophene-2-carboxamide

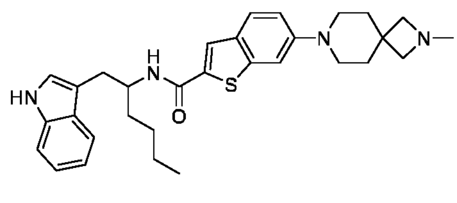

22

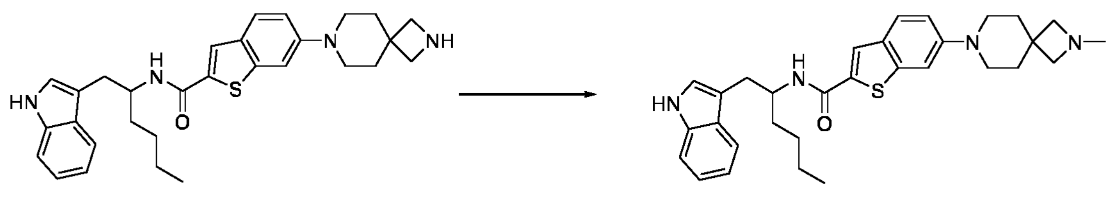

21d 22

Compound 22 was prepared using a synthesis method similar to Example 21, replacing 4-methylpiperidone

with polyformaldehyde, yield 44.57%.

$^1$H NMR (400 MHz, DMSO-d6) δ 10.65 (d, J = 2.4 Hz, 1H), 8.20 (d, J = 8.5 Hz, 1H), 7.83 (s, 1H), 7.60 (d, J = 9.0 Hz, 1H), 7.51 (d, J = 7.8 Hz, 1H), 7.33 (d, J = 2.2 Hz, 1H), 7.21 (d, J = 8.0 Hz, 1H), 7.09 - 7.01 (m, 2H), 6.94 (ddd, J = 8.1, 6.9, 1.2 Hz, 1H), 6.89 - 6.83 (m, 1H), 4.07 (q, J = 7.8, 6.4 Hz, 1H), 3.79 (s, 4H), 3.13 (d, J = 5.8 Hz, 4H), 2.86 (dd, J = 14.3, 6.7 Hz, 2H), 2.76 (s, 3H), 1.84 - 1.75 (m, 4H), 1.46 (dd, J = 9.4, 4.8 Hz, 2H), 1.30 - 1.04 (m, 4H), 0.76 - 0.66 (m, 3H)。

MS m/z (ESI): 515[M+H]$^+$。

Example 23 N-(1-(1H-indol-3-yl) hexane-2-yl)-6-morpholinobenzo[b]thiophene-2-carboxamide

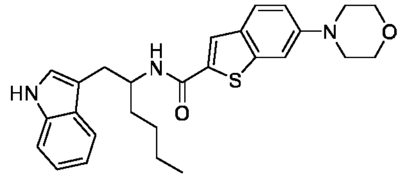

23

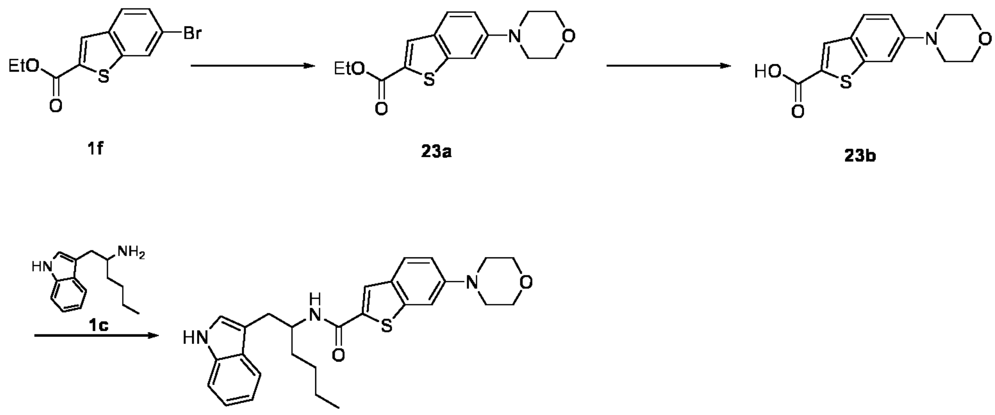

1f

23a

23b

1c

23

Step 1: ethyl 6-morpholinobenzo[b]thiophene-2-carboxamide 23a

Compound 1f (300 mg, 1.052 mmol), morpholine (137.5 mg, 1.578 mmol), palladium acetate (11.8 mg, 0.053 mmol), Xantphos (60.87 mg, 0.105 mmol), cesium carbonate (1.028 g, 3.156 mmol) were placed in a single-mouth bottle, 1,4-dioxane (12 mL) was added after nitrogen replacement, and the reaction was carried out at 100°C for 3 hours after the second nitrogen replacement. The reaction solution was filtered and the filtrate was concentrated to obtain a crude product. The crude product was purified by silica gel column chromatography (PE: EA=3:1) to obtain compound 23a (240 mg, yield 78.3%, yellow solid).

MS m/z(ESI):292.1[M+H]$^+$.

Step 2: 6-Morpholinobenzo[b]thiophene-2-carboxylic acid 23b

Compound 23a (240 mg, 0.824 mmol) was dissolved in tetrahydrofuran (3 mL), methanol and sodium hydroxide (164.8 mg, 4.120 mmol) in water (3 mL) were added, and the mixture was reacted at 100°C for 1 hour. LCMS detected that the reaction was complete and the product was generated. The reaction solution was concentrated, tetrahydrofuran and methanol were removed, water (10 mL) was added, and the pH was adjusted to 6-7 with 1M hydrochloric acid. Solids precipitated and were filtered. After drying the filter cake, compound 23b (190 mg, yellow solid, crude product) was obtained.

MS m/z(ESI):264.1[M+H]$^+$.

Step 3: N-(1-(1H-indol-3-yl) hexane-2-yl)-6-morpholinothieno[2,3-b] pyridine-2-carboxamide 23

Compound 23b (190 mg, 0.722 mmol) and compound 1c (156.1 mg, 0.722 mmol) were dissolved in DMF (10

mL), triethylamine (365.1 mg, 3.608 mmol) and 1-propylphosphoric acid cyclic anhydride (919.0 mg, 1.443 mmol) were added and reacted at room temperature for 1 hour. LCMS detected the formation of the product. Water (100 mL) was added to the reaction solution, and ethyl acetate (40 mL × 3) was used for extraction. The organic phase was washed with saturated salt water (30 mL × 2), dried with anhydrous sodium sulfate, filtered, and concentrated to obtain a crude product. The crude product was purified by silica gel column chromatography (PE: EA=1:1) and dried to give compound 23 (120 mg, yellow solid, yield: 36.0%).

MS m/z(ESI):462.6[M+H]$^+$.

Example 24 N-(1-(1H-indol-3-yl) hexane-2-yl)-6-(4-(dimethylamino) piperidin-1-yl) benzo[b]thiophene-2-carboxamide

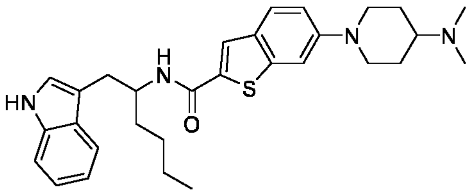

24

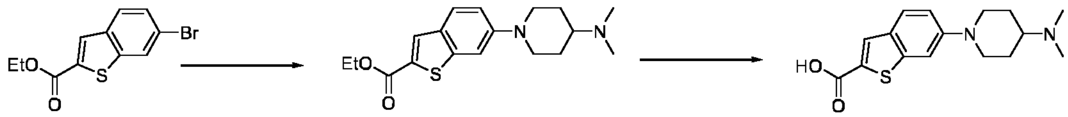

1f

24a

24b

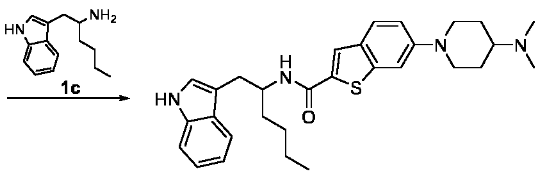

1c

24

Step 1: Ethyl 6-(4-(dimethylamino) piperidin-1-yl) benzo[b]thiophene-2-carboxylate 24a

Compound 1f (400 mg, 1.403 mmol), N, N-dimethylpiperidin-4-amine (269.8 mg, 2.104 mmol), palladium acetate (15.8 mg, 0.070 mmol), Xantphos (81.2 mg, 0.140 mmol), and cesium carbonate (1.371 g, 4.208 mmol) were placed in a single-mouth bottle, and 1,4-dioxane (12 mL) was added after nitrogen replacement. After the second nitrogen replacement, the reaction was carried out at 100°C for 3 hours. LCMS detected that the reaction was complete and the product was generated. The reaction solution was filtered, and the filtrate was concentrated and purified by silica gel column chromatography (DCM/MeOH=10:1) to obtain compound 24a (300 mg, yield 64.3%).

MS m/z(ESI):333.5[M+H]$^+$.

Step 2: 6-(4-(Dimethylamino) piperidin-1-yl) benzo[b]thiophene-2-carboxylic acid 24b

Compound 24a (300 mg, 0.902 mmol) was dissolved in tetrahydrofuran (4 mL), methanol and sodium hydroxide (180.5 mg, 4.512 mmol) in water (4 mL) were added, and the mixture was reacted at 70°C for 1 hour. LCMS detected that the reaction was complete and the product was generated. The reaction solution was concentrated to remove tetrahydrofuran and methanol, and water (10 mL) was added. The pH was adjusted to 6-7 with 1M hydrochloric acid, and the solid precipitated and filtered. After drying the filter cake, compound 24b (210 mg, gray solid, crude product) was obtained.

MS m/z(ESI):305.1[M+H]$^+$.

Step 3: N-(1-(1H-indol-3-yl) hexane-2-yl)-6-(4-(dimethylamino) piperidin-1-yl) benzo[b]thiophene-2-carboxamide 24

Compound 24b (110 mg, 0.361 mmol) and compound 1c (78.2 mg, 0.361 mmol) were dissolved in DMF (3

mL), DIEA (140.1 mg, 1.084 mmol) and HATU (137.4 mg, 0.361 mmol) were added, and the reaction was carried out at room temperature for 1 hour. LCMS showed that the reaction was complete. The reaction solution was diluted with ethyl acetate (100 mL), and the organic phase was separated and washed with water (50 mL×3). The organic phase was washed with saturated aqueous sodium carbonate solution (50 mL), dried with anhydrous sodium sulfate, concentrated, and purified by silica gel column chromatography (MeOH/DCM, 1:30) to obtain compound 24 (12 mg, yellow solid, yield 6.6%).

MS m/z(ESI):503.3[M+H]$^+$.

Example 25 N-(1-(1H-indol-3-yl) hexane-2-yl)-6-(6- methyl-2,6-diazaspiro [3.3] heptane-2-yl) benzo[b] thiophene-2-carboxamide

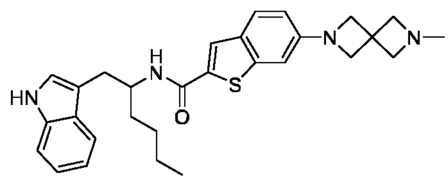

25

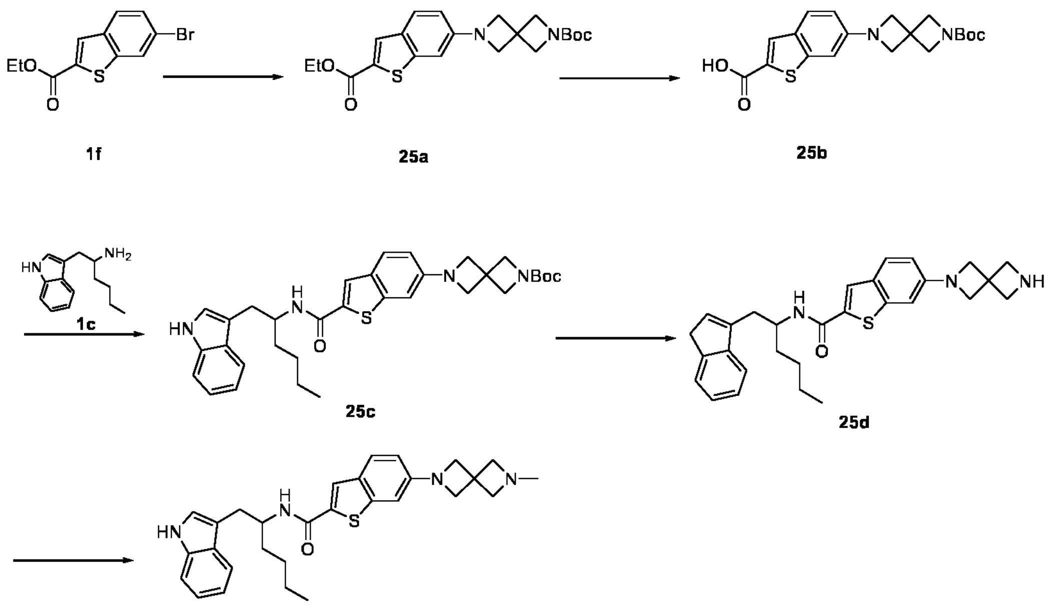


1f 25a 25b 25c 25d

25

Step 1: 6-(2-(ethoxycarbonyl) benzo [b] thiophene -6-yl)-2,6-dinitrogen miscellaneous screw [3.3] heptane-2-tert-butyl carboxylate 25a

Compound 1f (360 mg, 1.261mmol), 2, 6- diazaspiro [3.3] heptane-2-tert-butyl carboxylate (250 mg, 1.261mmol), palladium acetate (14.2 mg, 0.063mmol), Xantphos(73.0 mg,0.126mmol) and cesium carbonate (1.233 g, 3.783mmol) were placed in a single-mouth bottle, 1, 4-dioxane (12 mL)was added after nitrogen replacement, and the reaction was carried out at 100°C for 3 hours after the second nitrogen replacement. LCMS detected that the reaction was complete and the product was generated. The reaction solution was filtered and the filtrate was concentrated. Compound 25a (420 mg, yield 82.8%, yellow solid) was purified by silica gel column chromatography (PE: EA=3:1).

MS m/z(ESI):403.2[M+H]$^+$.

Step2: 6-(6-(tert-butoxycarbonyl)-2,6-diazaspiro [3.3] heptane-2-yl) benzo[b]thiophene-2-carboxylic acid 25b

Compound 25a (210 mg, 0.522mmol) was dissolved in tetrahydrofuran (3 mL) and added into methanol (3 mL) and aqueous solution of sodium hydroxide (104.3 mg, 2.609mmol, 3 mL water). The mixture was reated at 70°C for 1

hour. LCMS detected that the reaction was complete and the product was generated. The reaction solution was concentrated, tetrahydrofuran and methanol were removed, water (10 mL) was added, the pH was adjusted to 6-7 with 1M hydrochloric acid, dichloromethane was extracted, extracted with dichloromethane, and dried with anhydrous sodium sulfate to obtain the product compound 25b (190 mg, yellow solid, crude product).

MS m/z(ESI):375.1$[M+H]^+$.

Step 3: 6-(2-((1-(1H-indole-3-yl) hexane-2-yl) carbamyl benzo [b] thiophene-6-yl)-2,6- diazaspiro [3.3]heptane-2-carboxylic acid tert-butyl ester 25c

Compound 25b (190 mg, 0.507mmol) and compound 1c (131.6 mg, 0.0.608mmol) were dissolved in DMF (6 mL), DIEA (196.6 mg, 1.521mmol) and HATU (192.8mg,0.507mmol) were added, and the reaction was carried out at room temperature for 1 hour. LCMS showed that the reaction was complete. The reaction solution was diluted with ethyl acetate (100 mL), washed with water (30 mL×2), and the organic phase was washed with saturated sodium carbonate solution (50 mL). The organic phase was dried with anhydrous sodium sulfate and concentrated. Compound 25c (290 mg, yellow colloid, 99.8% yield) was purified by silica gel column chromatography (PE: EA=1:1).

MS m/z(ESI):573.3$[M+H]^+$.

Step 4: N-(1-(1H-indole-3-yl) hexane-2-yl)-6-(2,6-diazaspiro [3.3] heptane-2-yl) benzo [b]thiophene -2-carboxamide 25d

Compound 25c (290 mg, 0.506mmol) was dissolved in dichloromethane (5 mL), trifluoroacetic acid (576.9 mg, 5.06mmol) was added, and the mixture was reacted at room temperature for 2 hours until TLC showed that the reaction was complete. The reaction solution was concentrated, dissolved with saturated sodium bicarbonate aqueous solution (50 mL), extracted with dichloromethane (50 mL×3), separated the organic phase and dried with anhydrous sodium sulfate. Concentrated to obtain crude compound 25d (220 mg, yellow solid, crude).

MS m/z(ESI):473.3$[M+H]^+$.

Step5: N-(1-(1H-indole-3-yl) hexane-2-yl)-6-(6-methyl-2,6- diazaspiro [3.3] heptane-2-yl) benzo [b] thiophene-2- carboxamide 25

The compound 25d (220 mg, 0.465mmol) was dissolved in ethanol (6 mL), and paraformaldehyde (30 mg, 1.698mmol), acetic acid (2.8 mg, 0.0465mmol) and sodium cyanoborohydride (29.3 mg, 0.465mmol)were added. The mixture was reacted at room temperature for 1 hour until LCMS showed that the reaction was complete. The reaction solution was poured into water (30 mL) and extracted with dichloromethane (50 mL×3). The organic phase was dried with anhydrous sodium sulfate and filtered to obtain the crude compound, which was purified by silica gel column chromatography (PE: EA=1:1) to obtain the compound 25(38.22 mg, white solid, 15.1% yield).

$^1H$ NMR (400 MHz, DMSO-d6) δ 10.77 (d, J = 2.2 Hz, 1H), 8.29 (d, J = 8.5 Hz, 1H), 7.93 (s, 1H), 7.69 (d, J = 8.7 Hz, 1H), 7.62 (d, J = 7.9 Hz, 1H), 7.32 (d, J = 8.1 Hz, 1H), 7.14 (d, J = 2.3 Hz, 1H), 7.06 (t, J = 7.5 Hz, 1H), 7.01 - 6.90 (m, 2H), 6.60 (dd, J = 8.7, 2.1 Hz, 1H), 4.18 (d, J = 6.7 Hz, 1H), 3.99 (s, 4H), 3.56 (s, 4H), 2.97 (dd, J = 14.4, 6.7 Hz, 1H), 2.86 (dd, J = 14.4, 6.8 Hz, 1H), 2.38 (s, 3H), 1.57 (s, 2H), 1.25 (s, 4H), 0.83 (t, J = 6.8 Hz, 3H).

MS m/z(ESI):487.3$[M+H]^+$.

Example 26 N-(1-(5-fluoro-1H-indole-3-yl) hexane-2-yl)-6-(4-methyl piperazine-1-yl) benzo [b] thiophene -2- carboxamide

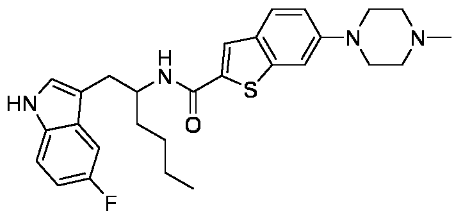

26

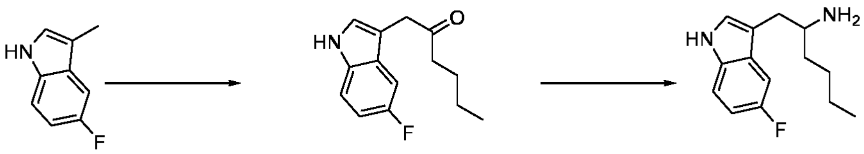

26a 26b 26c

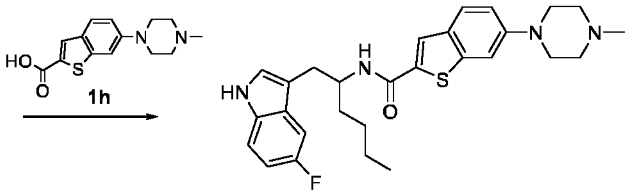

1h

26

Step1: 1-(5-fluoro-1H-indole-3-yl) hexan-2-ketone 26b

Compound 26a (1.0g, 6.704mmol) was dissolved in 1, 2-dichloroethane (20 mL), and aluminum chloride (2.68g, 20.1mmol) was added at 0°C. stirred at room temperature for 30 minutes, then the temperature was lowered to 0°C and valeryl chloride (889 mg, 7.37mmol) was added dropwise, and the reaction was carried out at room temperature for 48 hours. LCMS showed that the reaction was complete. The reaction solution was slowly poured into ice water (50 mL), extracted with dichloromethane (30 mL×2), the organic phase was separated and dried with anhydrous sodium sulfate, and concentrated to obtain a crude product, which was purified by silica gel column chromatography (PE:EA= 1:1) to obtain compound 26b (140 mg, brown colloid, yield 8.95%).

MS m/z(ESI):234.2$[M+H]^+$.

Step2: 1-(5-fluoro-1H-indole-3-yl) hexane-2-amine 26c

Compound 26b (140 mg, 0.6mmol) was dissolved in methanol (5 mL), ammonium acetate (92.5 mg, 1.2mmol) and sodium cyocyanoborohydride (63.8 mg, 1.2mmol) were added, and the mixture was heated to 70°C for 18 hours, LCMS showed that the reaction was complete. The reaction solution was concentrated, and dichloromethane (30 mL) was added. The organic phase was washed with saturated salt water (30 mL×3), dried with anhydrous sodium sulfate, and concentrated to obtain the compound 26c (80 mg, yellow colloid, crude product).

MS m/z(ESI):235.2$[M+H]^+$.

Step3: N-(1-(5-fluoro-1H-indole-3-yl) hexane-2-yl)-6-(4-methylpiperazine-1-yl) benzo [b] thiophene -2- carboxamide 26

Compound 26c (80 mg, 0.341mmol) and compound 1h(94.35 mg, 0.341mmol) were dissolved in DMF(3 mL), DIEA(132.37 mg, 1.024mmol) and HATU(129.82 mg, 0.341mmol) were added, and the reaction was carried out at room temperature for 1h, LCMS showed that the reaction was complete. The reaction solution was diluted with ethyl acetate (50 mL), the organic phase was washed with saturated salt water (30 mL×2), and dried with the anhydrous sodium sulfate concentrated, and purified by silica gel column chromatography (MeOH/DCM=1:10) to obtain compound 26 (5.23mg, white solid, yield 3.11%) .

$^1$H NMR (400 MHz, Methanol-d4) δ 7.57 (d, J = 8.9 Hz, 1H), 7.48 (s, 1H), 7.24 (ddd, J = 9.0, 5.9, 3.6 Hz, 2H), 7.14 (d, J = 2.2 Hz, 1H), 7.10 (s, 1H), 6.93 - 6.80 (m, 2H), 4.34 (t, J = 7.2 Hz, 1H), 3.27 - 3.13 (m, 4H), 3.07 - 2.88 (m, 2H), 2.85 (s, 3H), 2.19 - 1.92 (m, 1H), 1.63 (dt, J = 13.7, 7.7 Hz, 1H), 1.30 (dq, J = 22.0, 7.6 Hz, 2H), 1.20 (s, 6H), 0.83 (t, J = 7.1 Hz, 3H).

MS m/z(ESI):493.3$[M+H]^+$.

Example 27 N-(1-(1H-indole-3-yl) hexan-2-yl)-6-(4-methylpiperazine-1-yl) benzofuran-2- carboxamide

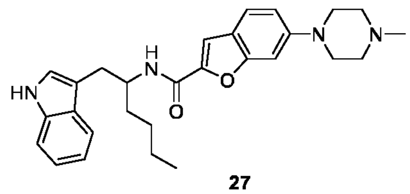

27

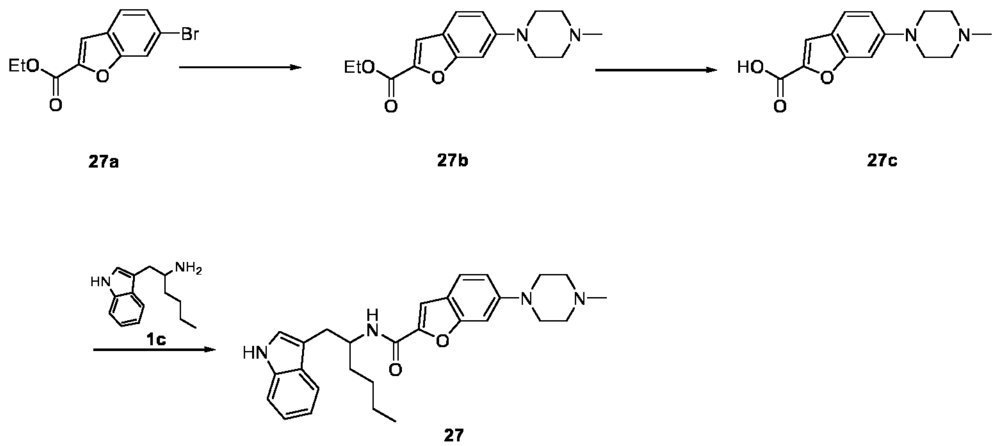


27a 27b 27c

27

Step1: 6-(4-methylpiperazine-1-yl) benzofuran-2-carboxylate ethyl ester 27b

Compounds 27a (1000 mg, 3.6mmol), palladium acetate (40 mg, 0.2mmol), Xantphos(202 mg, 0.35mmol), cesium carbonate (3.42 g, 10.5mmol) were added to the reaction bottle, 1-methylpiperazine (530 mg, 5.3mmol) , 1, 4-dioxane (10 mL) were added, nitrogen protection, stirring on, reaction at 100°C for 3 hours. After the reaction, water was added (50mL) to quench the reaction, ethyl acetate (40 mL×3) was extracted, organic layer was combined, dried with anhydrous sodium sulfate, and purified by silica gel column chromatography (PE: EA=1:1) to obtain compound 27b (650 mg, yield 78%).
MS m/z (ESI):288.2[M+H]$^+$.

Step2: 6-(4-methylpiperazine-1-yl) benzofuran-2-carboxylic acid 27c

Compound 27b (450 mg, 2.0mmol) was dissolved in tetrahydrofuran (5 mL), and methanol (5 mL) was added. Sodium hydroxide was dissolved in water (3 mL), then added to the reaction bottle, and the reaction was started and stirred at 70°C for 1 hour. After the reaction, the solvent was spin dried, the solid was dissolved with water, and the pH was adjusted to 5-6 with 1M hydrochloric acid, then the filter cake was dissolved with methanol (20 mL). The solvent was evaporated to obtain compound 27c (380 mg, yield 92%).
MS m/z (ESI):261.3 [M+H]$^+$.

Step3: N-(1-(1H-indole-3-yl) hexan-2-yl)-6-(4-methylpiperazine-1-yl) benzofuran-2-carboxamide 27

Compound 27c (308 mg, 1.2mmol) and compound 1c (200 mg, 0.9mmol) were added to the reaction bottle, DMF (5 mL) was added to dissolve, and triethylamine (467 mg, 4.6mmol) and 1-propylphosphate acid cyclic anhydride (441 mg, 1.4mmol) were added and stirring was started. The reaction was reacted at room temperature for 1 hour. After the reaction was completed, water (50 mL) was added to quench the reaction, and ethyl acetate (40 mL×3) was used for extraction. The organic layers were combined, dried with anhydrous sodium sulfate, concentrated, and purified and separated by preparative HPLC to obtain compound 27 (110 mg, 25% yield).
MS m/z (ESI):459.3[M+H]$^+$.

Example 28 N-(1-(1H-indole-3-yl) hexane-2-yl)-6-(4-methylpiperazine-1-yl)-1H-indole-2- carboxamide

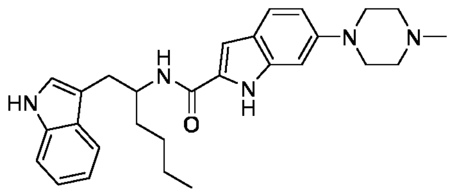

28

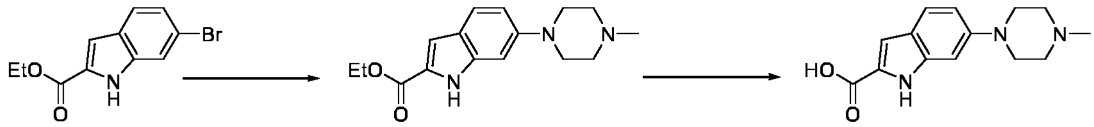

28a 28b 28c

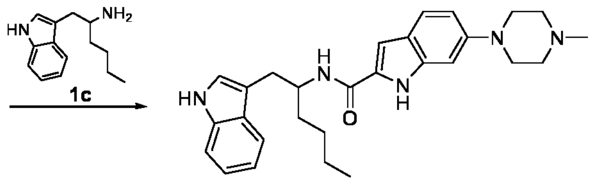


28

Step1: 6-(4-methylpiperazine-1-yl) -1H-indole-2-carboxylate ethyl ester 28b

Compounds 28a (1000 mg, 3.9mmol), palladium acetate (40 mg, 0.2mmol), Xantphos (202 mg, 0.35mmol), cesium carbonate (3.42 g, 10.5mmol) were added to the reaction bottle, 1-methylpiperazine (530 mg, 5.3mmol) and 1, 4-dioxane (10 mL) were added, nitrogen protection, stirring on, and the reaction was carried out at 70°C for 3 hours. After the reaction, water was added (50mL) to quench the reaction, ethyl acetate (40 mL×3) was extracted. The organic layer were combined, dried with anhydrous sodium sulfate, and then purified by silica gel column chromatography (PE: EA=1:1) to obtain compound 28b (350 mg, yield 39%).

MS m/z (ESI):288.2[M+H]$^+$.

Step2: 6-(4-methylpiperazine-1-yl) -1H-indole-2-carboxylic acid 28c

Compound 28b (350 mg, 2.0mmol) was dissolved in 5 mL of tetrahydrofuran, then 5 mL of methanol was added, sodium hydroxide was dissolved in 3 mL water and added to the reaction bottle, stirred, and reacted at 70°C for 1 hour. After the reaction, the solvent was dried by rotary evaporation, the solid was dissolved with water, and the pH was adjusted to 5-6 with 1M hydrochloric acid, filtered, and the filter cake was dissolved in methanol (30 mL). The solvent was evaporated to obtain 28c (210 mg, 77% yield).

MS m/z (ESI):260.3[M+H]$^+$.

Step3: N-(1-(1H-indole-3-yl) hexane-2-yl)-6-(4-methylpiperazine-1-yl)-1H-indole-2-carboxylic acid 28

Compound 28c (200 mg, 0.77mmol) and compound 1c (0.15 mg, 0.77mmol) were added to the reaction bottle, DMF (5 mL) was added to dissolve, triethylamine (467 mg, 4.6mmol) and 1-propylphosphoric acid cyclic anhydride (441 mg, 1.4mmol) were added, stirring was started, and the reaction was carried out at room temperature for 1 hour. After the reaction was completed, water (50 mL) was added to quench the reaction, and ethyl acetate (40 mL×3) was used for extraction. The organic layers were combined, dried with anhydrous sodium sulfate, concentrated, and purified and separated by preparative HPLC to obtain compound 28 (90 mg, yield 25%).

$^1$H NMR (400 MHz, DMSO-d6) δ 10.8 (s, 1H), 10.65 (s, 1H),7.85-7.70(d, J = 8 Hz 1H), 7.52-7.15(m, 6H), 6.90-6.72(m, 2H), 6.42(m, 1H), 4.42 - 4.30 (m, 1H), 3.26-3.12(m, 4H), 2.98-2.74(m, 2H), 2.52-2.48(m, 4H), 2.28(s, 3H), 1.67-1.53(m, 2H), 1.34-1.20(m, 4H), 0.87(t, J = 7.2 Hz,3H).

MS m/z (ESI): 458.2 [M+H]$^+$.

Example 29 N-(1-(1H-indole-3-yl) hexane-2-yl)-2-(4-methylpiperazine-1-yl) thieno [2, 3-d] pyrimidine-6- carboxamide

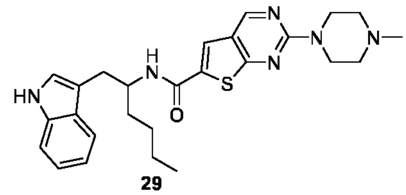

29

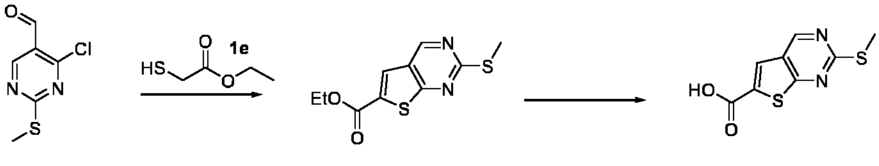


29a 29b 29c

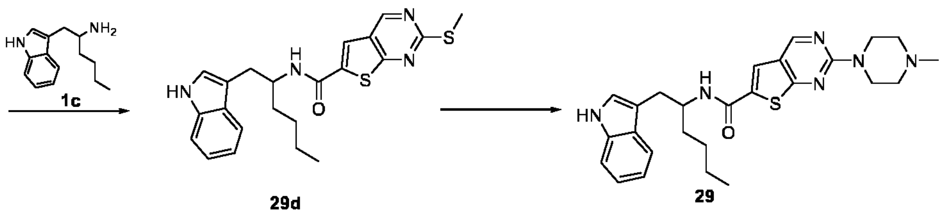


29d 29

Step1: 2-(methylthio) thieno [2,3-d] pyrimidine-6-carboxylate ethyl ester 29b

Compound 29a (1.0 g, 5.682mmol) was dissolved in DMF (5 mL), then potassium carbonate (1.18 g, 0.853mmol) and compound 1e (0.75 g, 6.25mmol) were added at room temperature. Finally, the reaction mixture was stirred at room temperature for 2 hours. The reaction mixture was quenched with water (50 mL), extracted with ethyl acetate (50 mL×3), and the organic phase was washed with saturated salt water (50 mL×3), dried with anhydrous sodium sulfate, filtered, concentrated, and purified by silica gel column chromatography (PE:EA=5:1) to obtain compound 29b (0.75 g, yield 60%).

MS m/z (ESI):255.3$[M+H]^+$.

Step2: 2-(methylthio) thieno [2,3-d] pyrimidine-6-carboxylate acid 29c

Compound 29b (750 mg, 2.0mmol) was dissolved in 5 mL tetrahydrofuran, then 5 mL methanol was added, sodium hydroxide was dissolved in 3 mL water and added to the reaction bottle, stirred, and reacted at 70 ° C for 1 hour. After the reaction, the solvent was dried by rotary evaporation, the solid was dissolved in water, and then 1 M hydrochloric acid was used to adjust pH to 5-6, filtered, and the filter cake was dissolved in methanol (20 mL). The solvent was evaporated to obtain compound 29c (650 mg, yield 91%).

MS m/z (ESI):227.4$[M+H]^+$.

Step3: N-(1-(1H-indole-3-yl) hexane-2-yl)-2-(methylthio) thieno [2,3-d] pyrimidine-6- carboxamide 29d

Compound 29c (200 mg, 0.77mmol) and compound 1c (0.15 mg, 0.77mmol) were added to the reaction bottle, 5 mL DMF was added to dissolve, and triethylamine (467 mg, 4.6mmol) and 1-propylphosphoric acid cyclic anhydride (441 mg, 1.4mmol) were added, and stirring was started. The reaction was reacted at room temperature for 1 hour. After the reaction was completed, water (50 mL) was added to quench the reaction, and ethyl acetate (50 mL×3) was used for extraction. The organic layers were combined, dried with anhydrous sodium sulfate, concentrated, and purified by silica gel column chromatography (PE:EA = 5:1) to obtain compound 29d (190 mg, yield 65%).

MS m/z (ESI):458.3$[M+H]^+$.

Step4: N-(1-(1H-indole-3-yl) hexane-2-yl)-2-(4-methylpiperazine-1-yl) thieno [2, 3-d] pyrimidine-6- carboxamide 29

Compound 29d (0.19 g, 2.427mmol) and 1-methylpiperazine (0.310 g, 3.09mmol) were placed in a microwave reactor and reacted at 180°C for 3 hours. The reaction mixture was quenched with water (30 mL), extracted with ethyl acetate (50 mL×3), and the organic phase was washed with saturated salt water (50 mL×3), dried with anhydrous sodium sulfate, filtered, concentrated, and then purified and separated by preparative HPLC to obtain compound 29 (29 mg, yield 15%).
MS m/z (ESI):477.5 $[M+H]^+$.

Example 30 N-(1-(5-methyl-1H-indole-3-yl) hexane-2-yl) -6-(4-methylpiperazine-1-yl) benzo[b] thiophene-2- carboxamide

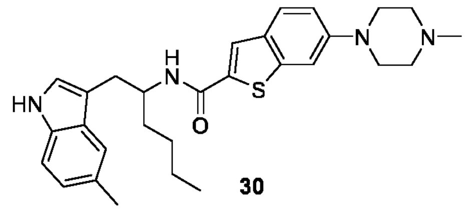

30

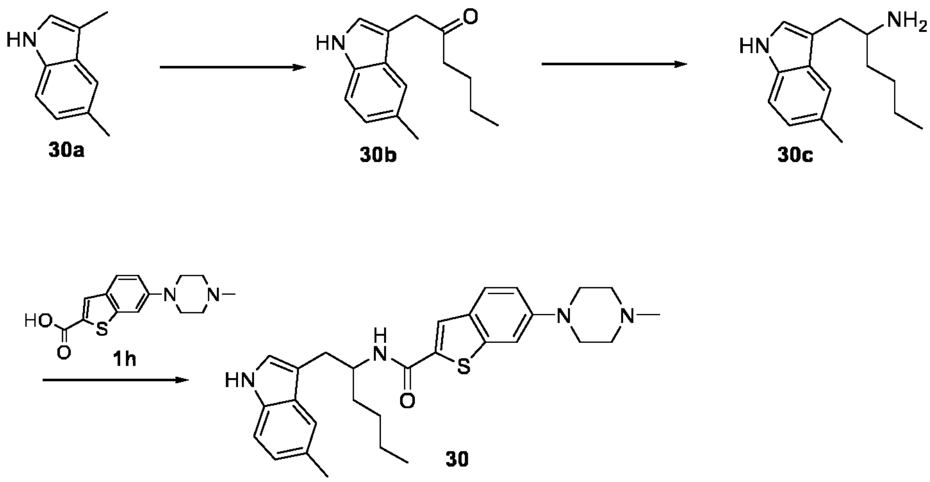

30a 30b 30c 1h 30

Step1: 1-(5-methyl-1H-indole-3-yl) hexane-2-ketone 30b

Compound 30a (1.0 g, 6.704mmol) was dissolved in 1,2-dichloroethane (20 mL), aluminum chloride (2.68 g, 20.1mmol) was added at 0°C, stirred at room temperature for 30 minutes, then the temperature was lowered to 0°C and valeryl chloride (889 mg, 7.37mmol) was added dropwise, and the reaction was carried out at room temperature for 48 hours. LCMS showed that the reaction was complete. The reaction solution was slowly poured into ice water (50 mL), extracted with dichloromethane (50 mL), and the organic phase was dried with anhydrous sodium sulfate and concentrated to obtain a crude product. The crude product was purified by silica gel column chromatography (PE: EA=1:1) to obtain compound 30b (210 mg, yield 12%).
MS m/z(ESI):231.2$[M+H]^+$.

Step2: 1-(5-methyl-1H-indole-3-yl) hexane-2-amine 30c

Compound 30b (210 mg, 0.9mmol) was dissolved in methanol (5 mL), and ammonium acetate (92.5 mg, 1.2mmol) and sodium cyanoborohydride (63.8 mg, 1.2mmol) were added. The mixture was heated to 70 °C for 18 hours. LCMS showed that the reaction was complete. The reaction solution was concentrated, and dichloromethane (30 mL) was added. The organic phase was washed with saturated salt water (40 mL×3), dried with anhydrous sodium sulfate, and concentrated to obtain compound 30c (120 mg, yellow colloid, crude product).
MS m/z(ESI):232.3$[M+H]^+$.

Step3: N-(1-(5-methyl-1H-indole-3-yl) hexane-2-yl)-6-(4-methylpiperazine-1-yl) benzo[b] thiophene-2-carboxamide 30

Compound 30c (120 mg, 0.441mmol) and compound 1h (100 mg, 0.441mmol) were dissolved in DMF (3 mL), and DIEA (132.37 mg, 1.024mmol) and HATU (129.82 mg, 0.341mmol) were added. The mixture was reacted for 1 hour at room temperature. LCMS showed that the reaction was complete. The reaction solution was diluted with ethyl acetate (50 mL), and the organic phase was washed with saturated salt water (50 mL$\times$3), dried with anhydrous sodium sulfate, filtered, concentrated, and separated by preparative HPLC to obtain compound 30 (15 mg, white solid, yield 11%).

$^1$H NMR (400 MHz, Methanol-d4) $\delta$ 7.68 (d, J = 8.9 Hz, 1H), 7.44 (s, 1H), 7.34 (ddd, J = 9.0, 5.9, 3.6 Hz, 2H), 7.14 (d, J = 2.2 Hz, 1H), 7.10 (s, 1H), 6.93 - 6.80 (m, 2H), 4.34 (t, J = 7.2 Hz, 1H), 3.27 - 3.13 (m, 4H), 3.07 - 2.88 (m, 2H), 2.85 (s, 3H), 2.44 (s, 3H),2.25 - 1.98 (m, 1H), 1.66 (dt, J = 13.7, 7.7 Hz, 1H), 1.47 (dq, J = 22.0, 7.6 Hz, 2H), 1.30 (s, 6H), 0.91 (t, J = 7.1 Hz, 3H).

MS m/z(ESI):489.3[M+H]$^+$.

Example 31 N-(1-(1H-pyrrole[3,2-c] pyridin-3-yl) hexane-2-yl)-6-(4-methyl piperazine-1-yl) benzo[b] thiophene-2- carboxamide

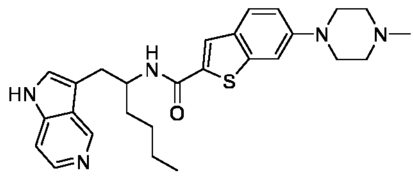

31

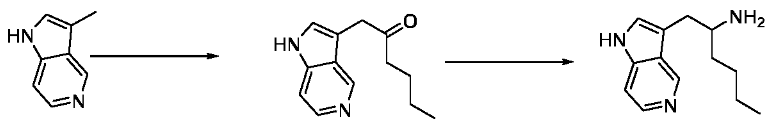

31a 31b 31c

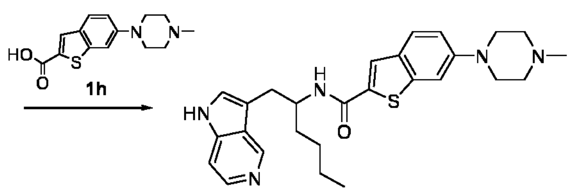

1h

31

Step1: 1-(1H-pyrrole [3, 2-C] pyridine-3-yl) hexan-2-ketone 31b

Compound 31a (1.0 g, 6.804mmol) was dissolved in 1,2-dichloroethane (20 mL), aluminum chloride (2.68 g, 20.1mmol) was added at 0°C, stirred at room temperature for 30 minutes, then the temperature was lowered to 0°C and valeryl chloride (889 mg, 7.37mmol) was added dropwise, and the mixture was reacted at room temperature for 48 hours. LCMS showed that the reaction was complete. The reaction solution was slowly poured into ice water (50 mL), extracted with dichloromethane (30 mL$\times$3), dried with anhydrous sodium sulfate, concentrated, and purified by silica gel column chromatography (PE:EA = 1:1) to obtain compound 31b (250 mg, brown colloid, yield 14%).

MS m/z(ESI):217.3[M+H]$^+$.

Step2: 1-(1H-pyrrole [3, 2-C] pyridin-3-yl) hexane-2-amine 31c

Dissolve compound 31b (250 mg, 1.1mmol) in methanol (5 mL), add ammonium acetate (92.5 mg, 1.2mmol), sodium cyanoborohydride (63.8 mg, 1.2mmol), and heat to 70 ° C for 18 hours. LCMS shows that the reaction is complete. The reaction solution is concentrated, and dichloromethane (30 mL) is added for extraction. The organic phase is washed with saturated salt water (30 mL$\times$3), dried with anhydrous sodium sulfate, and concentrated to obtain compound 31c (140 mg, yellow colloid, crude product).

MS m/z(ESI):218.2 [M+H]+.

Step3: N-(1-(1H- pyrrole [3, 2-c] pyridin-3-yl) hexane-2-yl)-6-(4-methylpiperazine-1-yl) benzo [b] thiophene-2-carboxamide 31

Compound 31c (98 mg, 0.451mmol) and compound 1h (110 mg, 0.451mmol) were dissolved in DMF (3 mL), DIEA(132.37 mg, 1.024mmol) and HATU (129.82 mg, 0.341mmol) were added, and the mixture was reacted for 1 hour at room temperature. LCMS showed that the reaction was complete. After the reaction solution was diluted with ethyl acetate (50 mL), the organic phase was washed with saturated salt water (30 mL×3), dried with anhydrous sodium sulfate, filtered, concentrated, and purified and separated by preparative HPLC to obtain compound 31 (19 mg, white solid, yield 11%).

MS m/z(ESI):476.4 [M+H]+.

Example 32 N-(1-(1H-indol-3-yl)-5-methyl-4-hexene-2-yl)-6-(4-methylpiperazin-1-yl) benzo[b]thiophene-2-carboxamide

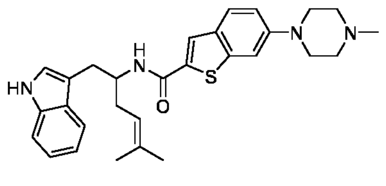

32

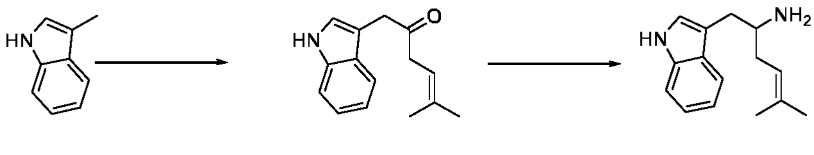

1a 32a 32b

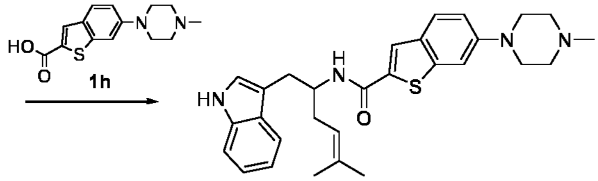

1h

32

Step 1: 1-(1H-indol-3-yl)-5-methyl-4-hexene-2-one 32a

Compound 1a (1.0 g, 7.63mmol) was dissolved in 1,2-dichloroethane (10 mL), aluminum chloride (2.68 g, 20.1mmol) was added at 0 ° C, stirred at room temperature for 30 minutes, then the temperature was lowered to 0 ° C and 4-methyl-3-pentenoyl chloride (889 mg, 9.50mmol) was added dropwise, and the reaction was carried out at room temperature for 48 hours. LCMS showed that the reaction was complete. The reaction solution was slowly poured into ice water (50 mL), extracted with dichloromethane (30 mL × 3), and the organic phase was dried with anhydrous sodium sulfate, concentrated, and purified by silica gel column chromatography (PE: EA = 1: 1) to obtain compound 32a (150 mg, brown colloid, yield 6.8%).

MS m/z(ESI):228.4 [M+H]+.

Step 2: 1-(1H-indol-3-yl)-5-methyl-4-hexene-2-amine 32b

Compound 32a (150 mg, 0.72mmol) was dissolved in methanol (5 mL), and ammonium acetate (92.5 mg, 1.2mmol) and sodium cyanoborohydride (63.8 mg, 1.2mmol) were added. The mixture was heated to 70 °C for 18 hours. LCMS showed that the reaction was complete. The reaction solution was concentrated, and dichloromethane (50 mL)

was added. The organic phase was washed with saturated salt water (30 mL×3), dried with anhydrous sodium sulfate, and concentrated to obtain compound 32b (140 mg, yellow colloid, crude product).

MS m/z(ESI):229.2 $[M+H]^+$.

Step3: N-(1-(1H-indol-3-yl)-5-methyl-4-hexene-2-yl)-6-(4-methylpiperazin-1-yl) benzo[b]

thiophene-2-carboxamide 32

Compound 32c (103 mg, 0.451mmol) and compound 1h (110 mg, 0.451mmol) were dissolved in DMF (3 mL), DIEA (132.37 mg, 1.024mmol) and HATU (145.8 mg, 0.451mmol) were added, and the reaction was carried out at room temperature for 1 hour. LCMS showed that the reaction was complete. After the reaction solution was diluted with ethyl acetate (50 mL), the organic phase was washed with saturated salt water (30 mL×3), dried with anhydrous sodium sulfate, filtered, concentrated, and purified and separated by preparative HPLC to obtain compound 32 (11 mg, white solid, yield 9%).

MS m/z(ESI):487.4 $[M+H]^+$.

Example 33 N-(1-(1H-indol-3-yl)-5-methylhexane-2-yl)-6-(4-methylpiperazin-1-yl) benzo[b]thiophene-2-carboxamide

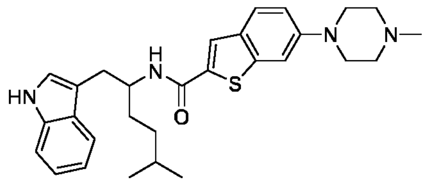

33

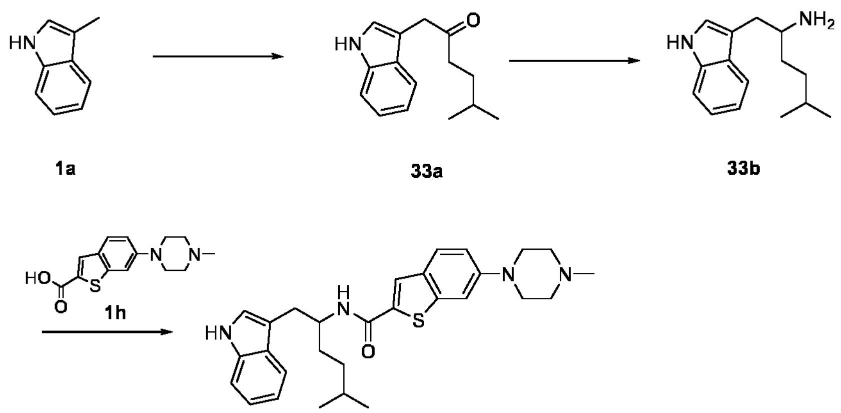

1a 33a 33b

1h

33

Step 1: 1-(1H-indol-3-yl)-5-methylhexane-2-one 33a

Compound 1a (1.0 g, 7.63mmol) was dissolved in 1,2-dichloroethane (10 mL), aluminum chloride (2.68 g, 20.1mmol) was added at 0°C, stirred at room temperature for 30 minutes, then the temperature was lowered to 0°C and 4-methylvaleryl chloride (910 mg, 9.50mmol) was added dropwise, and the mixture was reacted at room temperature for 48 hours. LCMS showed that the reaction was complete. The reaction solution was slowly poured into ice water (50 mL), extracted with dichloromethane (30 mL×3), and the organic phase was dried with anhydrous sodium sulfate, concentrated, and purified by silica gel column chromatography (PE:EA= 1:1) to obtain compound 33a (220 mg, brown colloid, yield 11.5%).

MS m/z(ESI):230.2 $[M+H]^+$.

Step 2: 1-(1H-indol-3-yl)-5-methylhexane-2-amine 33b

Compound 33a (220 mg, 0.92mmol) was dissolved in methanol (5 mL), and ammonium acetate (92.5 mg,

1.2mmol) and sodium cyanoborohydride (63.8 mg, 1.2mmol) were added. The mixture was heated to 70 °C for 18 hours. LCMS showed that the reaction was complete. The reaction solution was concentrated, and dichloromethane (50 mL) was added for extraction. The organic phase was washed with saturated salt water (30 mL × 3), dried with anhydrous sodium sulfate, and concentrated to obtain compound 33b (160 mg, yellow colloid, crude product).

MS m/z(ESI):231.2$[M+H]^+$.

Step3: N-(1-(1H-indol-3-yl)-5-methylhexane-2-yl)-6-(4-methylpiperazine-1-yl) benzo[b]thiophene -2-carboxamide 33

Compound 33b (111 mg, 0.481mmol) and compound 1h (110 mg, 0.481mmol) were dissolved in DMF (3 mL), DIEA(132.37 mg, 1.024mmol) and HATU (145.8 mg, 0.481mmol) were added, and the reaction was carried out at room temperature for 1 hour. LCMS showed that the reaction was complete. After the reaction mixture was diluted with ethylacetate (50 mL), the organic phase was washed with saturated salt water (30 mL×3), dried with anhydrous sodium sulfate, filtered, concentrated, and purified and separated by preparative HPLC to obtain compound 33 (21 mg, white solid, yield 16%).

MS m/z(ESI):489.3$[M+H]^+$.

Example 34 N-[1-(1H-indol-3-yl)-4-methoxybutyl-2-yl]-6-(4-methylpiperazin-1-yl)-1-benzothiophene-2-carboxamide

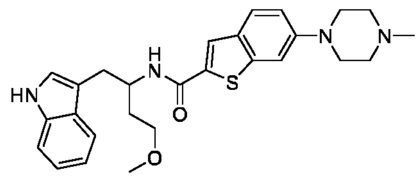

34

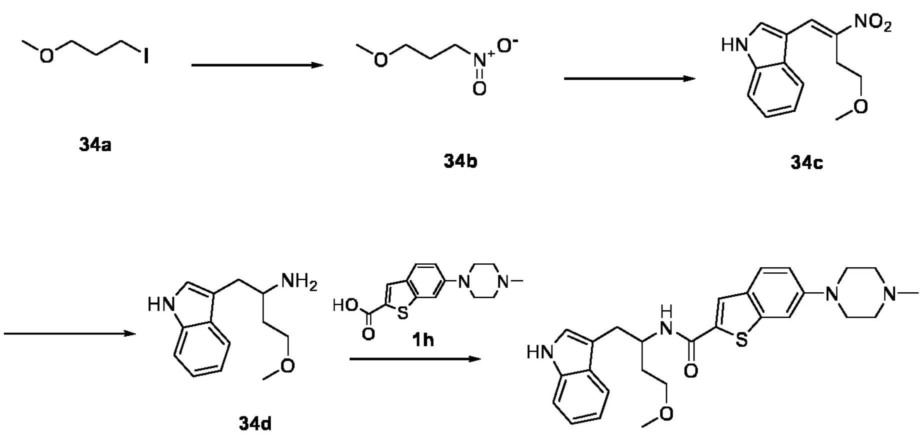

34a

34b

34c

34d

1h

34

Step 1: 1-methoxy-3-nitropropane 34b

methoxy-3-iodopropane 34a (1 g, 5mmol) and silver nitrite (1.54 g, 10mmol) were mixed in water (15 mL), heated to 70°C for 5 hours, and the reaction was completed. After the reaction mixture was filtered, it was extracted with dichloromethane (50 mL), and the dichloromethane layer was concentrated at low temperature to obtain compound 34b (480 mg, yield 80.6%).

Step 2: 3-[(4-methoxy-2-nitrobutyl-1-en-1-yl)-1H-indole 34c

1H-indole-3-carboxaldehyde (240 mg, 1.65mmol) and 1-methoxy-3-nitropropane 34b (480 mg, 4mmol) and ammonium acetate (90 mg, 1.16mmol) were mixed, sealed and heated to 70 °C for 3 hours, and the reaction was completed. After the reaction mixture was diluted with ethylacetate (50 mL), the organic phase was washed with saturated salt water (30 mL×3), concentrated and purified by silica gel column chromatography (PE: EA = 3: 1) to obtain compound 34c (220 mg, yield 54%).

MS m/z (ESI): 247.1 $[M+H]^+$.

Step 3: 1-(1H-indol-3-yl)-4-methoxybutyl-2-amine 34d

Compound 34c (220 mg, 0.89mmol) was dissolved in anhydrous tetrahydrofuran (3 mL), 1M lithium aluminum hydride solution (2.5 mL) was added at 70°C, and stirred at room temperature overnight, and the reaction was completed. After adding 15% sodium hydroxide solution (3 mL) to the reaction mixture to quench the reaction, dichloromethane (50 mL) was added for extraction, and the organic phase was washed with saturated salt water (30 mL×3), concentrated, and purified by silica gel column chromatography (PE:EA = 3:1) to obtain compound 34d (135 mg, yield 69.2%).

MS m/z(ESI):219.1$[M+H]^+$.

Step 4: N-[1-(1H-indol-3-yl)-4-methoxybutyl-2-yl]-6-(4-methylpiperazin-1-yl)-1-benzothiophene -2-carboxamide 34

Compound 34d (135 mg, 0.62mmol) and 6-(4-methylpiperazin-1-yl)-1-benzothiophene-2-carboxylic acid (171 mg, 0.62mmol) were mixed in DMF (3 mL), DIEA (0.2 mL, 1.2mmol) was added, and then HATU (235 mg, 0.62mmol) was added. The reaction mixture was stirred at room temperature for 2 hours, and the reaction was completed. The reaction mixture was diluted with ethyl acetate (50 mL), the organic phase was washed with saturated salt water (30 mL×3), dried with anhydrous sodium sulfate, and the crude product obtained after concentration was purified by silica gel column chromatography (PE:EA= 1:1) to obtain compound 34 (143 mg, yield 48.4%).

MS m/z(ESI):477.2$[M+H]^+$.

Example 35 (Z)-N-(1-(1H-indol-3-yl) -4-hexene-2-yl)-6-(4-methylpiperazin-1- yl) benzo[b]thiophene-2-carboxamide

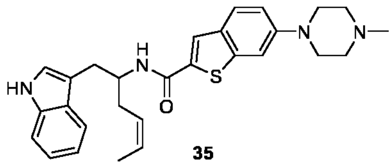

35

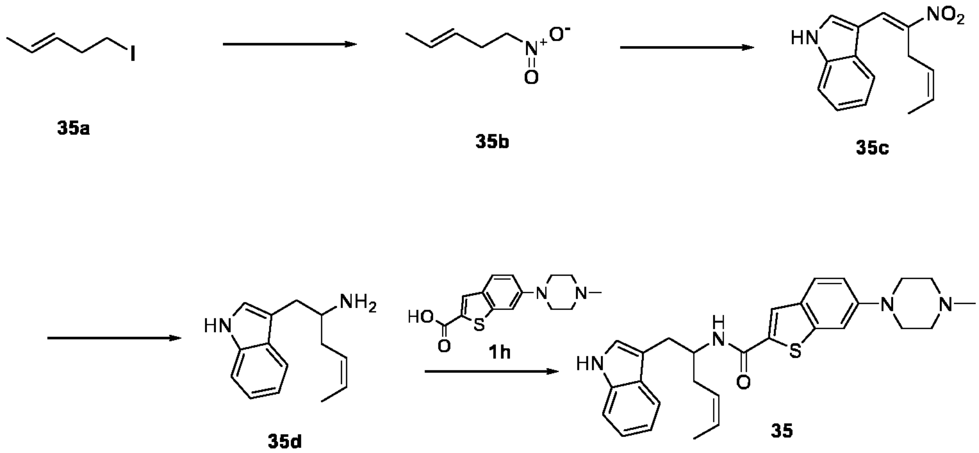

35a

35b

35c

35d

1h

35

Step 1: 5-Nitropent-2-ene 35b

Mix compound 35a (1 g, 5.1mmol) and silver nitrite (1.54 g, 10mmol) in water (15 mL), heat to 70°C for 5 hours, and the reaction is complete. After the reaction mixture was filtered, it was extracted with dichloromethane (30 mL×3), and the dichloromethane layer was concentrated at low temperature to obtain compound 35b (480 mg, yield 82.6%).

Step 2: 3-((1E, 4Z)-2-nitrohexa-1,4-diene-1-yl)-1H-indole 35c

1H-indole-3-carboxaldehyde (240 mg, 1.65mmol) and compound 35b (480 mg, 4mmol) and ammonium acetate (90 mg, 1.16mmol) were mixed, sealed and heated to 70°C for 3 hours, and the reaction was completed. After the

reaction mixture was diluted with ethylacetate (50 mL), the organic phase was washed with saturated salt water (30 mL×3), concentrated and purified by silica gel column chromatography (PE: EA = 3: 1) to obtain compound 35c (180 mg, yield 46%).

MS m/z (ESI): 243.1 [M+H] +.

Step 3: (Z)-1-(1H-indol-3-yl)-4- hexene-2-amine 35d

Compound 35c (180 mg, 0.74mmol) was dissolved in anhydrous tetrahydrofuran (3 mL), 1 M lithium aluminum hydride solution (2.5 mL) was added at 70 °C, and the reaction was stirred at room temperature overnight. Adding 15% sodium hydroxide solution (3 mL) to the reaction mixture to quench the reaction, dichloromethane (50 mL) was added for extraction, and the organic phase was washed with saturated salt water (30 mL×3), concentrated, and purified by silica gel column chromatography (PE: EA = 1: 1) to obtain compound 35d (95 mg, yield 59%).

MS m/z (ESI): 215.1 [M+H] +.

Step 4: (Z)-N-(1-(1H-indol-3-yl)-4-hexene-2-yl)-6-(4-methylpiperazin-1-yl) benzo[b] thiophene-2-carboxamide 35

Compound 35d (95 mg, 0.44mmol) and compound 1h (143 mg, 0.50mmol) were mixed in DMF (3 mL), DIEA (0.2 mL, 1.2mmol) was added, and then HATU (235 mg, 0.62mmol) was added. The reaction mixture was stirred at room temperature for 2 hours, and the reaction was completed. After the reaction mixture was diluted with ethyl acetate (50 mL), the organic phase was washed with saturated salt water (30 mL×3), dried with anhydrous sodium sulfate, and the crude product obtained after concentration was purified by silica gel column chromatography (PE:EA= 1:1) to obtain compound 35 (143 mg, yield 48.4%).

MS m/z(ESI):473.2[M+H]+.

Example 36 N-[1-(1H-indol-3-yl) hexyl-2-yl]-1-methyl-6-(4-methylpiperazin-1-yl) -1H-indole-2-carboxamide

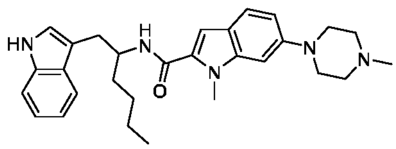

36

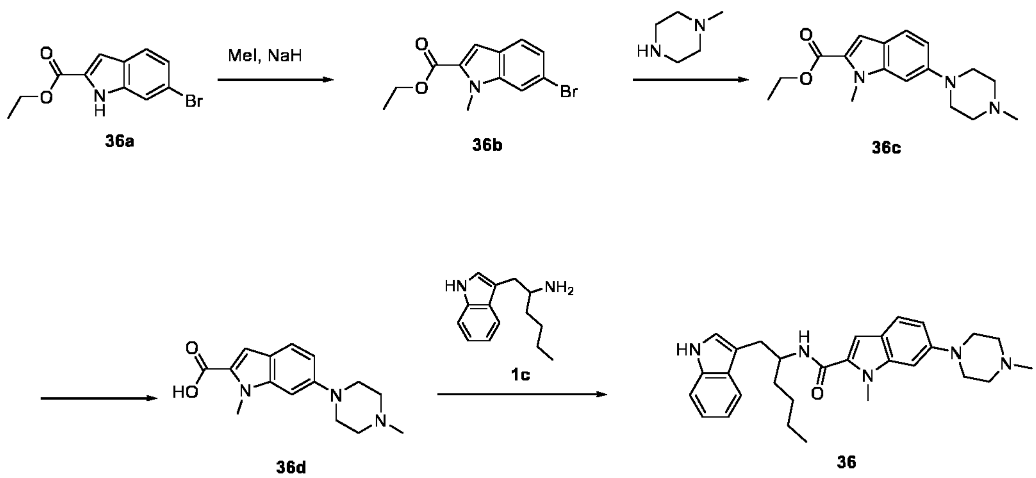


36a 36b 36c 1c 36d 36

Step 1: 6-bromo-1-methyl-indole-2-carboxylic acid ethyl ester 36b

Compound 36a (400 mg, 1.5mmol) was dissolved in anhydrous DMF (5 mL), sodium hydride (72 mg, 1.8mmol) was added, stirred for 10 minutes, iodomethane (0.123 mL, 2mmol) was added, and the reaction was continued to stir for 2 hours. The reaction was completed. Water (1 mL) was added to quench the reaction, the reaction mixture was diluted with ethyl acetate (50 mL), the organic phase was washed with saturated salt water (30 mL × 3), dried and

concentrated, and the crude product was purified by silica gel column chromatography (PE: EA = 1: 1) to obtain compound 36b (330 mg, yield 78%).

MS m/z (ESI): 283.0 $[M+H]^+$.

Step 2: 1-Methyl-6-(4-methylpiperazine-1-yl)-indole-2-carboxylic acid ethyl ester 36c

Compound 36b (330 mg, 1.17mmol), 1-methylpiperazine (117 mg, 1.17mmol), palladium acetate (25 mg, 0.11mmol), Xantphos (100 mg, 0.17mmol) and cesium carbonate (1.14 g, 3.5mmol) were mixed in 1,4-dioxane (10 mL), replaced with nitrogen protection, heated to 110 ° C for 4 hours, and the reaction was completed. The reaction mixture was concentrated and diluted with ethyl acetate (50 mL), washed with water (30 mL×2), dried and then spin-dried. The crude product was purified by silica gel column chromatography (PE: EA = 2: 1) to obtain compound 36c (280 mg, yield 79.5%).

MS m/z (ESI): 302.1 $[M+H]^+$.

Step 3: 1-Methyl-6-(4-methylpiperazin-1-yl)-indole-2-carboxylic acid 36d

Compound 36c (280 mg, 0.93mmol) was dissolved in tetrahydrofuran (10 mL), sodium hydroxide (200 mg, 5mmol) was dissolved in water (5 mL) and added there to, and heat to 70°C for 3 hours, and the reaction is complete. After the reaction mixture was concentrated to remove tetrahydrofuran, the pH was adjusted to 6 with 1M hydrochloric acid, solid precipitated and filtered to obtain compound 36d (210 mg, yield 82.7%).

MS m/z(ESI):274.2$[M+H]^+$.

Step 4: N-[1-(1H-indol-3-yl) hexyl-2-yl]-1-methyl-6-(4-methylpiperazin-1-yl)-1H-indole-2-carboxamide 36

Compound 36d (136 mg, 0.5mmol) was mixed with 1-(1H-indol-3-yl) hexyl-2-amine (108 mg, 0.5mmol) in DMF (3 mL), and DIEA (0.17 mL, 1mmol) was added, then HATU (190 mg, 0.5mmol) was added. The reaction mixture was stirred at room temperature for 2 hours, and the reaction was completed. The reaction mixture was diluted with ethyl acetate (50 mL), and the organic phase was washed with saturated salt water (30 mL×3), dried with anhydrous sodium sulfate, and concentrated to obtain a crude product which was purified by silica gel column chromatography (PE: EA = 1: 1) to obtain compound 36 (95 mg, yield 40.4%).

$^1H$ NMR (400 MHz, DMSO-d6) δ 10.8 (s, 1H), 7.80-7.64(d, J = 8 Hz 1H), 7.50-7.12(m, 6H), 6.92-6.70(m, 2H), 6.48-6.40(m, 1H), 4.42-4.30 (m, 1H), 3.45(s, 1H), 3.30-3.10(m, 4H), 2.96-2.72(m, 2H), 2.56-2.42(m, 4H), 2.28(s, 3H), 1.68-1.50(m, 2H), 1.36-1.20(m, 4H), 0.88(t, J = 7.2 Hz,3H).

MS m/z (ESI): 472.2 $[M+H]^+$.

Example 37 (E)-N-(1-(1H-indol-3-yl)-3-hexene-2-yl)-6-(4-methylpiperazin-1-yl) benzo-[b]thiophene-2-carboxamide

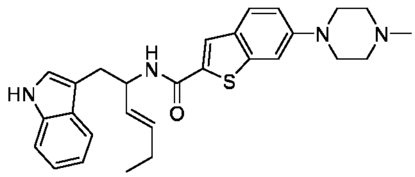

**37**

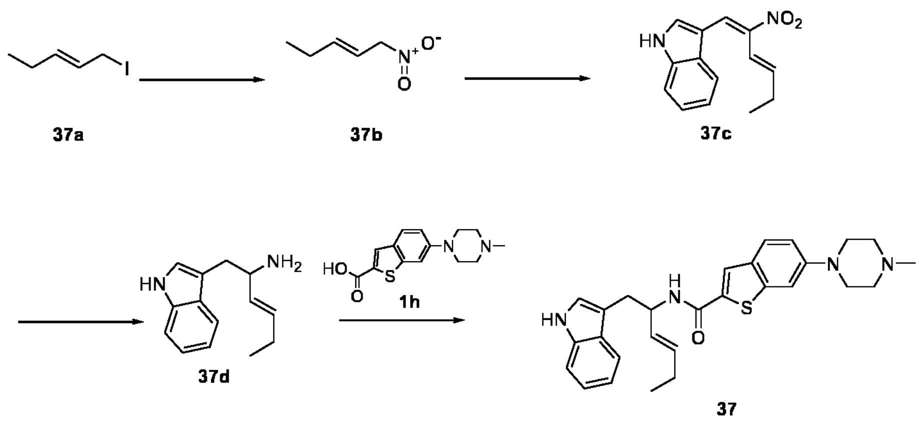

37a 37b 37c

37d 1h 37

Step 1: (E)-1-Nitropent-2-ene 37b

Mix compound 37a (1 g, 5.1mmol) and silver nitrite (1.54 g, 10mmol) in water (15 mL), heated to 70°C for 5 hours, and the reaction is complete. After the reaction mixture was filtered, it was extracted with dichloromethane (50 mL), and the dichloromethane layer was concentrated at low temperature to obtain compound 37b (480 mg, yield 82.6%).

Step 2: 3-((1E,3E)-2-nitrohexa-1,3-diene-1-yl)-1H-indole 37c

1H-indole-3-carboxaldehyde (240 mg, 1.65mmol), compound 37b (480 mg, 4mmol) and ammonium acetate (90 mg, 1. 16mmol) were mixed, sealed and heated to 70°C for 3 hours, and the reaction was completed. After the reaction mixture was diluted with ethyl acetate (50 mL), the organic phase was washed with saturated salt water (30 mL×3), concentrated and purified by silica gel column chromatography (PE:EA=3:1) to obtain compound 37c (210 mg, yield 51%).

MS m/z(ESI):243.1$[M+H]^+$.

Step 3: (E)-1-(1H-indol-3-yl) -3- hexene-2-amine 37d

Compound 37c (210 mg, 0.81mmol) was dissolved in anhydrous tetrahydrofuran (3 mL), 1M lithium aluminum hydride solution (2.5 mL) was added at 70°C, and stirred at room temperature overnight to complete the reaction. After adding 15% sodium hydroxide solution (3 mL) to the reaction mixture to quench the reaction, dichloromethane (50 mL) was added for extraction, and the organic phase was washed with saturated salt water (30 mL×3), concentrated, and purified by silica gel column chromatography (PE:EA = 1:1) to obtain compound 37d (105 mg, yield 61%).

MS m/z(ESI):215.1$[M+H]^+$.

Step 4: (E)-N-(1-(1H-indol-3-yl) -3- hexene -2-yl)-6-(4-methylpiperazin-1-yl) benzo[b]thiophene -2-carboxamide 37

Compound 37d (105 mg, 0.49mmol) and compound 1h (143 mg, 0.50mmol) were mixed in DMF (3 mL), DIEA (0.2 mL, 1.2mmol) was added, and then HATU (235 mg, 0.62mmol) was added. The reaction mixture was stirred at room temperature for 2 hours, and the reaction was completed. After the reaction mixture was diluted with ethyl acetate (50 mL), the organic phase was washed with saturated salt water (30 mL×3), dried with anhydrous sodium sulfate, and the crude product obtained after concentration was purified by silica gel column chromatography (PE: EA = 1:1) to obtain compound 37 (143 mg, yield 62%).

Example 38 (E)-N-(1-(1H-indol-3-yl)-3-hexene-2-yl)-6-(4-methylpiperazin-1-yl) benzo[b]-thiophene -2-carboxamide

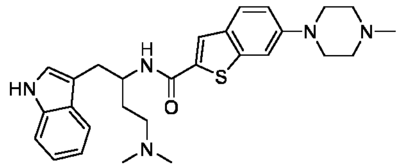

38

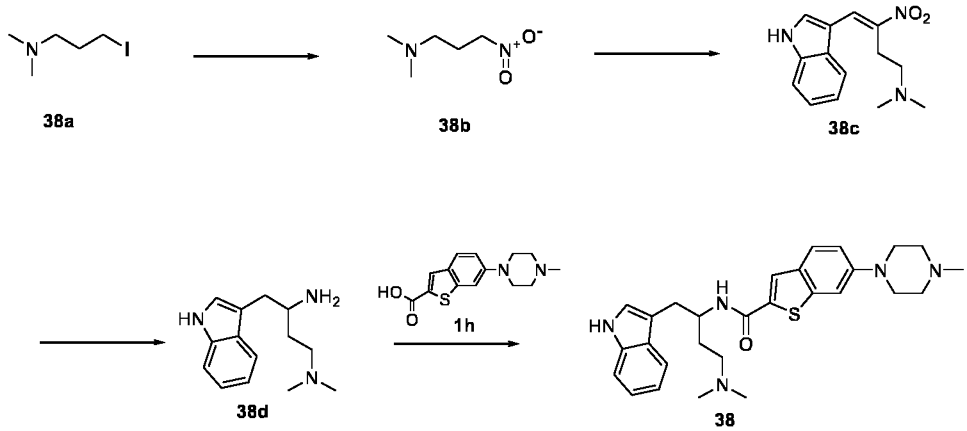

38a 38b 38c 38d 1h 38

Step 1: N, N-Dimethyl-3-nitropropane-1-amine 38b

Compound 38a (1 g, 4.7mmol) and silver nitrite (1.54 g, 10mmol) were mixed in water (15 mL), heated to 70 °C for 5 hours, and the reaction was completed. After the reaction mixture was filtered, it was extracted with dichloromethane (30 mL×3), and the dichloromethane layer was concentrated at low temperature to obtain compound 38b (510 mg, yield 86.2%).

Step 2: (E)-4-(1H-indol-3-yl)-N, N-dimethyl-3-nitrobut-3-ene-1-amine 38c

1H-indole-3-carboxaldehyde (240 mg, 1.65mmol), compound 38b (510 mg, 4.1mmol) and ammonium acetate (90 mg, 1.16mmol) were mixed, sealed and heated to 70 °C for 3 hours, and the reaction was completed. After the reaction mixture was diluted with ethyl acetate (50 mL), the organic phase was washed with saturated salt water (30 mL×3), concentrated and purified by silica gel column chromatography (PE: EA = 1: 1) to obtain compound 38c (210 mg, yield 51%).

MS m/z (ESI): 260.1 [M+H]$^+$.

Step 3: 4-(1H-indol-3-yl) -N1, N1-dimethylbutane-1, 3-diamine 38d

Compound 38c (210 mg, 0.81mmol) was dissolved in anhydrous tetrahydrofuran (3 mL), 1M lithium aluminum hydride solution (2.5 mL) was added at 70 °C, stirred at room temperature overnight, and the reaction was completed. The reaction mixture was quenched by adding 15% sodium hydroxide solution (3 mL), and then extracted with dichloromethane (50 mL). The organic phase was washed with saturated salt water (30 mL×3), concentrated, and purified by silica gel column chromatography (PE: EA = 1: 1) to obtain compound 38d (121 mg, yield 71%).

MS m/z (ESI): 232.1 [M+H]$^+$.

Step 4: (E)-N-(1-(1H-indol-3-yl) 3- hexene-2-yl)-6-(4-methylpiperazin-1-yl) benzo[b]-thiophene-2-carboxamide 38

Compound 38d (105 mg, 0.45mmol) and compound 1h (143 mg, 0.50mmol) were mixed in DMF (3 mL), DIEA (0.2 mL, 1.2mmol) was added, and then HATU (235 mg, 0.62mmol) was added. The reaction mixture was stirred at room temperature for 2 hours, and the reaction was completed. After the reaction mixture was diluted with ethyl acetate (50 mL), the organic phase was washed with saturated salt water (30 mL×3), dried with anhydrous sodium sulfate, and the crude product obtained after concentration was purified by silica gel column chromatography (PE: EA = 1: 1) to obtain compound 38 (143 mg, yield 66.7%).

MS m/z (ESI): 473.2 [M+H]$^+$.

Example 39 N-(1-ethoxy-3-(1H-indol-3-yl) propane-2-yl)-6-(4-methylpiperazin-1-yl) benzo[b]-thiophene-2-carboxamide

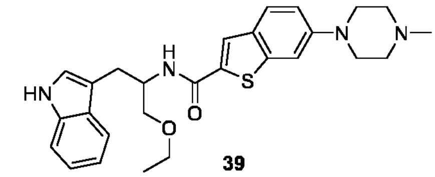

39

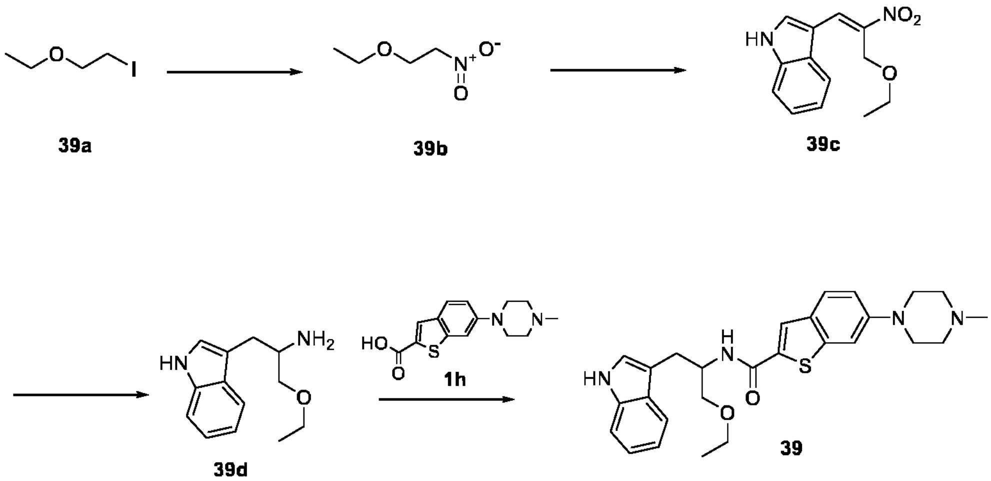

39a 39b 39c

39d 1h 39

Step 1: 1-ethoxy-2-nitroethane 39b

Mix compound 39a (1 g, 5.0mmol) and silver nitrite (1.54 g, 10mmol) in water (15 mL), heat to 70°C for 5 hours, and the reaction is complete. After the reaction mixture was filtered, it was extracted with dichloromethane (30 mL×3), and the dichloromethane layer was concentrated at low temperature to obtain compound 39b (580 mg, yield 92.2%).

Step 2: (E)-3-(3-ethoxy-2-nitroprop-1-en-1-yl)-1H-indole 39c

1H-indole-3-carboxaldehyde (240 mg, 1.65mmol), compound 39b (580 mg, 4. 1mmol) and ammonium acetate (90 mg, 1.16mmol) were mixed, sealed and heated to 70 °C for 3 hours, and the reaction was completed. After the reaction mixture was diluted with ethyl acetate (50 mL), the organic phase was washed with saturated salt water (30 mL×3), concentrated and purified by silica gel column chromatography (PE: EA = 1: 1) to obtain compound 39c (260 mg, yield 51%).

MS m/z (ESI): 247.2[M+H]+.

Step 3: 1-ethoxy-3-(1H-indol-3-yl) propane-2-amine 39d

Compound 39c (260 mg, 1.06mmol) was dissolved in anhydrous tetrahydrofuran (3 mL), 1M lithium aluminum hydride solution (2.5 mL) was added at 70°C, and stirred at room temperature overnight to complete the reaction. After adding 15% sodium hydroxide solution (3mL) to the reaction mixture to quench the reaction, dichloromethane (50mL) was added for extraction, and the organic phase was washed with saturated salt water (30mL×3), concentrated, and purified by silica gel column chromatography (PE: EA = 1:1) to obtain compound 39d (121 mg, yield 52%).

MS m/z(ESI):219.1[M+H]+.

Step 4: N-(1-ethoxy-3-(1H-indol-3-yl) propan-2-yl)-6-(4-methylpiperazin-1-yl) benzo[b]-thiophene-2-carboxamide 39

Compound 39d (120 mg, 0.55mmol) and compound 1h (143 mg, 0.50mmol) were mixed in DMF (3 mL), DIEA (0.2 mL, 1.2mmol) was added, and then HATU (235 mg, 0.62mmol) was added. The reaction mixture was stirred at room temperature for 2 hours, and the reaction was completed. After the reaction mixture was diluted with ethyl acetate (50 mL), the organic phase was washed with saturated salt water (30 mL×3), dried with anhydrous sodium sulfate, and

the crude product obtained after concentration was purified by silica gel column chromatography (PE: EA = 1:1) to obtain compound 39 (85 mg, yield 36%).

MS m/z (ESI): 473.2 $[M+H]^+$.

Example 40 (E)-N-(1-(1H-indol-3-yl)-5-methyl-3-hexene-2-yl)-6-(4-methylpiperazin-yl) benzo[b]-thiophene-2-carboxamide

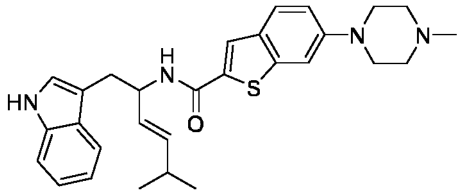

**40**

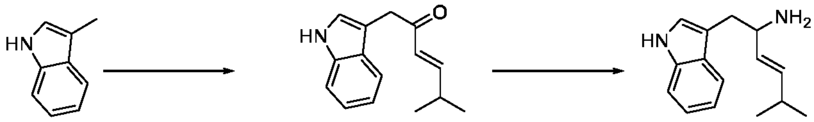

**40a** **40b** **40c**

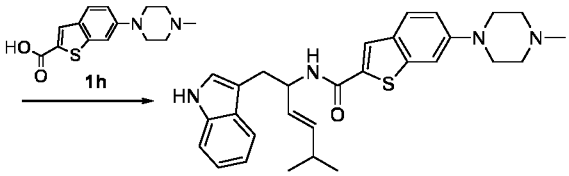

**1h**

**40**

Step 1: (E)-1-(1H-indol-3-yl)-5-methyl-3-hexene-2-one 40b

Compound 40a (1.0 g, 7.63mmol) was dissolved in 1,2-dichloroethane (10 mL), aluminum chloride (2.68 g, 20.1mmol) was added at 0°C, stirred at room temperature for 30 minutes, then (E)-4-methylpent-2-enoyl chloride (889 mg, 9.50mmol) was added dropwise at 0 °C, and reacted at room temperature for 48 hours. LCMS showed that the reaction was complete. The reaction solution was slowly poured into ice water, extracted with dichloromethane (50 mL×3), dried with anhydrous sodium sulfate, concentrated, and purified by silica gel column chromatography (PE:EA= 1:1) to obtain compound 40b (240 mg, brown colloid, yield 14%).

MS m/z(ESI):228.4$[M+H]^+$.

Step 2: (E)-1-(1H-indol-3-yl)-5-methyl-3-hexene-2-amine 40c

Compound 40b (240 mg, 1.06mmol) was dissolved in methanol (5 mL), and ammonium acetate (92.5 mg, 1.2mmol) and sodium cyanoborohydride (63.8 mg, 1.2mmol) were added. The mixture was heated to 70 °C for 18 hours. LCMS showed that the reaction was complete. The reaction solution was concentrated, and dichloromethane (50 mL × 3) was added for extraction. The organic phase was washed with saturated salt water (50 mL), dried with anhydrous sodium sulfate, and concentrated to obtain compound 40c (120 mg, yellow colloid, crude product).

MS m/z(ESI):229.2$[M+H]^+$.

Step 3: (E)-N-(1-(1H-indol-3-yl)-5-methyl-3-hexene-2-yl)-6-(4-methylpiperazin-1-yl) benzo[b]-thiophene-2-carboxamide 40

Compound 40c (120 mg, 0.526mmol) and compound 1h (110 mg, 0.451mmol) were dissolved in DMF (3 mL),

DIEA (132.37 mg, 1.024mmol) and HATU (145.8 mg, 0.451mmol) were added, and the reaction was carried out at room temperature for 1 hour. LCMS showed that the reaction was complete. The reaction solution was extracted with ethyl acetate (50 mL×3), and the organic phase was washed with saturated salt water (50 mL), dried with anhydrous sodium sulfate, filtered, concentrated, and purified and separated by preparative HPLC to obtain compound 40 (12 mg, white solid, yield 5.5%).

MS m/z(ESI):487.4[M+H]+.

Example 41 N-(4-ethoxy-1-(1H-indol-3-yl) butane-2-yl)-6-(4-methylpiperazin-1-yl) benzo[b]-thiophene-2-carboxamide

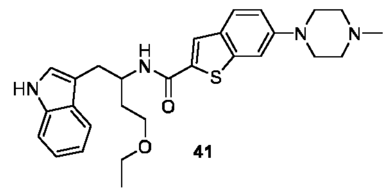

41

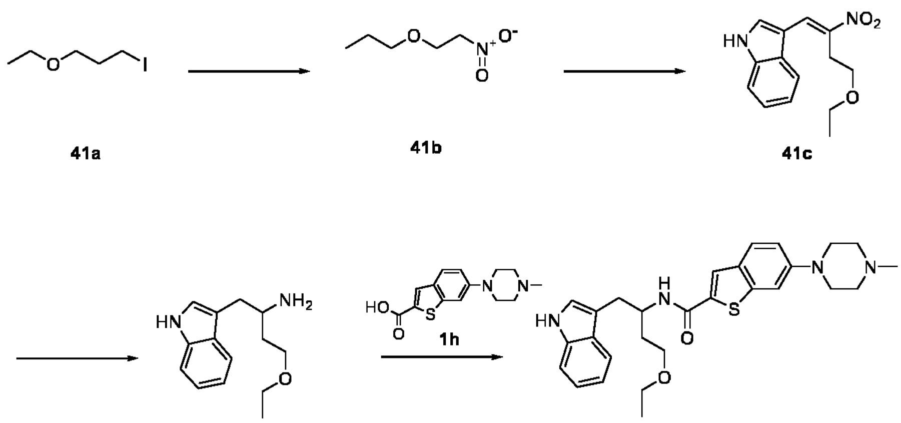

41a 41b 41c

1h

41d 41

Step 1: 1-(2-nitroethoxy) propane 41b

Mix compound 41a (1 g, 4.7mmol) and silver nitrite (1.54 g, 10mmol) in water (15 mL), heat to 70°C for 5 hours, and the reaction is complete. The reaction mixture was filtered and extracted with dichloromethane (50 mL). The dichloromethane layer was concentrated at low temperature to obtain compound 41b (460 mg, yield 86%).

Step 2: (E)-3-(3-ethoxy-2-nitro-1-propylene-1-yl)-1H-indole 41c

1H-Indole-3-carboxaldehyde (240 mg, 1.65mmol), compound 41b (460 mg, 3.8mmol) and ammonium acetate (90 mg, 1.16mmol) were mixed, sealed and heated to 70 °C for 3 hours, and the reaction was completed. The reaction mixture was diluted with ethyl acetate (50 mL), and the organic phase was washed with saturated salt water (30 mL × 3). After concentration, it was purified by silica gel column chromatography (PE: EA = 2: 1) to obtain compound 41c (260 mg, yield 51%).

MS m/z (ESI): 261.2 [M+H]+.

Step 3: 4-ethoxy-1- (1H-indol-3-yl) butane-2-amine 41d

Compound 41c (260 mg, 1.00mmol) was dissolved in anhydrous tetrahydrofuran (3 mL), 1M lithium aluminum hydride solution (2.5 mL) was added at 70 °C, and stirred at room temperature overnight, and the reaction was completed. The reaction mixture was quenched by adding 15% sodium hydroxide solution (3 mL), and then extracted with dichloromethane (50 mL). The organic phase was washed with saturated salt water (30 mL × 3), concentrated, and purified by silica gel column chromatography (PE: EA = 1: 1) to obtain compound 41d (121 mg, yield 52%).

MS m/z (ESI): 233.1 [M+H]+.

Step 4: N-(1-ethoxy-3-(1H-indol-3-yl) propane-2-yl)-6-(4-methylpiperazine-1-yl) benzo[b]-thiophene-2-carboxamide 41

Compound 41d (120 mg, 0.52mmol) and compound 1h (143 mg, 0.50mmol) were mixed in DMF (3 mL), DIEA (0.2 mL, 1.2mmol) was added, and then HATU (235 mg, 0.62mmol) was added. The reaction mixture was stirred at room temperature for 2 hours, and the reaction was completed. After the reaction mixture was diluted with ethyl acetate (50 mL), the organic phase was washed with saturated salt water (30 mL×3), dried with anhydrous sodium sulfate, and concentrated to obtain a crude product which was purified by silica gel column chromatography (PE: EA = 1: 1) to obtain compound 41 (110 mg, yield 46.6%).

MS m/z (ESI): 473.2 $[M+H]^+$.

Example 42 (S)-N-(1-(1H-indol-3-yl) hexane-2-yl)-6-(4-methylpiperazin-1-yl) benzo[b]thiophene-2-carboxamide

Example 43 (R)-N-(1-(1H-indol-3-yl) hexane-2-yl)-6-(4-methylpiperazin-1-yl) benzo[b]thiophene-2-carboxamide

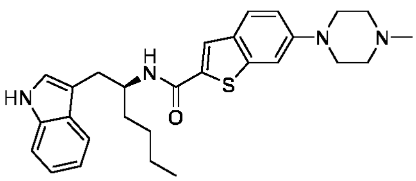

42

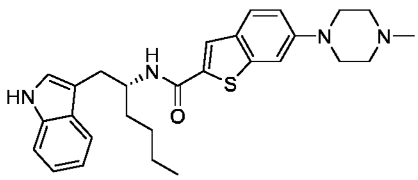

43

The racemic compound 1 (500 mg, 1.053mmol) was chirally resolved to give single compounds of compound 42 (150 mg) and compound 43 (190 mg).

The enantiomers were separated using a Chiralpak AD-H column (250×30 mm) in a THAR 80 preparative SFC. The enantiomers were dissolved in methanol (100 mg/mL) and 100 mg of the enantiomer was loaded per injection. The separation was achieved using a mobile phase of 40% 2-propanol (additive: 0.05% $NH_3$-$H_2O$) in carbon dioxide at a flow rate of 70 g and a system back pressure of 100 bar. The column temperature was maintained at 40°C and the peak was detected at 220 nm. The total cycle time was 6 minutes.

Compound 42 Chiral purity test (OJ-H, 45% MeOH (0.05% $NH_3$-$H_2O$) Peak RT: 1.95 (100%).

Compound 43 Chiral purity test (OJ-H, 45% MeOH (0.05% $NH_3$-$H_2O$) Peak 1 RT: 2.004 (1.16%) Peak 2 RT: 3.04 (98.54%).

Compound 42 MS m/z (ESI): 475.5 $[M+H]^+$.

Compound 43 MS m/z (ESI): 475.5 $[M+H]^+$.

Example 44 6-[4-(Cyclopropanesulfonyl) piperazine-1-yl]-N-[1-(1H-indol-3-yl) hexane-2-yl]-1-benzothiophene-2-carboxamide

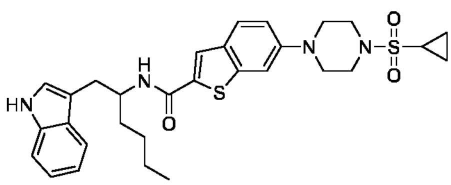

44

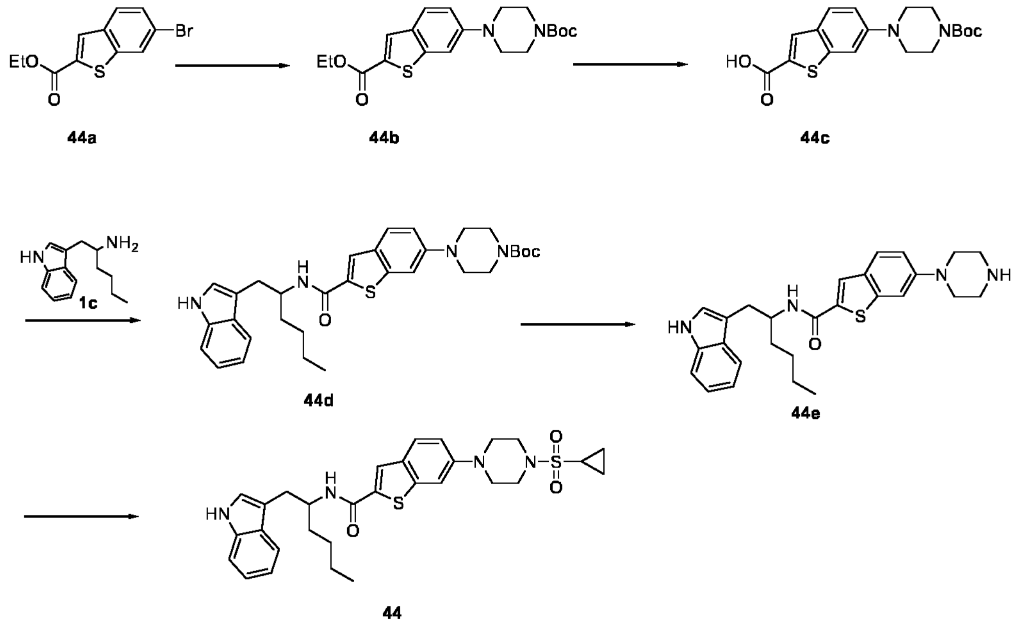

44a 44b 44c

1c

44d 44e

44

Step 1: tert-butyl-4-[2-(ethoxycarbonyl)-1-benzothiophen-6-yl] piperazine-1-carboxylate 44b

Ethyl 6-bromo-1-benzothiophene-2-carboxylate (2000 mg, 7mmol), tert-butyl piperazine-1-carboxylate (1959 mg, 10.5mmol), Xantphos (405 mg, 0.7mmol), palladium acetate (79 mg, 0.4mmol), cesium carbonate (11.4 g, 35mmol) were added to the reaction bottle, and 20 mL of 1,4-dioxane was added. Stirring was started under nitrogen protection, and the temperature was raised to 100 ° C for 3 hours. After the reaction was completed, 100 mL of water was added to quench the reaction, and ethyl acetate (50 mL×3) was used for extraction. The organic layers were combined, dried with anhydrous sodium sulfate, filtered, concentrated, and purified by silica gel column chromatography (PE: EA = 5: 1) to obtain compound 44b (2.3 g, yield 84%).

MS m/z (ESI): 391.2 $[M+H]^+$.

Step 2: 6-(4-[(tert-butoxy) carbonyl] piperazine-1-yl)-1-benzothiophene-2-carboxylic acid 44c

Compound 44b (1.2 g, 3.1mmol) was added to a reaction bottle, dissolved with 10 mL of methanol and 10 mL of tetrahydrofuran, sodium hydroxide (0.61 g, 15.4mmol) was dissolved with 10 mL of water and added to the reaction bottle, stirred, and reacted at 70°C for 1 hour. After the reaction, the solvent was removed by rotary evaporation, and water was added to dissolve, and the pH was adjusted to 5-6 with hydrochloric acid, filtered, and dried to obtain compound 44c (1.0 g, yield 90%).

MS m/z(ESI):363.1$[M+H]^+$.

Step 3: tert-butyl-4-(2-([1-(1H-indol-3-yl) hexane-2-yl] carbamoyl)-1-benzothiophene-6-yl) piperazine-1-carboxylate 44d

Compound 44c (500 mg, 1.38mmol) and compound 1c (251 mg, 1.16mmol) were dissolved in 5 mL DMF, and 1-methylimidazole (476 mg, 5.8mmol) and N, N, N', N'-tetramethyl chloroformamidine hexafluorophosphate (488 mg, 1.7mmol) were added, and stirring was started at room temperature for 1 hour. After the reaction was completed, 20 mL of water was added to quench the reaction, and ethyl acetate (50 mL×3) was used for extraction. The organic layers were combined, dried with anhydrous sodium sulfate, filtered, concentrated, and purified by silica gel column chromatography (PE: EA=2:1) to obtain compound 44d (560 mg, yield 86%).

MS m/z(ESI):561.3$[M+H]^+$.

Step 4: N-[1-(1H-indol-3-yl) hexane-2-yl]-6-(piperazine-1-yl)-1-benzothiophene-2-carboxamide 44e

Add compound 44d (560 mg, 1.0mmol) to a solution of hydrogen chloride in ethyl acetate (10 mL, 4.0M), start stirring, and react at room temperature for 1 hour. After the reaction is completed, the solvent is removed by rotary evaporation to obtain compound 44e (414 mg, yield 90%).

MS m/z(ESI):461.3$[M+H]^+$.

Step 5: 6-[4-(Cyclopropanesulfonyl) piperazine-1-yl]-N-[1-(1H-indol-3-yl) hexane-2-yl]-benzothiophene-2-carboxamide 44

Compound 44e (200 mg, 0.434mmol) was dissolved in 3 mL of dichloromethane, triethylamine (66 mg, 0.66mmol) was added, and stirred under ice bath. Cyclopropylsulfonyl chloride (64 mg, 0.456mmol) was diluted with 2 mL of dichloromethane and added dropwise to the reaction bottle. After the addition was completed, the reaction was transferred to room temperature for 1 hour. After the reaction was completed, 20 mL of water was added to quench the reaction, and ethyl acetate (50 mL×3) was used for extraction. The organic layers were combined, dried with anhydrous sodium sulfate, filtered, and concentrated to obtain a crude product. The crude product was purified by silica gel column chromatography (PE: EA=1:1) to obtain compound 44 (70 mg, yield 29%).

MS m/z(ESI):565.2[M+H]+.

Example 45 N-[1-(1H-indol-3-yl) hexane-2-yl]-6-(4-methylsulfonylpiperazine-1-yl)-benzothiophene-2-carboxamide

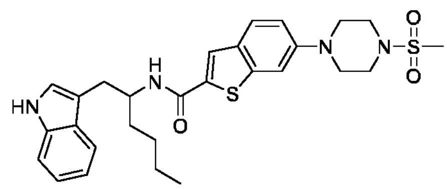

45

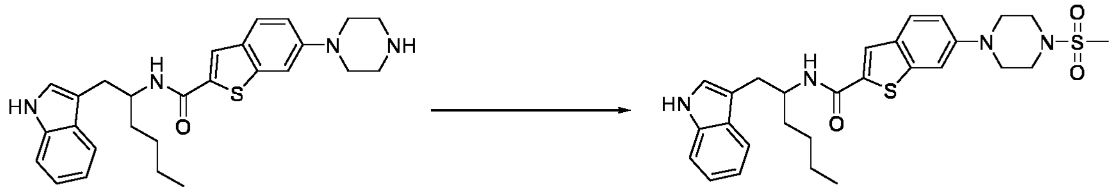

44e

45

Compound 44e (200 mg, 0.434mmol) was dissolved in 3 mL of dichloromethane, trimethylamine (66 mg, 0.651mmol) was added, and the mixture was stirred under ice bath. Methanesulfonyl chloride (49 mg, 0.434mmol) was diluted with 2 mL of dichloromethane and added dropwise to the reaction flask. After the addition was completed, the mixture was transferred to room temperature and reacted for 1 hour. After the reaction, 20 mL of water was added to quench the reaction, and the mixture was extracted with ethyl acetate (50 mL×3). The organic layers were combined, dried with anhydrous sodium sulfate, filtered, and concentrated to obtain a crude product, which was purified and separated by preparative HPLC to obtain compound 45 (30 mg, yield 13%).

$^1$H NMR (400 MHz, Chloroform-d) δ 8.16 (s, 1H), 7.65 (dd, J = 15.3, 8.4 Hz, 2H), 7.43 (s, 1H), 7.38 (d, J = 8.1 Hz, 1H), 7.20 (ddd, J = 8.2, 6.9, 1.2 Hz, 1H), 7.15 - 7.01 (m, 3H), 5.91 (d, J = 8.8 Hz, 1H), 4.47 (tdd, J = 11.1, 7.0, 4.3 Hz, 1H), 3.41 (dd, J= 6.3, 3.1 Hz, 4H), 3.33 (dd, J = 6.3, 3.2 Hz, 4H), 3.15 - 3.01 (m, 2H), 2.83 (s, 3H), 1.74 - 1.23 (m, 7H), 0.88 (t, J = 7.1 Hz, 3H).

MS m/z (ESI):539.2[M+H]$^+$.

Example 46 N-[1-(1H-indol-3-yl) hexane-2-yl]-6-(4-methyl-3-oxopiperazin-1-yl)-benzothiophene-2-carboxamide

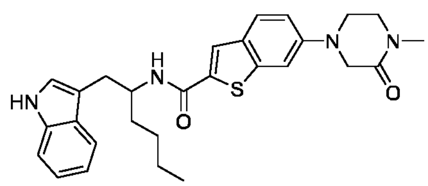

46

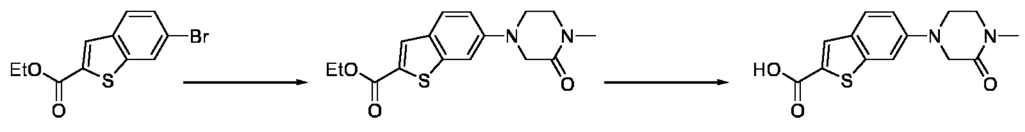

1f 46a 46b

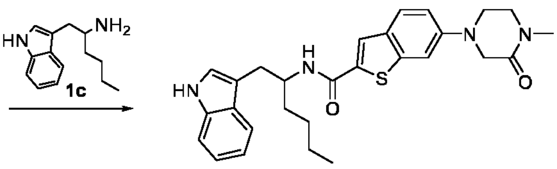

1c

46

Step 1: 6-(4-methyl-3-oxopiperazin-1-yl)-1-benzothiophene-2-carboxylic acid ethyl ester 46a

Compound 1f (500 mg, 1.753mmol), 1-methylpiperazin-2-one (300mg, 2.63mmol), Xantphos (101 mg, 0.175mmol), palladium acetate (20 mg, 0.088mmol) and cesium carbonate (1713 mg, 5.26mmol) were added to the reaction bottle, and 1,4-dioxane (10 mL) was added. Stirring was started under nitrogen protection, and the temperature was raised to 100 ° C for 3 hours. After the reaction was completed, water (50 mL) was added to quench the reaction, and ethyl acetate (50 mL×3) was used for extraction. The organic layers were combined, dried with anhydrous sodium sulfate, filtered, concentrated, and purified by column chromatography (PE:EA= 1:1) to obtain compound 46a (460 mg, yield 83%).
MS m/z(ESI):319.1[M+H]+.

Step 2: 6-(4-methyl-3-carbonylpiperazine-1-yl) benzo[b]thiophene-2-carboxylic acid 46b

Compound 46a (460 mg, 1.445mmol) was added to a reaction bottle, dissolved with 3 mL of methanol and 3 mL of tetrahydrofuran, lithium hydroxide monohydrate (182 mg, 4.335mmol) was dissolved with 3 mL of water and added to the reaction bottle, stirred, and reacted at room temperature for 1 hour. After the reaction was completed, the solvent was removed by rotary evaporation, and water was added to dissolve, and pH was adjusted to 5-6 with 1 M hydrochloric acid, filtered, and the filter cake was dried to obtain compound 46b (420 mg).
MS m/z(ESI):291.0[M+H]+.

a) Step 3: N-[1-(1H-indol-3-yl) hexane-2-yl]-6-(4-methyl-3-oxopiperazine-1-yl)-1-benzothiophene-carboxamide 46

Compound 46b (300 mg, 1.033mmol) and compound 1c (203 mg, 0.939mmol) were dissolved in 3 mL DMF, 1-methylimidazole (386 mg, 4.695mmol) and N, N, N', N'-tetramethyl chloroformamidine hexafluoro phosphate (395 mg, 1.409mmol) were added, and stirring was started at room temperature for 1 hour. After the reaction was completed, 20 mL of water was added to quench the reaction, and ethyl acetate (50 mL×3) was used for extraction. The organic layers were combined, dried with anhydrous sodium sulfate, filtered, concentrated, and purified and separated by preparative HPLC to obtain compound 44 (100 mg, yield 22%).
MS m/z(ESI):489.3$[M+H]^+$.
$^1$H NMR (400 MHz, DMSO-d6) δ 10.77 (d, J = 2.3 Hz, 1H), 8.34 (d, J = 8.5 Hz, 1H), 7.97 (s, 1H), 7.76 (d, J = 8.9 Hz, 1H), 7.63 (d, J = 7.9 Hz, 1H), 7.46 (d, J = 2.3 Hz, 1H), 7.33 (d, J = 8.0 Hz, 1H), 7.21-7.12 (m, 2H), 7.10-7.02 (m, 1H), 7.02-6.94 (m, 1H), 3.88 (s, 2H), 3.60 (dd, J = 6.5, 4.3 Hz, 2H), 3.47 (dd, J = 6.5, 4.3 Hz, 4H), 2.93 (s, 3H), 1.56 (d, J = 12.4 Hz, 1H), 1.41-1.20 (m, 6H), 0.83 (t, J = 6.8 Hz, 3H).

Example 47 N-[1-(1H-indol-3-yl) hexane-2-yl]-6-(4-methyl-2-oxypiperazin-1-yl)-1-**[0584]** benzothiophene-2-carboxamide

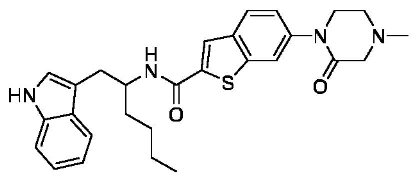

47

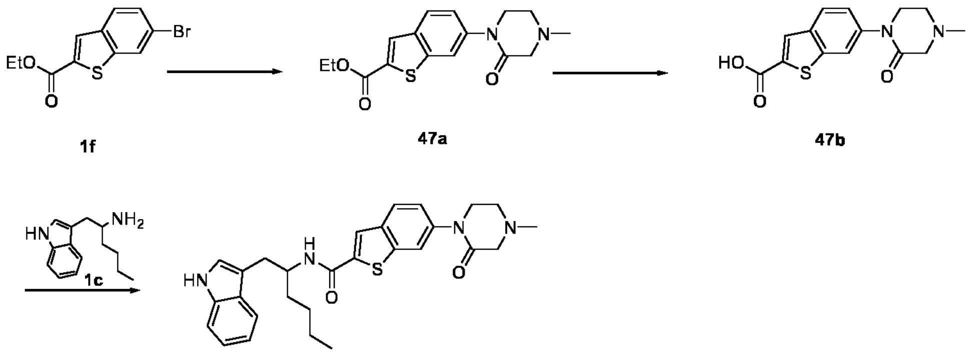

1f 47a 47b

1c

47

Step 1: 6-(4-methyl-2-oxopiperazin-1-yl)-1-benzothiophene-2-carboxylic acid ethyl ester 47a

Compound 1f (300 mg, 1.052mmol), 4-methylpiperazin-2-one (132 mg, 1.157mmol), cuprous iodide (160 mg, 0.842mmol), 1,10-phenanthroline (23 mg, 0.105mmol) and potassium carbonate (695 mg, 2.104mmol) were added to a reaction bottle, and then 2 mL of dimethyl sulfoxide was added, and microwave reaction was performed at a temperature of 130°C for 5 hours. After the reaction was completed, 20 mL of water was added to quench the reaction, and ethyl acetate (50 mL×3) was used for extraction. The organic layers were combined, dried with anhydrous sodium sulfate, filtered, concentrated, and purified by silica gel column chromatography (PE: EA=1:1) to obtain compound 47a (220 mg, yield 66%).

MS m/z(ESI):319.1[M+H]$^+$.

Step 2: 6-(4-methyl-2-oxopiperazine-1-yl)-1-benzothiophene-2-carboxylic acid 47b

Dissolve compound 47a (220 mg, 0.691mmol) in 3 mL of methanol, dissolve sodium hydroxide (83 mg, 2.073mmol) in 3 mL of water and add to the reaction bottle, start stirring, and react at room temperature for 1 hour. After the reaction, remove the solvent by rotary evaporation, add water to dissolve, adjust pH to 5-6 with 1M hydrochloric acid, filter, and dry the filter cake to obtain compound 47b (160 mg, yield 80%).

MS m/z(ESI):291.1[M+H]$^+$.

b) Step 3: N-[1-(1H-indol-3-yl) hexane-2-yl]-6-(4-methyl-2-oxypiperazine-1-yl)-1-benzothiophene-carboxamide 47

Compound 47b (160 mg, 0.551mmol) and compound 1c (119 mg, 0.551mmol) were dissolved in 5 mL DMF, 1-methylimidazole (226 mg, 2.755mmol) and N, N, N', N'-tetramethylchloroformamidine hexafluorophosphate (232 mg, 0.827mmol) were added, and stirring was started at room temperature for 1 hour. After the reaction was completed, 20 mL of water was added to quench the reaction, and ethyl acetate (50 mL×3) was used for extraction. The organic layers were combined, dried with anhydrous sodium sulfate, filtered, concentrated, purified and separated by preparative HPLC, and freeze-dried to obtain compound 47 (60 mg, yield 22%).

MS m/z(ESI):489.2[M+H]$^+$.

$^1$H NMR (400 MHz, Chloroform-d) δ 8.27 (s, 1H), 7.63 (dd, J = 21.1, 8.2 Hz, 2H), 7.36 (dd, J = 7.6, 2.9 Hz, 3H), 7.19 (ddd, J = 15.2, 8.4, 1.6 Hz, 2H), 7.14 - 7.06 (m, 2H), 6.24 (d, J = 9.4 Hz, 1H), 4.51 (d, J = 5.9 Hz, 1H), 3.68 (qt, J = 11.5, 5.3 Hz, 2H), 3.32 (s, 2H), 3.07 (qd, J = 15.0, 5.9 Hz, 2H), 2.81 (t, J = 5.4 Hz, 2H), 2.44 (s, 3H), 1.83 - 1.22 (m, 6H), 0.88 (t, J = 7.1 Hz, 3H).

Example 48 N-(1-(1H-indol-3-yl) hexane-2-yl)-6-(4-hydroxy-4-methylpiperidin-1-yl) benzo[b]-thiophene-2-carboxamide 48

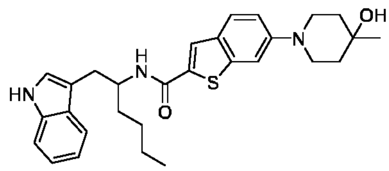

48

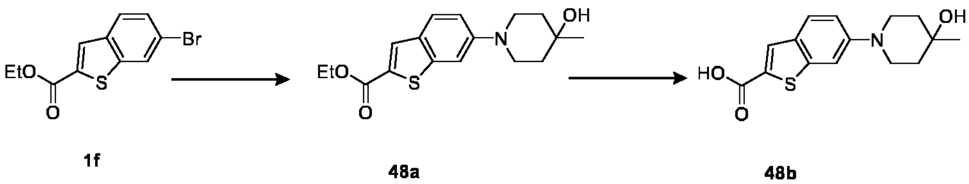

1f 48a 48b

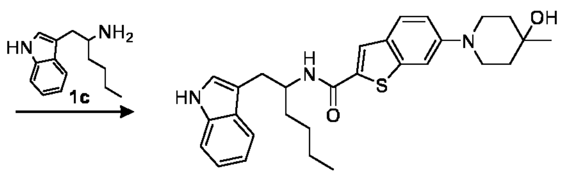


48

Step 1: 6-(4-hydroxy-4-methylpiperidin-1-yl) benzo[b]thiophene-2-carboxylic acid ethyl ester 48a

Compound 1f (1000 mg, 3.5mmol), palladium acetate (40 mg, 0.2mmol), Xantphos (202 mg, 0.35mmol) and cesium carbonate (3.42 g, 10.5mmol) were added to the reaction bottle, 4-methylpiperidin-4-ol (674 mg, 5.3mmol) was dissolved with 10 mL 1,4-dioxane, nitrogen was protected, stirring was started, and the reaction was carried out at 100°C for 3 hours. After the reaction was completed, water (50 mL) was added to quench the reaction, ethyl acetate (50 mL×3) was used for extraction, the organic layers were combined, dried with anhydrous sodium sulfate, concentrated, and purified by silica gel column chromatography (PE:EA = 5:1) to obtain compound 48a (510 mg, yield 46%).

MS m/z(ESI):320.2$[M+H]^+$.

Step 2: 6-(4-Hydroxy-4-methylpiperidin-1-yl) benzo[b]thiophene-2-carboxylic acid 48b

Compound 48a (0.24 g, 0.752mmol) was dissolved in methanol (3 mL) and water (3 mL), and then lithium hydroxide (0.05 g, 2.166mmol) was added at room temperature. Finally, the reaction mixture was stirred at room temperature for 1 hour. TLC showed that the reaction was complete. The reaction mixture was adjusted to pH 3-4 with dilute hydrochloric acid (5 mL, 2.0 M), and a white solid precipitated. The reaction mixture was filtered to collect the white solid to obtain compound 48b (190 mg, yield 87%).

MS m/z (ESI): 292.4 $[M+H]^+$.

Step 3: N-(1-(1H-indol-3-yl) hexane-2-yl)-6-(4-hydroxy-4-methylpiperidin-1-yl) benzo[b]-thiophene-2-carboxamide 48

Compound 1c (160 mg, 0.720mmol) and compound 48b (190 mg, 0.65mmol) were added to a reaction bottle, dissolved with 5 mL DMF, and then 1-methylimidazole (324mg, 3.94mmol) and N, N, N', N'-tetramethylchloroformamidine hexafluorophosphate (332 mg, 1.18mg) were added. Stirring was started at room temperature and the reaction was allowed to proceed for 1 hour. After the reaction was completed, water (20 mL) was added to quench the reaction, and ethyl acetate (50 mL×3) was used for extraction. The organic layers were combined, dried with anhydrous sodium sulfate, and concentrated to obtain a crude product, which was purified and separated by preparative HPLC to obtain compound 48 (35 mg, yield 11%).

MS m/z(ESI):490.3$[M+H]^+$.

$^1H$ NMR (400 MHz, Chloroform-d) δ 8.45 (s, 1H), 7.87 - 7.75 (m, 1H), 7.54 - 7.48 (m, 1H), 7.33 - 7.28 (m, 1H), 7.18 (s, 1H), 6.98 - 6.82 (m, 3H), 5.40 - 5.34 (m, 2H), 4.10 -4.02 (m, 1H), 3.24-3.04 (m, 4H), 2.86-2.61 (m, 2H), 2.25-2.11 (m, 4H), 1.82-1.71(m, 4H), 1.52-1.47(m, 4H),1.20(s, 3H), 0.88(t, 3H).

Example 49 N-(1-(1H-indol-3-yl) hexane-2-yl)-6-(3-hydroxy-3-methylazetidine-1-yl) benzo[b]-thiophene-2-carboxamide 49

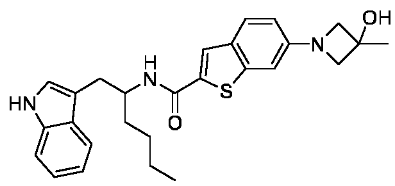

49

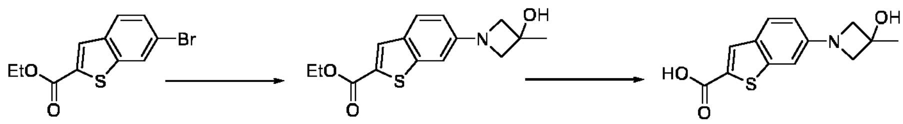

1f 49a 49b

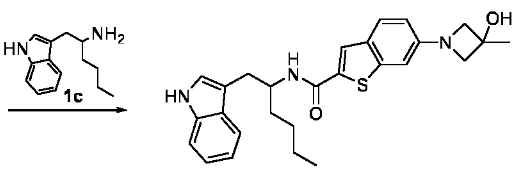

1c

49

Step 1: 6-(3-hydroxy-3-methylazetidine-1-yl) benzo[b]thiophene-2-carboxylic acid ethyl ester 49a

Compound 1f (1000 mg, 3.5mmol), palladium acetate (40 mg, 0.2mmol), Xantphos (202 mg, 0.35mmol) and cesium carbonate (3.42 g, 10.5mmol) were added to the reaction bottle, 3-methylazetidine-3-ol (450 mg, 5.3mmol) was dissolved in 10 mL 1,4-dioxane, nitrogen was protected, stirring was started, and the reaction was carried out at 100 ° C for 3 hours. After the reaction was completed, water (50 mL) was added to quench the reaction, ethyl acetate (50 mL×3) was extracted, the organic layers were combined, dried with anhydrous sodium sulfate, concentrated and purified by silica gel column chromatography (PE:EA=5:1) to obtain compound 49a (650 mg, yield 64%).

MS m/z(ESI):292.2[M+H]$^+$.

Step 2: 6-(3-Hydroxy-3-methylazetidin-1-yl) benzo[b]thiophene-2-carboxylic acid 49b

Compound 49a (0.65 g, 2.23mmol) was dissolved in methanol (5 mL) and water (5 mL), and then lithium hydroxide (0.281 g, 6.67mmol) was added at room temperature. Finally, the reaction mixture was stirred at room temperature for 1 hour. TLC showed that the reaction was complete. The reaction mixture was adjusted to pH=3-4 with dilute hydrochloric acid (5 mL, 2.0 M). A white solid precipitated. The reaction mixture was filtered to collect the white solid to obtain compound 49b (550 mg, yield 94%).

MS m/z(ESI):264.4[M+H]$^+$.

Step 3: N-(1-(1H-indol-3-yl) hexane-2-yl)-6-(3-hydroxy-3-methylazetidin-1-yl) benzo[b]-thiophene-2-carboxamide 49

Compound 1c (160 mg, 0.720mmol) and compound 49b (170 mg, 0.65mmol) were added to a reaction bottle, dissolved with DMF (5mL), and then 1-methylimidazole (324 mg, 3.94mmol) and N, N, N', N'-tetramethylchloroformamidine hexafluorophosphate (332 mg, 1.18 mg) were added. Stirring was started at room temperature and the reaction was allowed to proceed for 1 hour. After the reaction was completed, water (20 mL) was added to quench the reaction,

and ethyl acetate (50 mL×3) was used for extraction. The organic layers were combined, dried with anhydrous sodium sulfate, and concentrated to obtain a crude product, which was purified and separated by preparative HPLC to obtain compound 49 (45 mg, yield 15%).

MS m/z(ESI):462.3[M+H]$^+$.

Example 50 N-(1-(1H-indol-3-yl) hexane-2-yl)-6-(6-hydroxy-6-methyl-2-azaspiro [3.3] heptane-2-yl) benzo[b]thiophene-2-carboxamide 50

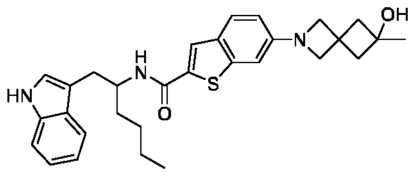

**50**

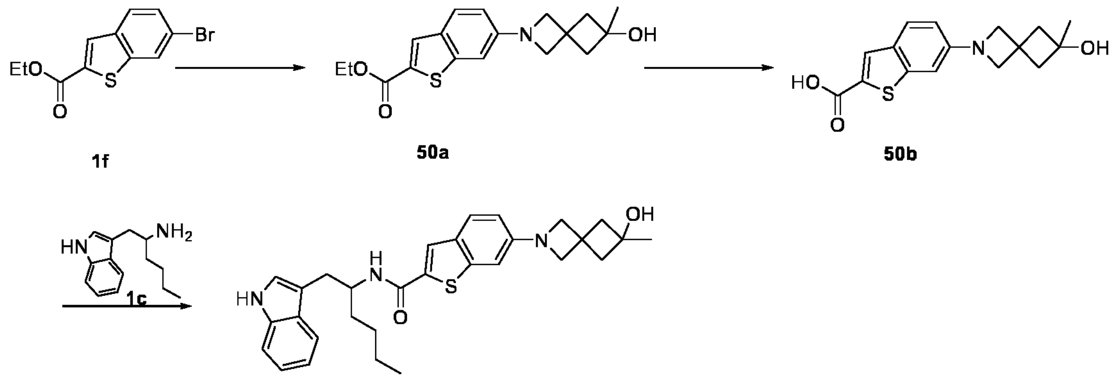

1f

50a

50b

1c

**50**

Step 1: 6-(6-hydroxy-6-methyl-2-azaspiro [3.3] heptane-2-yl) benzo[b]thiophene-2-carboxylic acid ethyl ester 50a

Compound 1f (1000 mg, 3.5mmol), palladium acetate (40 mg, 0.2mmol), Xantphos (202 mg, 0.35mmol) and cesium carbonate (3.42 g, 10.5mmol) were added to a reaction bottle, 6-methyl-2-azaspiro [3.3] heptane-6-ol (580 mg, 5.3mmol) was dissolved in 10 mL of 1,4-dioxane, nitrogen protection was applied, stirring was started, and the reaction was carried out at 100°C for 3 hours. After the reaction, water (50 mL) was added to quench the reaction, and ethyl acetate (50 mL × 3) was used for extraction. The organic layers were combined, dried with anhydrous sodium sulfate, concentrated, and purified by silica gel column chromatography (PE: EA = 5: 1) to obtain compound 50a (720 mg, yield 62%).

MS m/z (ESI): 332.2 [M+H]$^+$.

Step 2: 6-(6-Hydroxy-6-methyl-2-azaspiro [3.3] heptane-2-yl) benzo [b] thiophene-2-carboxylic acid 50b

Compound 50a (0.72 g, 2.10mmol) was dissolved in methanol (5 mL) and water (5 mL), and then lithium hydroxide (0.281 g, 6.60mmol) was added at room temperature. Finally, the reaction mixture was stirred at room temperature for 1 hour. TLC showed that the reaction was complete. The reaction mixture was adjusted to pH 3-4 with dilute hydrochloric acid (5 mL, 2.0 M). A white solid precipitated. The reaction mixture was filtered to collect the white solid to obtain compound 50b (580 mg, yield 88%).

MS m/z (ESI): 304.2 [M+H]$^+$.

Step 3: N-(1-(1H-indol-3-yl) hexane-2-yl)-6-(6-hydroxy-6-methyl-2-azaspiro [3.3] heptane-2-yl) benzo[b]thiophene-2-carboxamide 50

Compound 1c (160 mg, 0.720mmol) and compound 50b (170 mg, 0.56mmol) were added to a reaction bottle, dissolved with 5 mL DMF, and then 1-methylimidazole (324 mg, 3.94mmol) and N, N, N' N'-tetramethylchloroformamidine

hexafluorophosphate (332 mg, 1.18 mg) were added. Stirring was started at room temperature and the reaction was allowed to proceed for 1 h. After the reaction was completed, water (20 mL) was added to quench the reaction, and the mixture was extracted with ethyl acetate (50 mL×3). The organic layers were combined, dried with anhydrous sodium sulfate, and concentrated to obtain a crude product, which was purified and separated by preparative HPLC to obtain compound 50 (55 mg, yield 20%).

MS m/z(ESI):502.3[M+H]$^+$.

Example 51 N-(1-(1H-indol-3-yl) hexane-2-yl)-6-(3-hydroxy-3-methylpyrrolidin-1-yl) benzo[b]-thiophene-2-carboxamide 51

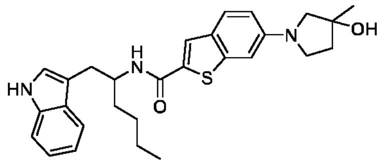

51

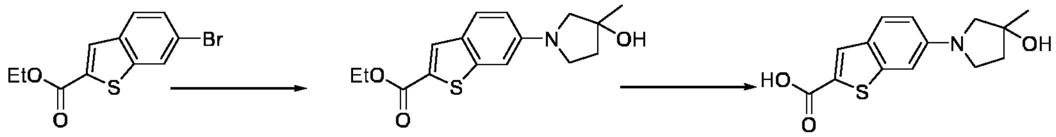

1f 51a 51b

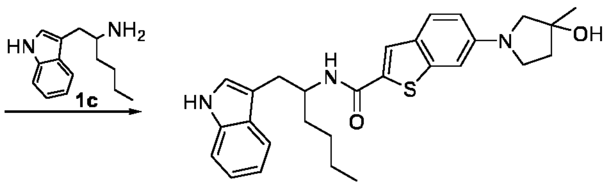


51

Step 1: 6-(6-hydroxy-6-methyl-2-azaspiro [3.3] heptane-2-yl) benzo[b]thiophene-2-carboxylic acid ethyl ester 51a

Compound 1f (1000 mg, 3.5mmol), palladium acetate (40 mg, 0.2mmol), Xantphos (202 mg, 0.35mmol) and cesium carbonate (3.42 g, 10.5mmol) were added to the reaction bottle, 3-methylpyrrolidin-3-ol (510 mg, 5.5mmol) was dissolved in 10 mL 1,4-dioxane, nitrogen was protected, stirring was started, and the reaction was carried out at 100 ° C for 3 hours. After the reaction was completed, water (50 mL) was added to quench the reaction, ethyl acetate (50 mL×3) was used for extraction, the organic layers were combined, dried with anhydrous sodium sulfate, concentrated, and purified by silica gel column chromatography (PE: EA = 5: 1) to obtain compound 51a (810 mg, yield 78%).

MS m/z (ESI): 306.2 [M+H]$^+$.

Step 2: 6-(3-Hydroxy-3-methylpyrrolidin-1-yl) benzo[b]thiophene-2-carboxylic acid 51b

Compound 51a (0.810 g, 2.66mmol) was dissolved in methanol (5 mL) and H2O (5 mL), and then lithium hydroxide (0.340 g, 8. 10mmol) was added at room temperature. Finally, the reaction mixture was stirred at room temperature for 1 hour. TLC showed that the reaction was complete. The reaction mixture was adjusted to pH = 3-4 with dilute hydrochloric acid (5 mL, 2.0 M). A white solid precipitated. The reaction mixture was filtered to collect the white solid to obtain compound 51b (610 mg, yield 83%).

MS m/z(ESI):278.2[M+H]$^+$.

Step 3: N-(1-(1H-indol-3-yl) hexane-2-yl)-6-(6-hydroxy-6-methyl-2-azaspiro [3.3] heptane-2-yl) benzo[b]thiophene-2-carboxamide 51

Compound 1c (160 mg, 0.720mmol) and compound 51b (170 mg, 0.61mmol) were added to a reaction bottle, dissolved with DMF (5 mL), and then 1-methylimidazole (324 mg, 3.94mmol) and N, N, N', N'-tetramethylchloroformamidine hexafluorophosphate (332 mg, 1.18 mg) were added, and stirring was started at room temperature for 1 hour. After the reaction was completed, water (20 mL) was added to quench the reaction, and ethyl acetate (50 mL×3) was used for extraction. The organic layers were combined, dried with anhydrous sodium sulfate, and concentrated to obtain a crude product, which was purified and separated by preparative HPLC to obtain compound 51 (51 mg, yield 17%).
MS m/z(ESI):476.3[M+H]$^+$.
$^1$H NMR (400 MHz, Chloroform-d) δ 8.45 (s, 1H), 7.87 - 7.75 (m, 1H), 7.54 - 7.48 (m, 1H), 7.33 - 7.28 (m, 1H), 7.18 (s, 1H), 6.98 - 6.82 (m, 3H), 5.40 - 5.34 (m, 2H), 4.10 - 4.02 (m, 1H), 3.92-3.72 (m, 1H), 3.11-3.02(m, 4H), 2.86 - 2.61 (m, 2H), 1.80-1.71 (m, 2H), 1.47-1.32(m, 6H), 1.25(s, 3H), 0.88(t, 3H).

Example 52 (S)-N-(1-(1H-indol-3-yl) hexane-2-yl)-6-(4-methylpiperazin-1-yl) thieno[3,2-c] pyridine-2-carboxamide

Example 53 (R)-N-(1-(1H-indol-3-yl) hexane-2-yl)-6-(4-methylpiperazin-1-yl) thieno[3,2-c] pyridine-2-carboxamide

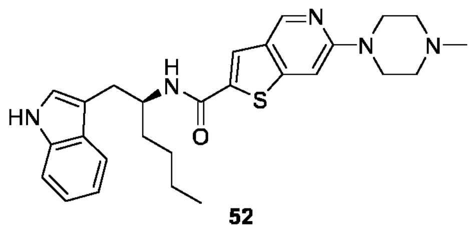

52

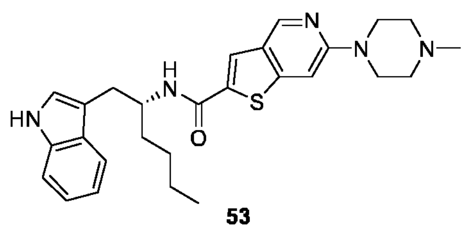

53

The racemic compound 8 (1.6g, 3.36mmol) was chirally separated to give single compounds of compound 52 (0.48g) and compound 53 (0.42g).
Using DAICEL CHIRALPAK®IB (250*40 mm 10 um) on THAR 80 preparative SFC to separate the enantiomers. Compound 8 (1.6 g, 3.36mmol) was dissolved in 80 mL of methanol, and then chiral separation was performed using the SFC system. Mobile phase A used supercritical fluid carbon dioxide, and mobile phase B used methanol. The mobile phase ratio was A: B = 45:55. The mobile phase was kept isocratic during the entire separation process, with a flow rate of 70 mL/min. After the separation was completed, the mixture was dried at 40°C to obtain compound 52 (0.48 g) and compound 53 (0.42 g).
Chiral purity test of compound 52 (OJ, 40%MeOH (0.1% DEA)): Peak 1 RT: 2.527 (0.37%) Peak 2 RT: 4.470 (99.63%).
Chiral purity test of compound 53 (OJ, 40%MeOH (0.1% DEA)): Peak 1 RT: 2.470 (99.13%) Peak 2 RT: 4.522 (0.87%).
Compound 52 MS m/z (ESI): 477.5 [M+H]$^+$.
Compound 53 MS m/z (ESI): 477.5 [M+H]$^+$.

Comparative Example 1

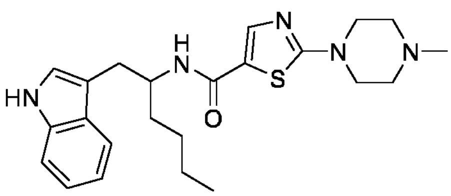

Comparative Compound 1

Comparative Compound 1 was prepared according to the synthesis method of Example 1 of WO2015116663A1.

Biological Example 1 Detection of the ability of compounds to inhibit α-synuclein self-aggregation by HPLC

The test compound (for poorly soluble compounds, dissolve with an appropriate amount of DMSO first) and fresh α-synuclein monomers (purchased from Sino Biological) were added to pure water to a final protein concentration of 250μg/ml, and the concentration of the test compound was 200μg/mL, followed by incubation at 37°C for 9 days. The peak area of α-synuclein multimers after incubation was detected by HPLC and the inhibition rate was calculated. Analysis showed that compared with the model group (without test compound), the peak area of α-synuclein multimers in the test compound group after incubation was significantly reduced. Inhibition rate = (peak area of model group - peak area of example group) / peak area of model group × 100%.

The inhibition rate of the example compounds of the present invention in inhibiting α-synuclein aggregation is shown in Table 1 below.

Table 1

| Example | 1 | 2 | 3 | 4 | 5 | 6 | 7 | 8 | 9 | 10 |
|---|---|---|---|---|---|---|---|---|---|---|
| inhibition rate (%) | 98.3 | 86.8 | 95.3 | 90.6 | 98.6 | 97.8 | 71.5 | 73.7 | 78.1 | 73.1 |
| Example | 11 | 12 | 13 | 14 | 15 | 16 | 17 | 18 | 19 | 20 |
| inhibition rate (%) | 73.4 | 86.9 | 94.4 | 73.5 | 96.2 | 96.3 | 98.2 | 76.9 | 93.5 | 95.6 |
| Example | 21 | 22 | 23 | 24 | 25 | 26 | 27 | 28 | 29 | 30 |
| inhibition rate (%) | 78.2 | 83.8 | 89.7 | 89.7 | 90.8 | 93.3 | 87.6 | 73.0 | 96.7 | 71.4 |
| Example | 31 | 32 | 33 | 34 | 35 | 36 | 37 | 38 | 39 | 40 |
| inhibition rate (%) | 95.3 | 86.1 | 93.4 | 97.5 | 85.4 | 85.3 | 86.7 | 78.3 | 92.6 | 76.8 |
| Example | 41 | 42 | 43 | 44 | 45 | 46 | 47 | 48 | 49 | 50 |
| inhibition rate (%) | 82.5 | 97.2 | 98.6 | 89.7 | 82.3 | 78.9 | 73.5 | 96.5 | 92.9 | 95.7 |
| Example | 51 | Comparative Example 1 | | | | | | | | |
| inhibition rate (%) | 90.3 | 63.7 | | | | | | | | |

Table 1 shows that the compounds of the present invention can significantly inhibit the self-aggregation of α-synuclein.

Biological Example 2 Immunofluorescence detection of the ability of compounds to inhibit the self-aggregation of α-synuclein (cell experiment)

Construct a lentiviral expression plasmid expressing an α-synuclein mutant, package the virus to infect SH-SY5Y cells, screen stable cell lines, and use immunofluorescence to detect the effect of the compound on α-synuclein aggregates. At the same time, an empty vector was infected as a control. An α-synuclein phosphorylation antibody was used to detect the aggregation of the protein in cells. By immunoblotting, cells infected with virus-expressing α-synuclein showed high expression levels of α-synuclein monomers (14 kd) and oligomers (i.e., dimers, trimers, and tetramers) in soluble and insoluble fractions compared with the model group (without test compound). After treatment with the test compound, the effect of the test compound on α-synuclein polymers was detected. The higher the fluorescence intensity, the stronger the protein aggregation ability. Treatment with a control inactive compound did not affect the expression level of α-synuclein.

The experimental results show that the compounds of the present invention can significantly inhibit the self-aggregation of α-synuclein in cells.

Biological Example 3 The effect of compounds on the survival rate of transiently transfected cells was detected by the MTT method

The MTT (3-(4,5-dimethylthiazole-2)-2,5-diphenyltetrazolium bromide) method, also known as the MTT colorimetric method, is a method for detecting cell survival and growth. The detection principle is that the succinate dehydrogenase in the mitochondria of living cells can reduce exogenous MTT to water-insoluble blue-purple crystalline formazan and deposit it in the cells, while dead cells do not have this function. Dimethyl sulfoxide (DMSO) can dissolve the formazan in the cells, and its absorbance value (OD value) is measured at a wavelength of 490nm using an enzyme-linked immunosorbent assay, which can indirectly reflect the number of living cells. The larger the OD value, the higher the cell survival rate.

In order to detect the effect of the test compound on the cell survival rate, the MTT method was used to detect the effect of the compound on the survival rate of transient cells. The method is as follows: collect logarithmic phase cells, adjust the concentration of the cell suspension, add 100μL to each well, and plate the cells to be tested to adjust the density to 10,000 wells (the edge wells are filled with sterile phosphate buffer). Incubate at 5% CO2 and 37°C until the cell monolayer covers the bottom of the well (96-well flat-bottom plate). After the cells adhere to the wall, the test compound can be added. Three replicate wells are set for each test compound. Incubate in a cell culture incubator for 48 hours and observe under an inverted microscope. Add 20μL of MTT solution (5 mg/mL, i.e. 0.5% MTT) to each well and continue culturing for 4 hours. If the drug can react with MTT, centrifuge first and then discard the culture medium. Carefully rinse with phosphate buffer three times, and then add the culture medium containing MTT. Terminate the culture and carefully aspirate the culture medium in the wells. Add 150μL of dimethyl sulfoxide to each well and oscillate at a low speed on a shaker for 10 minutes to fully dissolve the crystals. Measure the absorbance of each well at a wavelength of 490 nm using an enzyme-linked immunosorbent assay. The normal group is cells that have not been treated in any way, and the model group is cells that have been transferred with α-synuclein particles.

The experimental results show that the compounds of the present invention can increase the survival rate of cells.

Biological Example 4 Determination of half-life in C57BL/6 mice

C57BL/6 mice (male) were selected, and the compound of the present invention was intravenously administered at a dose of 2mpk. About 0.03 mL of whole blood was collected from the mouse orbital venous plexus at 0 h before administration, 0.0833, 0.25, 0.5, 1, 2, 4, 6, 8 and 24 h after administration. The blood was placed in a centrifuge tube containing EDTA-K2 anticoagulant, and placed in crushed ice after collection. Centrifuge at 2000g and 4°C for 10 min within 0.5 hours, and 10μL of plasma was quantitatively divided and placed in another clean centrifuge tube, placed in a -20°C refrigerator, and the blood drug concentration was determined by LC-MS/MS method. The half-life $t_{1/2}$ was calculated as shown in Table 2 below.

Table 2

| Example | $t_{1/2}$(h) |
|---|---|
| 1 | 2.21 |
| 3 | 1.46 |
| 42 | 1.17 |
| 43 | 1.32 |
| Comparative Example 1 | 0.39 |

The experimental results show that the half-life of the compound of the present invention is relatively long in vivo, which can effectively prolong the action time of the drug in vivo.

Biological Example 5 Determination of brain/plasma ratio

Experimental purpose

Male C57BL/6 mice were used as research subjects, and brain perfusion technology was used to investigate the pharmacokinetic characteristics of the compound of the present invention transported through the blood-brain barrier, and the drug concentrations in the blood and brain tissue samples of mice after 0.25h, 1h, 4h and 8h were detected, and the ratio of the average drug concentrations in brain tissue and plasma was calculated.

II. Materials and methods

Drug delivery system

Oral adinistration solution (1.000mg·mL$^{-1}$) of the compound of the present invention was prepared: Weigh 3.116mg of the compound and place it in an EP tube, then add DMSO 0.156mL, Solutol 0.312mL and ultrapure water 2.648mL (the volume ratio of the three is 5:10:85, v: v: v), vortex ultrasound to fully dissolve it, and the final actual concentration is 1.000mg·mL-1 of the clear administration solution.

$$3.116\text{mg}\times 100\% \div 3.116\text{mL} = 1.000\text{mg}\cdot\text{mL}^{-1}$$

2. Experimental animals

C57BL/6 mice (male), purchased from Sibeifu (Beijing) Biotechnology Co., Ltd., experimental animal production license number: SCXK (Beijing) 2019-0010.

Fasting: Overnight fasting, no water withdrawal, feed provided 4 hours after medication.

3. Animal test plan

Select qualified healthy C57BL/6 mice, 3 per group. After oral administration (10 mg/kg), about 150μL of whole blood was collected from the orbital venous plexus of mice at 0.25h, 1h, 4h and 8h after medication and placed in a centrifuge tube containing EDTA-K2 anticoagulant. After administration, the mice were anesthetized about 1 minute before the scheduled time point, and brain perfusion was performed at 0.25h, 1h, 4h and 8h after administration. After perfusion, the mouse brain tissue samples were dissected with ophthalmic scissors and ophthalmic forceps, and the remaining blood on the tissue surface was immediately rinsed with ultrapure water within 5min, wiped dry, weighed, and 4 times the volume of acetonitrile-water solution (10:90, v: v) was added and homogenized, and then the homogenate was placed in a -20°C refrigerator for testing. The collected samples were placed in an ice bath during the entire experimental operation.

4. Whole blood sample collection

According to the time points specified in the above experimental protocol, about 150μL of whole blood was collected from the orbital venous plexus of C57BL/6 mice after administration and placed in a 1.5mL centrifuge tube containing EDTA-K2. The collected whole blood was centrifuged at 2000g, 4°C for 10min, and all plasma was divided and placed in another clean centrifuge tube, and then placed in a -20°C refrigerator for testing.

5. Brain perfusion method

Adjust the peristaltic pump device to ensure that it works normally, the liquid flows out smoothly and stably, and there is no air in the flow path. Anesthetize the mice after administration with a mixed anesthetic of 70% $CO_2$ and 30% $O_2$. After the animal is fully anesthetized, quickly open the chest cavity with surgical scissors to expose the heart. Clamp the superior vena cava and inferior vena cava with hemostatic forceps, pierce the ventricle with a needle, and cut a small hole in the atrium. Turn on the peristaltic pump switch and perfuse for 2-5 minutes. The perfusion fluid is 100U/1000mL heparin sodium saline. After the perfusion is completed, use ophthalmic scissors and ophthalmic forceps to dissect the mouse brain tissue sample, immediately rinse the remaining blood on the tissue surface with ultrapure water within 5 minutes, wipe dry, weigh, add 4 times the volume of acetonitrile-water solution (10:90, v: v) and homogenize, and then place the homogenate in a -20°C refrigerator for testing.

Calculate the ratio of the average drug concentration in brain tissue to that in plasma, i.e., the brain/plasma ratio. The brain/plasma ratio at 4h is shown in Table 3 below:

Table 3

| Example | Brain/plasma ratio |
|---|---|
| 1 | 1.53 |
| 3 | 0.38 |
| 42 | 2.05 |

(continued)

| Example | Brain/plasma ratio |
|---|---|
| 43 | 1.70 |
| Comparative Example 1 | 0.35 |

The experimental results show that the compounds of the present invention can easily penetrate the blood-brain barrier and enter the brain to exert their pharmacological effects.

Biological Example 6 Behavioral and histological detection of MPTP-induced mouse PD model

Experimental purpose

The animal model of Parkinson's disease (PD) was induced by intraperitoneal injection of MPTP into C57BL/6J mice for 20 consecutive days to produce reliable and reproducible damage to the dopaminergic pathways in the substantia nigra and striatum, and the pharmacodynamic evaluation of the test compounds (including the compounds of the present invention and control compound 1) on the treatment of PD was carried out from the perspectives of behavior (rotarod, grip strength test) and histology (TH immunofluorescence staining).

II. Materials and Methods

Experimental animals

C57BL/6J mice, 8 weeks old, male, 10 mice in each group.

2. Preparation of drug administration

Solvent stock solution: DMSO, Solutol®HS 15, ultrapure water, the volume ratio of the three is 5:10:85.
Test compound: prepare concentrations of 0.008, 0.024, 0.08, 0.4 mg/mL, weigh 0.0004, 0.0012, 0.0040, 0.0200g respectively into EP tubes, add DMSO 2.5mL, Solutol®HS 15, ultrapure water 42.5 mL, vortex to dissolve.

3. Dosage method

MPTP, Day 1-2 (10 mg/kg), Day 3-4 (15 mg/kg), Day 5-6 (20 mg/kg), Day 7-20 (25 mg/kg), intraperitoneal administration, once a day;
Test compound, Day-7-20 (0.1, 0.3, 1, 5 mg/kg), oral administration, once a day.

III. Behavioral test

Rotarod test

Adaptation to the environment: Place the animal in the test room to adapt to the environment for 30-60 minutes before the test;

Adaptation training: Place each experimental animal on the rotarod fatigue instrument for 5 minutes of adaptive training;
Formal test: The parameters of the rotarod fatigue instrument are set to the speed: 20 rpm/min, the test time: 5 minutes, and the mice are placed on the rotarod in batches for testing. After each round, be sure to remove the feces and urine, and wipe them dry with 75% alcohol;
Result analysis: Count the time each animal spends on the rod.

2. Grip Test

Place the mouse on the platform, with both forelimbs on the gripping rod.
Grab the mouse's tail and pull it backward in a straight line. When animals move backward involuntarily, they will instinctively grab any object to prevent retreat until the pulling force exceeds their grip.

After the animal loses its grip, the preamplifier automatically records the maximum pulling force and displays it on the LCD screen. The amplifier can provide digital or analog output.

After the measurement, the average of the maximum pulling force of each animal is calculated.

IV Immunohistochemistry

After the behavioral study, 3 animals in each group were perfused for heart perfusion; collection sites: striatum (Str), substantia nigra (SN).

Perfusion-fixation-sugar precipitation-sectioning

The mice were anesthetized with isoflurane.

Use straight scissors to cut the abdominal and thoracic cavities, expose the heart, first perfuse with saline to flush out the blood, and then perfuse with paraformaldehyde for initial fixation.

Cut off the head, carefully open the skull with forceps, remove the brain tissue and fix it in paraformaldehyde solution for 24 hours.

Take out and place in 20% sucrose solution for 24h on the second day.

Take out and place in 30% sucrose solution for 24h on the third day.

Take out and place in 35% sucrose solution for 24h on the fourth day.

Take out the brain tissue, embed it with OCT embedding medium, and cut 16μm brain slices with a freezing slicer.

2. Immunofluorescence staining (TH)

Rewarm the slices for 30min;
Block: 10% serum + 0.3% TritonX-100 [about 50μl per slice], room temperature for 1h;
Shake the slices, add primary antibody (diluted with PBS), 4°C overnight;
Rewarm for 30min;
Wash the primary antibody (PBS), 5min×3 times;
Add secondary antibody (diluted with PBS) in backlight, room temperature for 2h;
Wash the secondary antibody (PBS), 5min×3 times;
Add DAPI mounting medium.

The experimental results show that the compounds of the present invention have a lower effective concentration.

**Claims**

**1.** A compound of formula (I) or a pharmaceutically acceptable salt thereof,

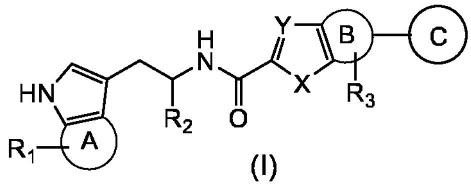

(I)

Wherein,

X is selected from O, S and $NR_a$;
Y is selected from $CR_b$ and N;
Ring A is selected from benzene ring and pyridine ring;
Ring B is selected from benzene ring, pyridine ring, pyridazine ring, pyrimidine ring and pyrazine ring;
Ring C is selected from

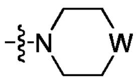, 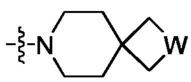, 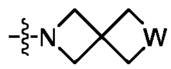, 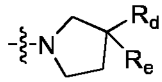,

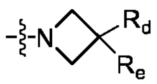, 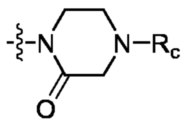 and 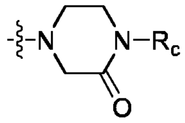;

W is selected from O, $NR_c$ and $CR_dR_e$;
$R_1$ is absent or is selected from fluoro, chloro, bromo, iodo and $C_{1-6}$ alkyl;
$R_2$ is selected from $C_{1-6}$ alkyl, $C_{2-6}$ alkenyl, -$C_{1-4}$ alkylene-O-$C_{1-4}$ alkyl, and -$C_{1-4}$ alkylene-$NR_aR_b$;
$R_3$ is absent or is selected from fluoro, chloro, bromo, iodo, cyano and $C_{1-6}$ alkyl;
$R_a$ and $R_b$ are independently selected from H and $C_{1-6}$ alkyl;
$R_c$ is selected from H, cyano, $C_{1-6}$ alkyl, cyclopropyl, cyclobutyl, cyclopentyl, cyclohexyl, phenyl, -$SO_2R_f$, and 1-$C_{1-6}$ alkyl-4-piperidinyl;
$R_d$ and $R_e$ are independently selected from H, fluoro, chloro, bromo, iodo, hydroxyl, cyano, -$NR_aR_b$, $C_{1-6}$ alkyl, cyclopropyl, cyclobutyl, cyclopentyl, cyclohexyl, phenyl, and 1-$C_{1-6}$ alkyl-4-piperidinyl;
$R_f$ is selected from methyl, ethyl, n-propyl, isopropyl, cyclopropyl, cyclobutyl, cyclopentyl, and cyclohexyl.

2. The compound or pharmaceutically acceptable salt thereof of claim 1, **characterized in that**

X is O, Y is $CR_b$; or
X is S, Y is $CR_b$; or
X is $NR_a$, Y is $CR_b$; or
X is S, YisN.

3. The compound or pharmaceutically acceptable salt thereof of claim 1, **characterized in that** the compound is the compound of formula (II),

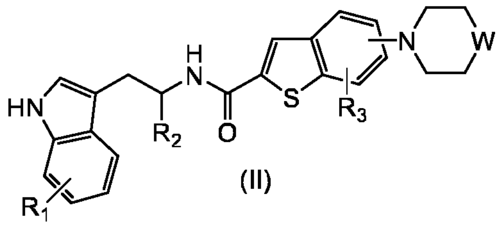

(II)

.

4. The compound or pharmaceutically acceptable salt thereof of claim 1, **characterized in that** the ring C is selected from

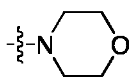, 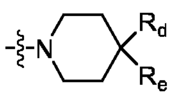, 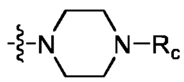, 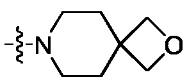,

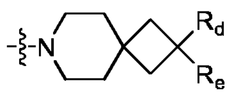, 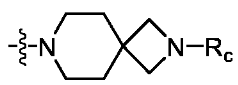, 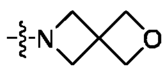, 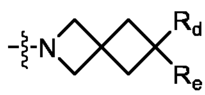, 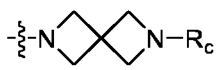,

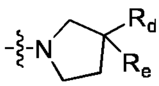, 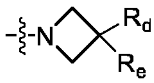, 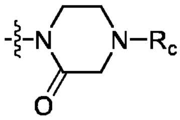 and 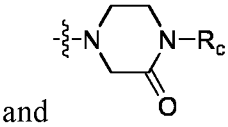.

5. The compound or pharmaceutically acceptable salt thereof according to any one of claims 1 to 4, **characterized in that**
$R_1$ is absent or is selected from fluoro, chloro, methyl, ethyl, n-propyl and isopropyl.

6. The compound or pharmaceutically acceptable salt thereof according to any one of claims 1 to 4, **characterized in that**
$R_2$ is selected from $C_{4-6}$ alkyl, $C_{4-6}$ alkenyl, $-C_{1-3}$ alkylene-O-$C_{1-3}$ alkyl and $-C_{1-3}$ alkylene-$NR_aR_b$.

7. The compound or pharmaceutically acceptable salt thereof according to any one of claims 1 to 4, **characterized in that**
$R_2$ is selected from n-butyl, isopentyl, $-CH_2CH_2OCH_3$, $-CH_2OCH_2CH_3$, - $CH_2CH_2OCH_2CH_3$, $-CH_2CH_2N(CH_3)_2$, $-CH_2CHCHCH_3$, $-CHCHCH_2CH_3$ and - $CH_2CHC(CH_3)_2$.

8. The compound or pharmaceutically acceptable salt thereof according to any one of claims 1 to 4, **characterized in that**
$R_3$ is absent or is selected from fluoro, chloro, cyano, methyl, ethyl, n-propyl and isopropyl.

9. The compound or pharmaceutically acceptable salt thereof according to any one of claims 1 to 4, **characterized in that**
$R_c$ is selected from H, cyano, methyl, ethyl, n-propyl, isopropyl, cyclopropyl, phenyl, - $SO_2R_f$ and 1-$C_{1-3}$ alkyl-4-piperidinyl.

10. The compound or pharmaceutically acceptable salt thereof according to any one of claims 1 to 4, **characterized in that**
$R_d$ and $R_e$ are independently selected from H, fluoro, chloro, hydroxyl, cyano, $-NR_aR_b$, methyl, ethyl, n-propyl, isopropyl, cyclopropyl, phenyl and 1-$C_{1-3}$ alkyl-4-piperidinyl.

11. The compound or pharmaceutically acceptable salt thereof according to any one of claims 1 to 10, **characterized in that**
$R_f$ is selected from methyl, ethyl, n-propyl, isopropyl and cyclopropyl.

12. The compound or pharmaceutically acceptable salt thereof according to any one of claims 1 to 11, **characterized in that**
$R_a$ and $R_b$ are independently selected from H, methyl, ethyl, n-propyl and isopropyl.

13. The compound or pharmaceutically acceptable salt thereof of claim 3, **characterized in that**

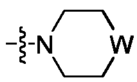

is selected from

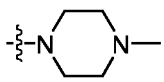, 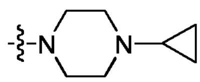, 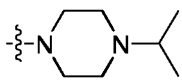,

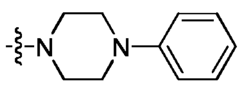, 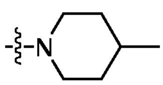, 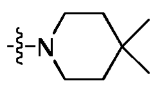, 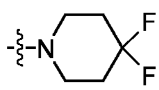, 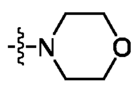, 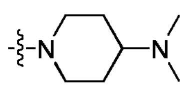,

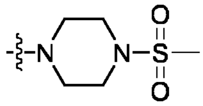, 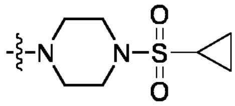, 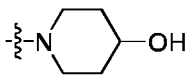 and 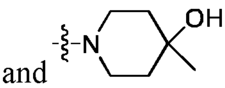.

**14.** The compound or pharmaceutically acceptable salt thereof of claim 3, **characterized in that** $R_1$ is absent or is selected from fluoro and methyl.

**15.** The compound or pharmaceutically acceptable salt thereof of claim 3, **characterized in that** $R_2$ is selected from n-butyl, $-CH_2CH_2OCH_3$ and $-CH_2CHC(CH_3)_2$.

**16.** The compound or pharmaceutically acceptable salt thereof of claim 3, **characterized in that** $R_3$ is absent or is selected from fluoro and methyl.

**17.** The compound or pharmaceutically acceptable salt thereof of claim 1, **characterized in that** the compound is selected from:

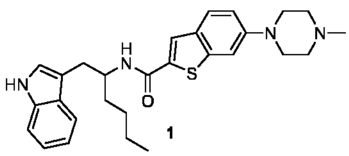
1

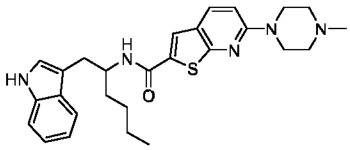
2

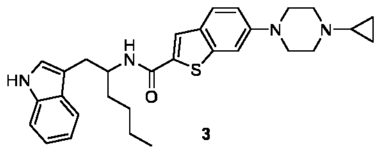
3

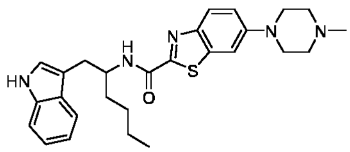
4

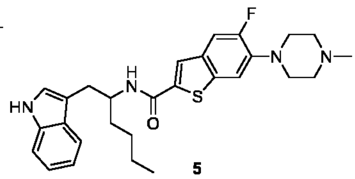
5

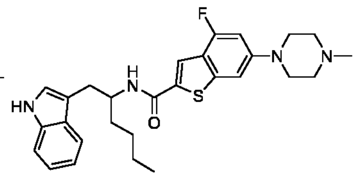
6

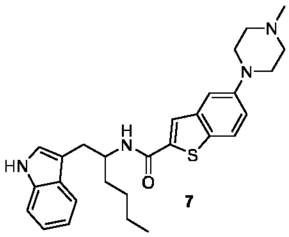
7

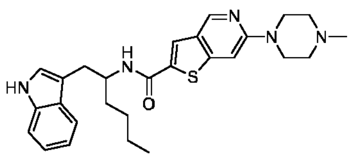
8

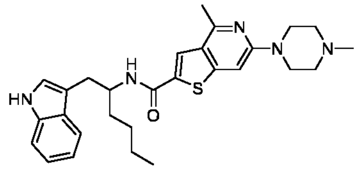
9

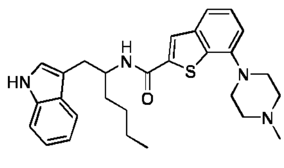
10

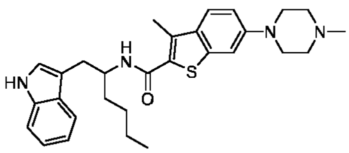
11

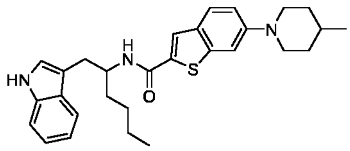
12

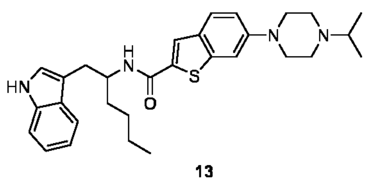
13

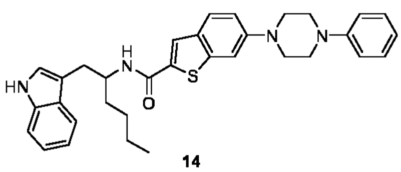
14

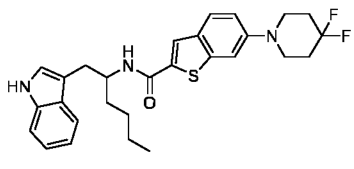
15

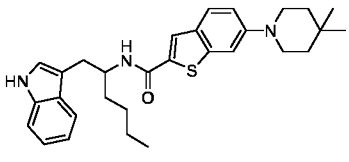
16

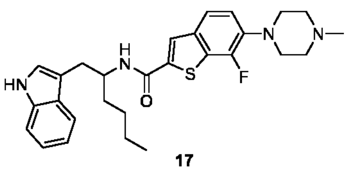
17

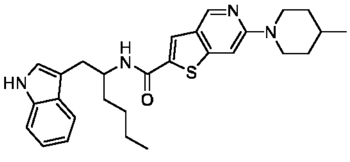
18

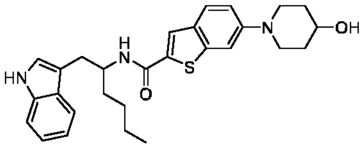
19

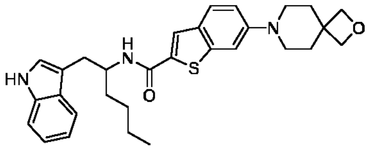
20

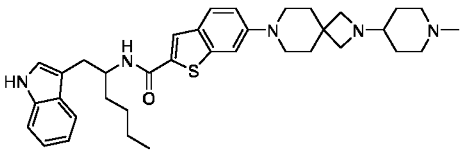
21

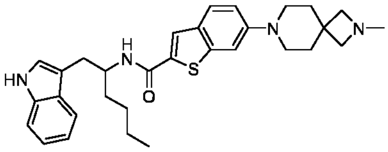
22

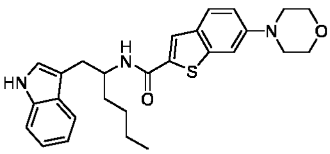
23

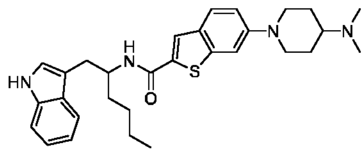
24

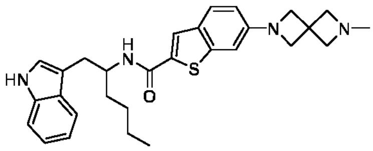
25

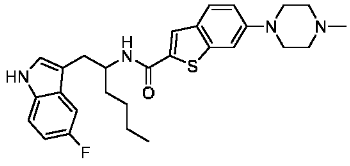
26

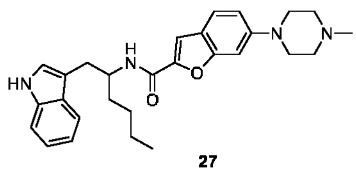
27

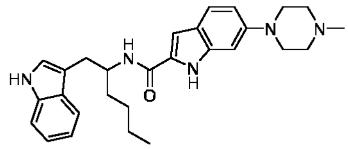
28

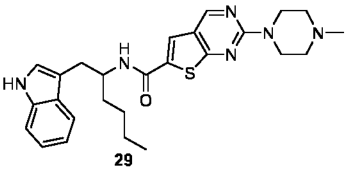
29

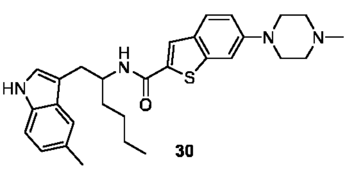
30

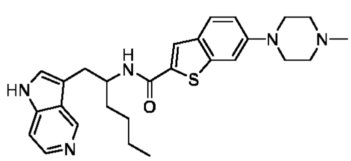
31

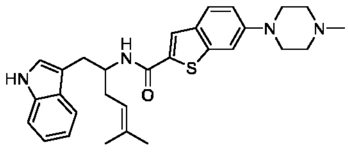
32

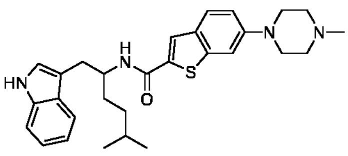
33

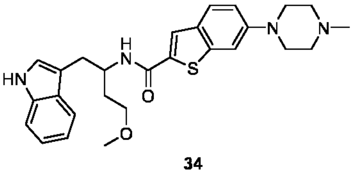
34

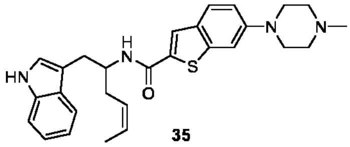
35

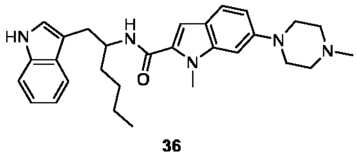
36

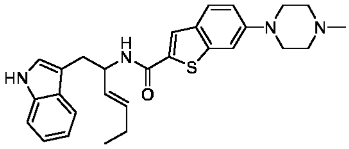
37
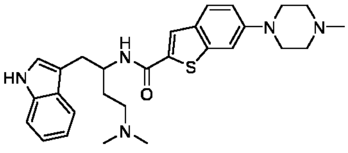
38
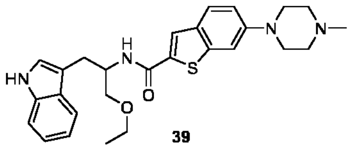
39

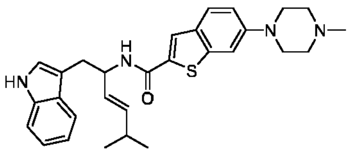
40
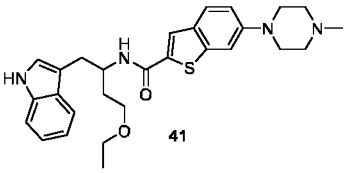
41
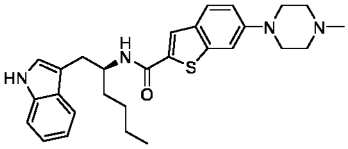
42

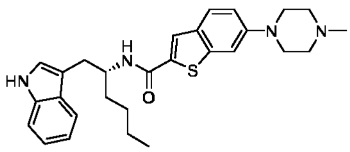
43
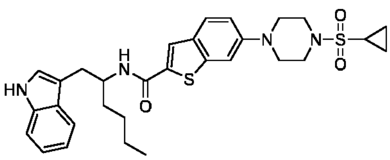
44
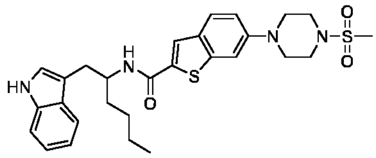
45

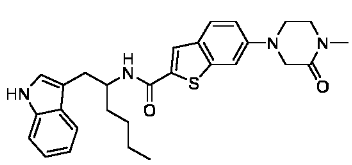
46
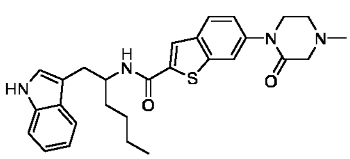
47
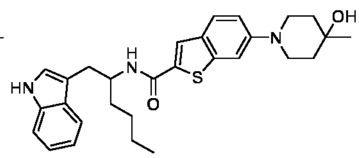
48

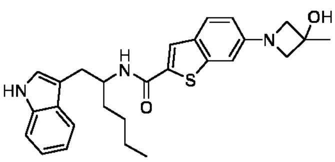
49
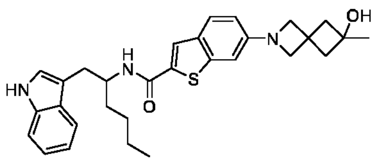
50
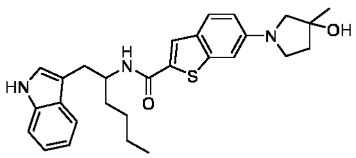
51

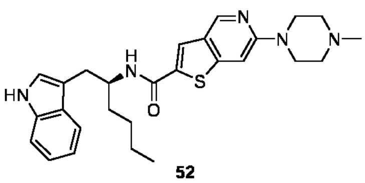
52
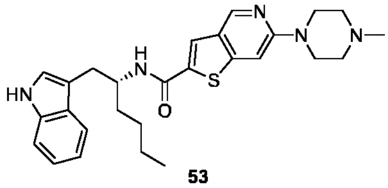
53

**18.** A pharmaceutical composition comprising the compound or pharmaceutically acceptable salt thereof according to any one of claims 1 to 17, optionally comprising one or more pharmaceutically acceptable excipients.

**19.** A method for treating a neurodegenerative disease associated with protein aggregation, the method comprising administering to a subject in need thereof a therapeutically effective amount of at least one compound or a pharmaceutically acceptable salt thereof according to any one of claims 1 to 17.

**20.** The method of claim 19, **characterized in that**
the neurodegenerative disease comprises Alzheimer's disease, Parkinson's disease, frontotemporal dementia, Lewy body disease, Parkinson's disease dementia, multiple system atrophy, amyotrophic lateral sclerosis and Huntington's disease.

## INTERNATIONAL SEARCH REPORT

International application No.
**PCT/CN2022/141820**

**A. CLASSIFICATION OF SUBJECT MATTER**

C07D409/14(2006.01)i;C07D209/04(2006.01)i;C07D487/02(2006.01)i;C07D495/02(2006.01)i;C07D247/00(2006.01)i; C07D241/04(2006.01)i;A61K31/4045(2006.01)i;A61K31/496(2006.01)i;A61P25/28(2006.01)i;A61P25/16(2006.01)i

According to International Patent Classification (IPC) or to both national classification and IPC

**B. FIELDS SEARCHED**

Minimum documentation searched (classification system followed by classification symbols)

C07D; A61K; A61P

Documentation searched other than minimum documentation to the extent that such documents are included in the fields searched

Electronic data base consulted during the international search (name of data base and, where practicable, search terms used)

WPI, CAPLUS(STN), REGISTRY(STN), CNPAT, CNKI: 上海京新生物医药有限公司, 浙江京新药业股份有限公司, 蒋青云, 房鑫, 李平, 李宁宁, 谢志翰, 吴银辉, 马宁田, 侯建, 蛋白质聚集, 神经变性, 帕金森, 阿尔茨海默, 路易体病, 额颞痴呆, 亨廷顿病, 肌萎缩侧索硬化, 多系统萎缩症, protein aggregate?, neurodegenerat?, Parkinson?, Alzheimer?, Lewy body, fronto temporal dementia, Huntington, amyotrophic lateral sclerosis, multiple system atrophy, 结构式检索, structural formula search

**C. DOCUMENTS CONSIDERED TO BE RELEVANT**

| Category* | Citation of document, with indication, where appropriate, of the relevant passages | Relevant to claim No. |
|---|---|---|
| A | CN 106132960 A (NEUROPORE THERAPIES INC.) 16 November 2016 (2016-11-16) claims 1-4, and description embodiments 1-28 | 1-20 |
| A | CN 102725284 A (NEUROPORE THERAPIES INC.) 10 October 2012 (2012-10-10) claims 1-16 | 1-20 |
| A | CN 110167937 A (UCB BIOPHARMA SPRL) 23 August 2019 (2019-08-23) claims 1-15 | 1-20 |
| A | CN 110198938 A (UCB BIOPHARMA SPRL) 03 September 2019 (2019-09-03) claims 1-14 | 1-20 |
| A | CN 110191885 A (UCB BIOPHARMA SPRL) 30 August 2019 (2019-08-30) claims 1-16 | 1-20 |
| A | WO 2017020010 A1 (NEUROPORE THERAPIES, INC.) 02 February 2017 (2017-02-02) claims 1-57 | 1-20 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **09 March 2023** | **16 March 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | |
| Facsimile No. **(86-10)62019451** | Telephone No. |

Form PCT/ISA/210 (second sheet) (July 2022)

**INTERNATIONAL SEARCH REPORT**

International application No.
**PCT/CN2022/141820**

**C. DOCUMENTS CONSIDERED TO BE RELEVANT**

| Category* | Citation of document, with indication, where appropriate, of the relevant passages | Relevant to claim No. |
|---|---|---|
| A | WO 2021055591 A1 (BIAL-BIOTECH INVESTMENTS, INC.) 25 March 2021 (2021-03-25) claims 1-105, 111-116 | 1-20 |
| A | WO 2010012399 A1 (BAYER SCHERING PHARMA AKTIENGESELLSCHAFT et al.) 04 February 2010 (2010-02-04) claims 1-7, 17-19 | 1-20 |

Form PCT/ISA/210 (second sheet) (July 2022)

**INTERNATIONAL SEARCH REPORT**

International application No.
**PCT/CN2022/141820**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **19-20**
because they relate to subject matter not required to be searched by this Authority, namely:

Claims 19-20 relate to a method for treating a disease. The present report is provided on the basis of the use of a compound in the preparation of a drug for the treatment of the disease.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2022/141820**

| Patent document cited in search report | Publication date (day/month/year) | Patent family member(s) | Publication date (day/month/year) |
|---|---|---|---|
| CN 106132960 A | 16 November 2016 | ECSP 16070327 A | 31 May 2018 |
| | | HRP 20201107 T1 | 30 October 2020 |
| | | HRP 20201107 T8 | 11 December 2020 |
| | | CR 20160394 A | 01 November 2016 |
| | | BR 112016017344 A2 | 03 October 2017 |
| | | SI 3099684 T1 | 31 August 2018 |
| | | SA 516371579 B1 | 16 July 2019 |
| | | WO 2015116663 A1 | 06 August 2015 |
| | | WO 2015116663 A8 | 15 September 2016 |
| | | HRP 20180813 T1 | 27 July 2018 |
| | | LT 3348556 T | 10 August 2020 |
| | | EP 3099684 A1 | 07 December 2016 |
| | | EP 3099684 B1 | 04 April 2018 |
| | | EP 3099684 B8 | 04 July 2018 |
| | | AP 2016009347 A0 | 31 July 2016 |
| | | KR 20160113287 A | 28 September 2016 |
| | | KR 102383038 B1 | 05 April 2022 |
| | | EA 201691529 A1 | 30 January 2017 |
| | | EA 032374 B1 | 31 May 2019 |
| | | HK 1231470 A1 | 22 December 2017 |
| | | IL 246987 A0 | 29 September 2016 |
| | | IL 246987 B | 01 December 2021 |
| | | MX 2016009896 A | 11 January 2017 |
| | | PE 20161393 A1 | 08 January 2017 |
| | | LT 3099684 T | 25 June 2018 |
| | | SI 3348556 T1 | 30 October 2020 |
| | | NZ 722487 A | 29 April 2022 |
| | | HUE 040274 T2 | 28 March 2019 |
| | | ES 2808978 T3 | 02 March 2021 |
| | | ES 2808978 T8 | 22 March 2021 |
| | | US 2018093979 A1 | 05 April 2018 |
| | | US 10358443 B2 | 23 July 2019 |
| | | EP 3348556 A1 | 18 July 2018 |
| | | EP 3348556 B1 | 29 April 2020 |
| | | CY 1120348 T1 | 10 July 2019 |
| | | CL 2016001918 A1 | 13 January 2017 |
| | | ME 03800 B | 20 April 2021 |
| | | US 2016207912 A1 | 21 July 2016 |
| | | US 9738635 B2 | 22 August 2017 |
| | | ZA 201605246 B | 25 May 2022 |
| | | UA 118209 C2 | 10 December 2018 |
| | | PT 3099684 T | 22 October 2018 |
| | | PH 12016501493 A1 | 06 February 2017 |
| | | SG 11201606108 RA | 30 August 2016 |
| | | HUE 050964 T2 | 28 January 2021 |
| | | TR 201809440 T4 | 23 July 2018 |
| | | US 2019308965 A1 | 10 October 2019 |
| | | US 11078196 B2 | 03 August 2021 |
| | | AU 2015211119 A1 | 11 August 2016 |
| | | AU 2015211119 A8 | 15 December 2016 |

Form PCT/ISA/210 (patent family annex) (July 2022)

**INTERNATIONAL SEARCH REPORT**
**Information on patent family members**

International application No.
**PCT/CN2022/141820**

| Patent document cited in search report | Publication date (day/month/year) | Patent family member(s) | Publication date (day/month/year) |
|---|---|---|---|
| | | AU 2015211119 B2 | 30 May 2019 |
| | | DK 3348556 T3 | 03 August 2020 |
| | | PL 3348556 T3 | 02 November 2020 |
| | | ES 2675301 T3 | 10 July 2018 |
| | | JP 2019163321 A | 26 September 2019 |
| | | JP 6783900 B2 | 11 November 2020 |
| | | CA 2937967 A1 | 06 August 2015 |
| | | CA 2937967 C | 26 July 2022 |
| | | JP 2017505779 A | 23 February 2017 |
| | | JP 6619741 B2 | 11 December 2019 |
| | | PT 3348556 T | 27 July 2020 |
| | | RS 60547 B1 | 31 August 2020 |
| | | MY 187450 A | 22 September 2021 |
| | | RS 57533 B1 | 31 October 2018 |
| | | PL 3099684 T3 | 28 September 2018 |
| | | DK 3099684 T3 | 16 July 2018 |
| CN 102725284 A | 10 October 2012 | BR 112012014807 A2 | 27 June 2017 |
| | | US 2013035342 A1 | 07 February 2013 |
| | | US 8846682 B2 | 30 September 2014 |
| | | CA 2784744 A1 | 14 July 2011 |
| | | NZ 600449 A | 31 October 2014 |
| | | RU 2012129882 A | 27 January 2014 |
| | | WO 2011084642 A1 | 14 July 2011 |
| | | EP 2513090 A1 | 24 October 2012 |
| | | JP 2013514980 A | 02 May 2013 |
| | | JP 5922031 B2 | 24 May 2016 |
| | | US 2014364610 A1 | 11 December 2014 |
| | | JP 2016074679 A | 12 May 2016 |
| | | AU 2010339841 A1 | 05 July 2012 |
| | | AU 2010339841 B2 | 25 September 2014 |
| | | MX 2012006740 A | 07 September 2012 |
| | | MX 335989 B | 07 January 2016 |
| | | KR 20120112548 A | 11 October 2012 |
| CN 110167937 A | 23 August 2019 | ES 2884145 T3 | 10 December 2021 |
| | | CO 2019007830 A2 | 31 July 2019 |
| | | KR 20190112022 A | 02 October 2019 |
| | | CA 3045221 A1 | 02 August 2018 |
| | | CL 2019001891 A1 | 22 November 2019 |
| | | EA 201991755 A1 | 22 January 2020 |
| | | MX 2019008256 A | 09 September 2019 |
| | | RU 2019126167 A | 26 February 2021 |
| | | IL 267993 A | 26 September 2019 |
| | | AU 2018212438 A1 | 25 July 2019 |
| | | AU 2018212438 B2 | 17 December 2020 |
| | | AU 2018212438 C1 | 06 May 2021 |
| | | WO 2018138088 A1 | 02 August 2018 |
| | | US 2019367513 A1 | 05 December 2019 |
| | | US 10889584 B2 | 12 January 2021 |
| | | JP 2020505369 A | 20 February 2020 |
| | | JP 7073385 B2 | 23 May 2022 |

Form PCT/ISA/210 (patent family annex) (July 2022)

**INTERNATIONAL SEARCH REPORT**
**Information on patent family members**

International application No.
**PCT/CN2022/141820**

| Patent document cited in search report | Publication date (day/month/year) | Patent family member(s) | Publication date (day/month/year) |
|---|---|---|---|
| | | BR 112019011924 A2 | 29 October 2019 |
| | | EP 3573986 A1 | 04 December 2019 |
| | | EP 3573986 B1 | 26 May 2021 |
| CN 110198938 A | 03 September 2019 | IL 267994 A | 26 September 2019 |
| | | CA 3048947 A1 | 02 August 2018 |
| | | MA 47370 A | 24 March 2021 |
| | | RU 2019126170 A | 26 February 2021 |
| | | CL 2019001752 A1 | 22 November 2019 |
| | | CO 2019007835 A2 | 31 July 2019 |
| | | EP 3573982 A1 | 04 December 2019 |
| | | EP 3573982 B1 | 16 June 2021 |
| | | ES 2882285 T3 | 01 December 2021 |
| | | WO 2018138086 A1 | 02 August 2018 |
| | | MX 2019007053 A | 29 August 2019 |
| | | BR 112019011932 A2 | 29 October 2019 |
| | | AU 2018212436 A1 | 25 July 2019 |
| | | KR 20190111080 A | 01 October 2019 |
| | | US 2019367502 A1 | 05 December 2019 |
| | | US 10913735 B2 | 09 February 2021 |
| | | EA 201991756 A1 | 23 January 2020 |
| | | JP 2020505372 A | 20 February 2020 |
| | | JP 7100043 B2 | 12 July 2022 |
| CN 110191885 A | 30 August 2019 | EP 3573981 A1 | 04 December 2019 |
| | | EP 3573981 B1 | 16 March 2022 |
| | | JP 2020505368 A | 20 February 2020 |
| | | JP 7100042 B2 | 12 July 2022 |
| | | ES 2918048 T3 | 13 July 2022 |
| | | RU 2019126164 A | 26 February 2021 |
| | | MX 2019007054 A | 29 August 2019 |
| | | AU 2018213886 A1 | 25 July 2019 |
| | | BR 112019011931 A2 | 29 October 2019 |
| | | CL 2019001750 A1 | 22 November 2019 |
| | | US 2020291013 A1 | 17 September 2020 |
| | | US 11091474 B2 | 17 August 2021 |
| | | KR 20190112031 A | 02 October 2019 |
| | | CO 2019007833 A2 | 20 August 2019 |
| | | IL 268003 A | 26 September 2019 |
| | | EA 201991753 A1 | 14 January 2020 |
| | | CA 3048946 A1 | 02 August 2018 |
| | | WO 2018138085 A1 | 02 August 2018 |
| | | MA 47369 A | 04 December 2019 |
| WO 2017020010 A1 | 02 February 2017 | US 2018222899 A1 | 09 August 2018 |
| | | US 10975066 B2 | 13 April 2021 |
| | | EP 3328379 A1 | 06 June 2018 |
| | | EP 3328379 A1 | 28 July 2021 |
| | | EP 3328379 A4 | 19 June 2019 |
| | | ES 2885049 T3 | 13 December 2021 |
| WO 2021055591 A1 | 25 March 2021 | KR 20220102607 A | 20 July 2022 |
| | | JP 2022548381 A | 18 November 2022 |
| | | US 2022380319 A1 | 01 December 2022 |

Form PCT/ISA/210 (patent family annex) (July 2022)

**INTERNATIONAL SEARCH REPORT**
**Information on patent family members**

International application No.
**PCT/CN2022/141820**

| Patent document cited in search report | Publication date (day/month/year) | Patent family member(s) | Publication date (day/month/year) |
|---|---|---|---|
| | | BR 112022004801 A2 | 21 June 2022 |
| | | EP 4031532 A1 | 27 July 2022 |
| | | AU 2020348771 A1 | 17 March 2022 |
| | | CA 3151039 A1 | 25 March 2021 |
| WO 2010012399 A1 | 04 February 2010 | EP 2149554 A1 | 03 February 2010 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2015116663 A1